# EUROPEAN PATENT APPLICATION

(11) **EP 4 663 163 A1**
(43) Date of publication of application: **17.12.2025**
(21) Application number: 24769910.1
(22) Date of filing: 11.03.2024
(51) Int. Cl.: A61F 2/24

(54) **DELIVERY DEVICE FOR HEART VALVE PROSTHESIS, THREADING MEMBER AND HANDLE OF DELIVERY DEVICE, AND RECOVERY DEVICE AND DELIVERY DEVICE FOR RECOVERY THREADS OF HEART VALVE PROSTHESIS**

(30) Priority: 13.03.2023 CN 202310239885; 13.03.2023 CN 202310239884; 13.03.2023 CN 202310240162; 13.03.2023 CN 202310245124; 13.03.2023 CN 202310245109
(71) Applicant: Mitrassist Lifesciences Limited, Shanghai 201807 (CN)
(72) Inventor: LI, Xiaopeng, Shanghai 201807 (CN); ZHONG, Wei, Shanghai 201807 (CN); SHEN, Bin, Shanghai 201807 (CN); LI, Hongdeng, Shanghai 201807 (CN); YIN, Jiang, Shanghai 201807 (CN); YUAN, Hu, Shanghai 201807 (CN)
(74) Representative: Berggren Oy
(86) International application number: PCT/CN2024/081013
(87) International publication number: WO 2024/188217

(57) **Abstract**

An interventional delivery device for a heart valve prosthesis, and a threading member (101) of the delivery device. The interventional delivery device for the heart valve prosthesis comprises: an outer tube (103); an inner tube (102) located in the outer tube (103); a threading member (101) provided on the inner tube (102), the threading member (101) being provided with a threading hole (114) and a positioning portion (111); a suture end positioning member (105), one end of the suture end positioning member (105) being detachably positioned in the positioning portion (111) of the threading member (101); and a traction member (106), one end of the traction member (106) passing through the threading hole (114) to be connected to a heart valve prosthesis (104) and to be connected to the suture end positioning member (105), and the other end of the traction member extending out of the outer tube (103). By means of such an arrangement, the relative position of the heart valve prosthesis (104) and the heart can be adjusted, thereby improving the implantation accuracy of the heart valve prosthesis (104).

## Description

### Cross-Reference to Related Applications

The present application claims the priority of the Chinese patent application with application number 202310239885X, titled "INTERVENTIONAL DELIVERY DEVICE FOR HEART VALVE PROSTHESIS AND THREADING MEMBER THEREOF", filed with the China National Intellectual Property Administration on March 13, 2023, the entire content of which is incorporated herein by reference. The present application claims the priority of the Chinese patent application with application number 2023102401621, titled "RETRIEVAL DEVICE FOR RETRIEVING CONNECTION SUTURE AND DELIVERY DEVICE FOR HEART VALVE PROSTHESIS", filed with the China National Intellectual Property Administration on March 13, 2023, the entire content of which is incorporated herein by reference. The present application claims the priority of the Chinese patent application with application number 2023102451245, titled "RETRIEVAL DEVICE FOR RETRIEVING SUTURE FOR HEART VALVE PROSTHESIS AND DELIVERY DEVICE THEREOF", filed with the China National Intellectual Property Administration on March 13, 2023, the entire content of which is incorporated herein by reference. The present application claims the priority of the Chinese patent application with application number 2023102398845, titled "INTERVENTIONAL DELIVERY DEVICE FOR HEART VALVE PROSTHESIS AND HANDLE THEREOF", filed with the China National Intellectual Property Administration on March 13, 2023, the entire content of which is incorporated herein by reference. The present application claims the priority of the Chinese patent application with application number 2023102451090, titled "DELIVERY DEVICE FOR HEART VALVE PROSTHESIS", filed with the China National Intellectual Property Administration on March 13, 2023, the entire content of which is incorporated herein by reference.

### Technical Field

The present application relates to the technical field of medical devices, and in particular, to a delivery device for a heart valve prosthesis, a threading member thereof, a handle thereof, and a retrieval device for a retrieval suture of a heart valve prosthesis and a delivery device thereof.

### Background Art

Heart valve disease is one of the most common heart diseases in China, mainly caused by valve damage due to rheumatic fever. In recent years, with the aging of the population, degenerative valve changes (including calcification and myxoid degeneration) and metabolic disorder-related valve damage have also increased significantly in China. Currently, minimally invasive surgical implantation of heart valve prostheses through interventional procedures has provided medical staff with a new treatment method featuring smaller trauma, fewer complications, and faster postoperative retrieval.

At present, heart valve prostheses can be introduced into the body via catheters, i.e., placed through minimally invasive interventional surgery without thoracotomy, thus minimizing trauma and accelerating retrieval. This offers a novel solution for patients with heart valve stenosis who cannot have their lives prolonged or suffering relieved by conventional treatments.

The implantation of a heart valve prosthesis typically relies on a delivery device to transport and deploy the prosthesis at a predetermined site. However, existing delivery devices still fail to meet application requirements adequately, particularly in that they cannot achieve circumferential rotation of the heart valve prosthesis, leading to low accuracy in deployment positioning.

During valve treatment, a delivery device is required to transport, control the shape, and deploy the heart valve prosthesis. However, when medical staff operate the existing delivery devices, they sometimes need to rotate the operating handle to implant the valve prosthesis. Yet, after rotating the handle, the operation direction must be adjusted accordingly, causing operational inconvenience. When the implantation position of the heart valve prosthesis is inaccurate, it is difficult to adjust the position of the heart valve prosthesis relative to the heart, thereby increasing the surgical risk and reducing the success rate of the surgery.

Compared with traditional surgical methods that require incising the diseased site, interventional therapy is now more commonly used to deliver interventional devices (such as artificial heart valve stents) to the diseased site via an interventional delivery device, which is then withdrawn to leave the interventional device in place as a replacement for the diseased organ. This approach significantly reduces patient suffering.

During interventional valve therapy, an interventional delivery device is needed to transport, control the shape, and deploy the heart valve prosthesis. When the implantation position is inaccurate, fine-tuning of the prosthesis' local position relative to the heart is sometimes required. However, in existing technologies, it is often difficult to adjust the local position of the heart valve prosthesis relative to the heart, which causes operational inconvenience for medical staff, increases surgical risks, and reduces surgical success rates. When the implantation position of a heart valve prosthesis is inaccurate, it is sometimes necessary to tighten the multiple connection rings at the proximal end of the heart valve prosthesis, re-housing it into the tubular component for redeployment. However, during retrieval, the multiple connecting rings must be tightened simultaneously, risking that they may not lock into the clamping grooves of the fixing head synchronously, thus preventing heart valve prosthesis retrieval.

### Summary

One of the technical problems to be solved by the present application is to provide a delivery device for a heart valve prosthesis and a threading member thereof, which can adjust the relative position between the heart valve prosthesis and the heart, and improve the accuracy of implantation of the heart valve prosthesis.

One of the technical problems to be solved by the present application is to provide a retrieval device capable of individually adjusting corresponding retrieval connection sutures and a delivery device for a heart valve prosthesis.

One of the technical problems to be solved by the present application is to provide a retrieval device for a retrieval suture of a heart valve prosthesis and a delivery device thereof, which can fine-tune the implantation position of the heart valve prosthesis to improve the accuracy of implantation of the heart valve prosthesis.

One of the technical problems to be solved by the present application is to provide an interventional delivery device for a heart valve prosthesis and a handle thereof, which can improve operational convenience.

One of the technical problems to be solved by the present application is to provide a delivery device for a heart valve prosthesis, which can realize circumferential positioning of the heart valve prosthesis and improve the accuracy of the implantation position of the heart valve prosthesis.

To solve the above technical problems, the present application adopts the following technical solutions.

The present application provides an interventional delivery device for a heart valve prosthesis, comprising: an outer tube; an inner tube located in the outer tube; a threading member disposed on the inner tube, the threading member being provided with a threading through hole (threading hole) and a positioning area (positioning portion); a suture end positioning member, one end of the suture end positioning member being detachably positioned in the positioning area of the threading member; and a traction member, one end of the traction member passing through the threading through hole to be connected to a heart valve prosthesis and to be connected to the suture end positioning member, and the other end of the traction member extending out of the outer tube.

As an embodiment, the threading member is provided with an abutment platform configured to abut against the heart valve prosthesis.

As an embodiment, the abutment platform comprises an outer ring portion extending radially outward from the threading member and a tapered portion gradually narrowing toward the proximal end from the outer ring portion.

As an embodiment, the tapered portion is provided with a plurality of threading through holes penetrating through the abutment platform.

As an embodiment, the number of the threading through holes is three, and the three threading through holes are circumferentially arranged along the tapered portion.

As an embodiment, the threading member comprises a plurality of support tabs extending distally from the outer ring portion, wherein an accommodation space is formed between two adjacent support tabs, and the accommodation space is in communication with the threading through hole.

As an embodiment, the positioning area is provided on the side of the abutment platform facing away from the support tabs.

As an embodiment, the number of the traction members is three, and the three traction members respectively pass through the three threading through holes and are configured to connect with the heart valve prosthesis.

As an embodiment, the interventional delivery device for a heart valve prosthesis further comprises a handle, wherein the outer tube extends from the handle, a receiving space for accommodating the heart valve prosthesis is provided between the outer tube and the inner tube, and the outer tube is adapted to move axially along the handle to unfold the heart valve prosthesis or retract it into the receiving space.

As an embodiment, the interventional delivery device further comprises a heart valve prosthesis provided with a plurality of connection ring sets, wherein in a contracted state of the heart valve prosthesis, each of the connection ring sets abuts against the abutment platform and corresponds to the threading through holes on the same side;
one end of each of the plurality of traction members is connected to the handle, and the other end respectively passes through the threading through hole and the connection ring set corresponding to the threading through hole, and is connected to the suture end positioning member in a detachable manner.

As an embodiment, the threading member is provided with a protrusion for fitting against one side of the connection ring set.

As an embodiment, the threading member is provided with a passageway (channel) configured to guide an inner tube therethrough.

As an embodiment, the positioning area is provided with a positioning hole extending axially along the threading member, the extending direction of the positioning hole is parallel to the extending direction of the passageway, and one end of the suture end positioning member is insertable into the positioning hole.

The present application provides a threading member of an interventional delivery device for a heart valve prosthesis, comprising: a passageway configured to guide an inner tube therethrough; a threading through hole configured to guide a traction member therethrough; and a positioning area configured to position a suture end positioning member.

As an embodiment, the threading member comprises an abutment platform configured to abut against a heart valve prosthesis, wherein the abutment platform comprises an outer ring portion and a tapered portion gradually narrowing toward the proximal end from the outer ring portion, and threading through holes are circumferentially disposed on the abutment platform.

As an embodiment, the end face of the abutment platform is further provided with a plurality of support tabs, the support tabs extend from the end face of the outer ring portion toward the distal end of the threading member, an accommodation space is formed between two adjacent support tabs, and the accommodation space is in communication with the threading through hole.

As an embodiment, the threading member is provided with a protrusion, and the protrusion and the support tabs are located on the same side of the abutment platform.

As an embodiment, the positioning area is provided with an axially extending positioning hole, and the extending direction of the positioning hole is parallel to the extending direction of the passageway.

The present application provides a retrieval device for retrieving a connection suture, comprising: a body (retrieval body) configured to be connected to a handle; and a suture winding/unwinding assembly mounted on the body, the suture winding/unwinding assembly comprising: a rod rotatable relative to the body; and a plurality of single-rotation units disposed on the rod and configured to rotate in one direction for suture winding relative to the rod, each single-rotation unit being provided with: a retaining portion (fixing portion) configured to connect to the connection suture; and a suture winding portion configured to wind the connection suture.

As an embodiment, the single-rotation unit comprises a unidirectional rotating member and an operating member cooperatively connected to the unidirectional rotating member, the unidirectional rotating member being mounted on the rod, and the operating member being assembled to the rod via the unidirectional rotating member.

As an embodiment, the operating member comprises an operating portion protruding from the body and the suture winding portion connected to the operating portion, wherein the retaining portion is located on the suture winding portion.

As an embodiment, the retaining portion comprises a protrusion or at least two first through holes configured to be fixed to the connection suture.

As an embodiment, the operating member is provided with a mounting recess (mounting groove) extending through the operating portion and the suture winding portion, the unidirectional rotating member being located in the mounting recess.

As an embodiment, the unidirectional rotating member is a unidirectional bearing.

As an embodiment, the body comprises a retrieval handle and a cover detachably connected to the retrieval handle, the rod extending through the cover perpendicular to the axial direction of the retrieval handle.

As an embodiment, the suture winding/unwinding assembly comprises a flange portion (flange) located at one end of the rod and fixedly connected to the rod, a first connection hole is provided on the side of the cover facing the flange portion, and the diameter of the flange portion is greater than the diameter of the first connection hole.

As an embodiment, the suture winding/unwinding assembly comprises a rotating cap capable of driving the entire rod to rotate, and the rotating cap is located at the other end opposite to the flange portion.

As an embodiment, the suture winding/unwinding assembly further comprises a locking portion provided on the rotating cap for cooperating with the cover to prevent the rod from accidental rotation.

As an embodiment, the locking portion comprises a locking gear, and the cover is provided with a locking tooth groove adapted to the locking gear.

As an embodiment, the cover comprises a convex cylinder protruding toward the rotating cap along the extending direction of the rod, and the locking tooth groove is provided on the inner wall of the convex cylinder.

As an embodiment, the convex cylinder is provided with a second connection hole, the second connection hole is coaxial with the first connection hole, and the rod is threadedly connected to the rotating cap through the first and second connection holes.

As an embodiment, a telescopic member is further provided between the flange portion and the cover, with both ends of the telescopic member abutting against the flange portion and the cover respectively, for engaging the locking gear with the locking tooth groove.

As an embodiment, the outer surface of the rod is provided with two clamping grooves at intervals; the retrieval device comprises a stop block embedded in the suture winding portion of the operating member adjacent to the rotating cap, and further comprises two clamping members that cooperate with the clamping grooves, wherein the plurality of single-rotation units are positioned between the two clamping members, one of the clamping members abuts against the single-rotation unit distal to the rotating cap, the other clamping member abuts against the stop block, and the two clamping members are configured to axially retain and restrict the plurality of single-rotation units along the rod.

As an embodiment, the plurality of single-rotation units are sequentially arranged along the axial direction of the rod, the operating member adjacent to the rotating cap is a first operating member, the operating member abutting against the first operating member is a second operating member, and an insertion recess (insertion groove) adapted to the suture winding portion is provided on the side of the first and second operating members facing away from the rotating cap.

As an embodiment, the cover is provided with a plurality of movable openings corresponding in number to the single-rotation units, and the operating portion partially protrudes from the movable openings.

As an embodiment, the retrieval handle is provided therein with a threading passage (threading channel) for guiding the connection suture, the cover is provided therein with a move chamber in communication with the movable opening, and a threading hole for guiding the connection suture into the move chamber is further provided at a side of the distal end of the cover.

As an embodiment, the retrieval device further comprises a support block (support member) disposed in the move chamber and fixedly connected to the cover, wherein the support block is provided with a notch, and the suture winding portion on the operating member adjacent to the rotating cap is embedded in the notch.

The present application further provides a delivery device for a heart valve prosthesis, comprising: a handle; and a retrieval device for retrieving a connection suture of a heart valve prosthesis as mentioned above, wherein the body is disposed at the proximal end of the handle.

As an embodiment, the body is detachably connected to the handle.

The present application further provides a retrieval device for a retrieval suture of a heart valve prosthesis, comprising: a body; and a suture winding/unwinding assembly disposed on the body, wherein the suture winding/unwinding assembly comprises a main rotating member for synchronously driving a plurality of retrieval sutures and an auxiliary adjusting member for driving one retrieval suture, the auxiliary adjusting member comprises a suture adjusting portion, the main rotating member comprises a suture winding portion, and the suture adjusting portion and the suture winding portion are respectively located at different positions along the axial direction of the body.

As an embodiment, the suture adjusting portion is provided with a first through hole for one retrieval suture to pass through, and the suture adjusting portion is rotatable relative to the body to adjust the position or state of the heart valve prosthesis via the retrieval suture.

As an embodiment, the suture adjusting portion is of a columnar structure, the auxiliary adjusting member comprises a drive portion fixedly connected to the suture winding portion, and the radial dimension of the drive portion is larger than that of the suture winding portion.

As an embodiment, the auxiliary adjusting member is mounted on the body via a single-rotation unit (unidirectional rotating unit), and the single-rotation unit is located at the side opposite to the drive portion.

As an embodiment, the number of the auxiliary adjusting members is three.

As an embodiment, the three auxiliary adjusting members are arranged in a staggered manner.

As an embodiment, the three auxiliary adjusting members comprise one first auxiliary adjusting member and two second auxiliary adjusting members, wherein the first auxiliary adjusting member and the two second auxiliary adjusting members are spaced apart along a distal-to-proximal direction of the body, and the distances from the first auxiliary adjusting member to the two second auxiliary adjusting members are equal.

As an embodiment, the suture winding portion and the suture adjusting portion are mounted on the body perpendicularly to each other.

As an embodiment, the body comprises a cover and a retrieval handle, and the cover is detachably connected to the retrieval handle.

As an embodiment, the main rotating member comprises a rotating cap capable of driving the entire suture winding portion to rotate, and the rotating cap is located at one end of the suture winding portion.

As an embodiment, the main rotating member further comprises a locking portion provided on the rotating cap and facing the cover, for cooperating with the cover to prevent the suture winding portion from accidental rotation.

As an embodiment, the locking portion comprises a locking gear, and the cover is provided with a locking tooth groove adapted to the locking gear.

As an embodiment, the cover comprises a convex cylinder protruding toward the rotating cap along the extending direction of the suture winding portion, and the locking tooth groove is provided on the inner wall of the convex cylinder.

As an embodiment, the suture winding portion is of a rod-like structure, the cover is provided with a first connection hole, the suture winding portion is rotatably connected to the cover through the first connection hole, and the suture winding portion is provided with a protrusion or a second through hole for connecting the retrieval suture.

As an embodiment, one end of the suture winding portion is provided with a flange located on the side opposite to the rotating cap, and the diameter of the flange is larger than that of the first connection hole.

As an embodiment, the convex cylinder is provided with a second connection hole coaxial with the first connection hole, and the suture winding portion is threadedly connected to the rotating cap through the first and second connection holes.

As an embodiment, the cover is provided therein with an extension block extending toward the proximal end of the cover, and the extension block and the retrieval handle are respectively provided with a first mounting groove and a second mounting groove for mounting the single-rotation unit.

As an embodiment, the retrieval handle is provided with a threading passage (threading channel) for guiding the retrieval suture to pass out, and the side of the cover facing the threading passage is provided with a plurality of threading holes for guiding the retrieval suture to penetrate into the cover.

The present application further provides a delivery device for a heart valve prosthesis, comprising: a handle (main handle); and a retrieval device for a retrieval suture of a heart valve prosthesis as mentioned above, wherein the body is disposed at the proximal end of the handle and is detachably connected to the handle.

As an embodiment, the delivery device further comprises a heart valve prosthesis, wherein a plurality of retrieval sutures respectively pass through a plurality of connection ring sets on the heart valve prosthesis, and the auxiliary adjusting member is rotatable relative to the body by a certain angle to adjust the position or state of the heart valve prosthesis via one of the retrieval sutures.

The present application further provides an interventional delivery device for a heart valve prosthesis, comprising: a handle; a delivery assembly extending within the handle and axially movable relative to the handle, wherein the delivery assembly comprises a transmission rod, and at least two first drive assemblies circumferentially arranged along the transmission rod, the first drive assemblies being in transmission connection with the transmission rod and configured to drive the transmission rod to move axially relative to the handle.

As an embodiment, the interventional delivery device for a heart valve prosthesis further comprises a second drive assembly disposed on the transmission rod, wherein the first drive assemblies are in transmission connection with the second drive assembly, and the first drive assemblies are adapted to drive the second drive assembly to rotate, so that the transmission rod moves axially relative to the handle.

As an embodiment, the first drive assembly comprises a first drive member and a first support member sleeved on the outer circumference of the first drive member, the first support member being in tight fit connection with the first drive member, and an end of the first drive member being provided with a first drive portion; the second drive assembly comprises a second drive member and a second support member sleeved on the outer circumference of the second drive member, the second support member being in tight fit connection with the second drive member, and an end of the second drive member being provided with a second drive portion, wherein the second drive portion being engaged with the first drive portion, the transmission rod passing through the second drive member and being in threaded transmission connection with the second drive member, and the first drive member being adapted to drive the second drive member to rotate, so that the transmission rod moves axially relative to the handle.

As an embodiment, the first drive portion comprises a plurality of first drive teeth, the second drive portion comprises a plurality of second drive teeth, a drive tooth groove is formed between two adjacent second drive teeth, and the plurality of first drive teeth are engaged with the plurality of drive tooth grooves.

As an embodiment, the interventional delivery device for a heart valve prosthesis further comprises a power rod, wherein the first drive member is provided with a recess formed by recessing from one end of the first drive member toward the first drive portion, and one end of the power rod is insertable into the recess to drive the first drive member to rotate.

As an embodiment, the handle comprises a first housing provided with a mounting groove corresponding to the first drive assembly, the mounting groove is provided with an annular boss therein, and the first support member is located in the mounting groove and abuts against the annular boss.

As an embodiment, a limiting groove is provided in the first housing, and the second support member is embedded in the limiting groove.

As an embodiment, an end cover for blocking the first support member is further provided at an opening of the mounting groove, and the end cover is detachably connected to a peripheral wall of the mounting groove.

As an embodiment, a chamber is provided in the first housing, the chamber being in communication with the limiting groove and extending axially along the first housing; and

a transmission member is mounted in the chamber, the delivery assembly further comprises a sheath tube, both the proximal end of the sheath tube and the distal end of the transmission rod being respectively connected to the transmission member, the transmission rod being adapted to move axially along the chamber such that the transmission member slides along the chamber and drives the sheath tube to move axially relative to the first housing.

As an embodiment, a reinforcing tube is sleeved on the proximal end of the sheath tube and fixedly connected to the sheath tube, the proximal end of the reinforcing tube is fixedly connected to the transmission member, and the reinforcing tube is provided with a plurality of holes for glue application.

As an embodiment, a sliding block is provided at the proximal end of the transmission rod, a sliding groove is further provided in the first housing, the sliding block is embedded in the sliding groove and slidably engaged with the sliding groove, and the proximal end of the sliding groove is provided with a stop portion for stopping and position limiting for the sliding block.

As an embodiment, a through channel (channel) is provided at the distal end of the first housing, and the distal end of the reinforcing tube is provided with a tapered head for passing through the through channel; the through channel is in communication with the chamber, and the sheath tube passes through the through channel and is connected to the transmission member in the chamber.

The present application further provides a handle of an interventional delivery device for a heart valve prosthesis, comprising a first housing, wherein at least two mounting grooves for accommodating first drive assemblies are circumferentially provided on the first housing.

As an embodiment, an insertion post is provided at the distal end of the first housing, and a plurality of teeth are provided at the terminal of the insertion post;
the handle comprises a second housing, an insertion groove is provided at the proximal end of the second housing, the insertion post is inserted into the insertion groove; a receiving groove for accommodating the plurality of teeth is provided at the proximal end of the second housing, the receiving groove is in communication with the insertion groove, and the receiving groove allows the plurality of teeth to rotate.

As an embodiment, the first housing comprises a first upper housing and a second lower housing, and the first upper housing and the second lower housing are mutually snapped;
the second housing comprises a second upper housing and a second lower housing, and the second upper housing and the second lower housing are mutually snapped.

As an embodiment, three mounting grooves are circumferentially provided on the first housing.

The present application further provides a delivery device for a heart valve prosthesis, comprising: a handle; an outer tube (outer sheath) connected to the handle; and an inner tube assembly extending within the outer tube and rotatable circumferentially relative to the outer tube; wherein the inner tube assembly comprises a first inner tube and a second inner tube extending within the first inner tube, and a receiving space for accommodating the heart valve prosthesis is provided between the first inner tube and the second inner tube.

As an embodiment, the proximal ends of both the first inner tube and the second inner tube are connected to the handle; the first inner tube comprises an extension tube segment connected to the handle and extending distally within the outer tube, and a receiving tube segment protruding from the distal end of the outer tube for accommodating the heart valve prosthesis, wherein the radial dimension of the receiving tube segment is larger than that of the extension tube segment.

In a possible implementation, the handle comprises a first housing and a second housing connected to the first housing, the second housing being rotatable circumferentially relative to the first housing, wherein a fixing member is provided within the first housing and connected to the proximal end of the outer tube; the handle further comprises a transmission member, a chamber is provided within the second housing, the transmission member is mounted within the chamber, the proximal end of the first inner tube is connected to the transmission member, and the proximal end of the second inner tube is connected to the second housing, such that when the transmission member rotates with the second housing relative to the first housing, the first inner tube and the second inner tube rotate circumferentially in synchronization relative to the outer tube.

In a possible implementation, the fixing member comprises a fixing post and a fixing portion connected to the fixing post, the proximal end of the outer tube is fixedly connected to the fixing post, a mounting cavity is provided within the first housing, the fixing post is embedded in the mounting cavity, the fixing portion is provided with at least one fixing hole, the first housing is provided with at least one fixing mating hole corresponding to the fixing hole, and a fastener passes through the fixing hole and the fixing mating hole to fixedly connect the fixing member to the first housing.

In a possible implementation, the first inner tube and the second inner tube both pass through the fixing member and are respectively connected to the transmission member and the proximal end of the second housing.

In a possible implementation, a reinforcing tube is sleeved on the proximal end of the first inner tube and fixedly connected to the first inner tube, and the proximal end of the reinforcing tube is fixedly connected to the transmission member.

In a possible implementation, the reinforcing tube is provided with a plurality of holes for glue application.

In a possible implementation, the first housing is located at the distal end of the handle, the second housing is located at the proximal end of the handle, the chamber extends axially along the second housing, and the transmission member is adapted to move axially along the second housing relative to the chamber to drive the first inner tube to move axially relative to the second inner tube.

In a possible implementation, a transmission rod is further provided in the second housing, the distal end of the transmission rod is fixedly connected to the transmission member, the proximal end of the transmission rod is provided with a sliding block, a sliding groove is further provided in the second housing, the sliding block is embedded in the sliding groove and slidably engaged with the sliding groove, and the proximal end of the sliding groove is provided with a stop portion for stopping and position limiting for the sliding block.

In a possible implementation, a drive assembly is further provided on the second housing for driving the transmission rod to move axially relative to the handle.

In a possible implementation, the drive assembly comprises a first drive member disposed on the transmission rod and in threaded connection with the transmission rod, the drive assembly further comprises at least two second drive members circumferentially arranged along the transmission rod, the second drive members are in transmission connection with the first drive member for driving the first drive member to rotate.

In a possible implementation, an insertion groove is provided at the proximal end of the first housing, an insertion post is provided at the distal end of the second housing, the insertion post is inserted into the insertion groove; a locking member is provided at the terminal of the insertion post, a receiving groove for accommodating the locking member is further provided at the proximal end of the first housing, the receiving groove is in communication with the insertion groove, and the receiving groove allows the locking member to rotate.

In a possible implementation, a locking mating member is further provided in the first housing, and the locking mating member is mated with the locking member to lock or unlock the relative position between the first housing and the second housing.

In a possible implementation, the locking member comprises a locking gear provided at the terminal of the insertion post, the locking gear forming a plurality of locking grooves; the locking mating member comprises a movable member and at least one locking post provided on the movable member, and when the movable member is driven, the movable member moves axially along the first housing, so that the locking post is adapted to be inserted into or disengaged from the locking groove.

In a possible implementation, a mounting gap is provided in the first housing; the movable member comprises a drive block, a connection block connected to the drive block, and a mounting block connected to the connection block, the connection block being slidably connected to the mounting gap, the drive block and the mounting block being located inside and outside the first housing respectively, and the mounting block being provided with the locking post.

In a possible implementation, a through-channel (channel) is provided in the insertion post, the distal end of the reinforcing tube is provided with a tapered head for passing through the through channel; the through channel is in communication with the chamber, the first inner tube passes through the through channel, the proximal end of the first inner tube is connected to the transmission member in the chamber; the second inner tube extends within the first inner tube and passes through the through channel to be connected to the proximal end of the second housing.

The technical solutions of the present application have the following effects.
1. In the present application, a threading member is provided on the inner tube, and the threading member is provided with a threading through hole and a positioning area, one end of the suture end positioning member is detachably connected to the threading member through the positioning area, one end of the traction member passes through the threading through hole and is connected to the heart valve prosthesis, and then connected to the suture end positioning member. When the outer tube slides proximally, the heart valve prosthesis is implanted. When the implantation position of the heart valve prosthesis is inaccurate, since the traction member exerts a restrictive effect on the proximal end of the heart valve prosthesis, the traction member can be controlled to provide tension to the heart valve prosthesis, thereby adjusting the relative position of the heart valve prosthesis relative to the heart and achieving precise deployment of the heart valve prosthesis.
2. The threading through holes are provided through the tapered portion. On the one hand, they are used to guide the traction member to pass through. On the other hand, the threading through holes are provided on the outer circumference of the passageway, which reduces the obstruction to blood flow during the operation.
3. The abutment platform is matched with the protrusion, so that when the heart valve prosthesis is not accurately implanted in the correct position, the traction member can tighten the heart valve prosthesis again to pull the heart valve prosthesis back to the abutment platform and fit with the protrusion, thereby redeploying the heart valve prosthesis again.
4. The retrieval device includes a body connected to the handle and a suture winding/unwinding assembly provided on the body. The suture winding/unwinding assembly includes a rod rotatable relative to the body and a plurality of single-rotation units provided on the rod and capable of unidirectional rotation relative to the rod to achieve unidirectional suture winding. Each single-rotation units correspondingly control a single connection suture. At the same time, the single-rotation unit is provided with a retaining portion fixedly connected to the connection suture to prevent the connection suture from falling off, and a suture winding portion around which the connection suture can be wound. When the heart valve prosthesis needs to be retrieved, the single-rotation units can be sequentially rotated in one direction to wind the connection suture around the suture winding portion and tighten the heart valve prosthesis, thereby completing the retrieval of the heart valve prosthesis, improving the success rate of retrieving the heart valve prosthesis, and facilitating the operation of medical staff.
   Additionally, because the proximal end of the heart valve prosthesis is constrained by the connection sutures, when the heart valve prosthesis is incorrectly positioned, the single-rotation units can be rotated to wind the sutures around the suture winding portions. This action pulls the heart valve prosthesis to enable precise local adjustments, thereby enhancing implantation accuracy.
5. The suture winding/unwinding assembly includes a locking portion that cooperates with the cover to prevent the rod from accidental rotation. By providing the locking portion, accidental rotation of the rod can be prevented, further preventing medical staff from misoperating the delivery device, which may cause premature deployment of the heart valve prosthesis.
6. Two clamping grooves are spaced on the outer surface of the rod, and two clamping members are respectively clamped on the clamping grooves to axially limit the plurality of single-rotation units along the rod, preventing the single-rotation units from moving axially along the rod, so as to improve the stability of the retrieval device and facilitate the operation of medical staff.
7. The retrieval device for a retrieval suture includes a body and a suture winding/unwinding assembly disposed on the body, wherein the suture winding/unwinding assembly includes a main rotating member capable of synchronously driving a plurality of retrieval sutures. The main rotating member includes a suture winding portion, so that when the main rotating member rotates relative to the body, the retrieval sutures can be wound around the suture winding portion, thereby enabling the suture winding/unwinding assembly to retrieve or deploy the heart valve prosthesis.
   The suture winding/unwinding assembly further includes an auxiliary adjusting member capable of driving one retrieval suture. The auxiliary adjusting member includes a suture adjusting portion, which can be used to pull the corresponding retrieval suture to apply auxiliary force to the heart valve prosthesis, and the retrieval suture further pulls the heart valve prosthesis, thereby realizing fine adjustment of the local position of the heart valve prosthesis relative to the heart and improving the accuracy of implantation of the heart valve prosthesis.
   In addition, the suture adjusting portion and the suture winding portion are respectively located at different positions in the axial direction of the body, so that the auxiliary adjusting member and the main rotating member do not interfere with each other during operation, avoiding the problems that the heart valve prosthesis cannot be retrieved and deployed, and the position of the heart valve prosthesis relative to the heart cannot be finely adjusted.
8. The main rotating member includes a locking portion provided on the rotating cap and cooperating with the cover to prevent the suture winding portion from accidental rotation. By providing the locking portion, accidental rotation of the rod can be prevented, further preventing medical staff from misoperating the delivery device and causing premature deployment of the heart valve prosthesis.
9. The auxiliary adjusting member is mounted on the body through a single-rotation unit, so that the auxiliary adjusting member can finely adjust the position of the heart valve prosthesis by unidirectional rotation of the unidirectional rotating member relative to the body, and after the position of the heart valve prosthesis is adjusted, the unidirectional rotating member can also lock the auxiliary adjusting member against reverse rotation, preventing adjustment failure caused by unintended backtracking.
10. The handle of the delivery device is provided with a delivery assembly, which includes a transmission rod. The delivery device further includes at least two first drive assemblies, which are arranged circumferentially around the transmission rod and are in transmission connection with the transmission rod. By arranging at least two first drive assemblies circumferentially around the transmission rod, when the handle is rotated, medical staff can operate the first drive assemblies from multiple angles to drive the transmission rod to move axially relative to the handle, thereby implanting the valve prosthesis into the body and improving the operational convenience of the delivery device.
11. A plurality of mounting grooves are evenly distributed on the circumferential side of the first housing, and the mounting grooves correspondingly accommodate the first drive assemblies, so as to facilitate medical staff to drive the transmission rod to move axially relative to the first housing through the first drive assemblies on the handle.
12. A receiving space for accommodating the heart valve prosthesis is provided between the first inner tube and the second inner tube of the delivery device, and meanwhile, the first inner tube and the second inner tube can synchronously rotate circumferentially relative to the outer tube, thereby achieving the purpose of circumferential positioning of the heart valve prosthesis and improving the accuracy of implantation of the heart valve prosthesis.
13. The outer tube is sleeved on the outer circumference of the first inner tube, and the first inner tube and the outer tube work independently of each other, so that the first inner tube can be more easily rotated circumferentially relative to the outer tube to perform circumferential positioning on the heart valve prosthesis.

### Brief Description of Drawings

To more clearly illustrate the technical solutions of the embodiments of the present disclosure, the following briefly introduces the drawings required for the embodiments. It should be understood that the following drawings only show some embodiments of the present disclosure and therefore should not be considered as limiting the scope. For those of ordinary skill in the art, other related drawings can be obtained based on these drawings without creative efforts.
FIG. 1 is a schematic partial structural view of an interventional delivery device for a heart valve prosthesis provided in an embodiment of the present application;
FIG. 2 is a schematic partial structural view of a threading member provided in an embodiment of the present application;
FIG. 3 is a schematic structural view of the threading member from one perspective provided in an embodiment of the present application;
FIG. 4 is a schematic structural view of the threading member from another perspective provided in an embodiment of the present application;
FIG. 5 is a schematic structural view of an interventional delivery device for a heart valve prosthesis provided in an embodiment of the present application;
FIG. 6 is a schematic view of the connection mode between a traction member and a suture end positioning member provided in an embodiment of the present application;
FIG. 7 is a schematic structural view of a retrieval device for retrieving connection sutures provided in an embodiment of the present application;
FIG. 8 is a schematic top view of a retrieval device for retrieving connection sutures provided in an embodiment of the present application;
FIG. 9 is a schematic cross-sectional view taken along line A-A in FIG. 8;
FIG. 10 is a schematic partial exploded view of a retrieval device for retrieving connection sutures provided in an embodiment of the present application;
FIG. 11 is a schematic partial exploded view of a retrieval device for retrieving connection sutures from another perspective provided in an embodiment of the present application;
FIG. 12 is a schematic exploded view of a retrieval device for retrieving connection sutures from one perspective provided in an embodiment of the present application;
FIG. 13 is a schematic exploded view of a retrieval device for retrieving connection sutures from another perspective provided in an embodiment of the present application;
FIG. 14 is a schematic partial exploded view of a delivery device for a heart valve prosthesis provided in an embodiment of the present application;
FIG. 15 is a schematic structural view of a retrieval device for a retrieval suture provided in an embodiment of the present application;
FIG. 16 is a schematic top view of a retrieval device for a retrieval suture provided in an embodiment of the present application;
FIG. 17 is a schematic cross-sectional view taken along line A-A in FIG. 16;
FIG. 18 is a schematic exploded view of a retrieval device for a retrieval suture provided in an embodiment of the present application;
FIG. 19 is a schematic exploded view of a retrieval device for a retrieval suture from one perspective provided in an embodiment of the present application;
FIG. 20 is a schematic exploded view of a retrieval device for a retrieval suture from another perspective provided in an embodiment of the present application;
FIG. 21 is a schematic structural view of a delivery device provided in an embodiment of the present application;
FIG. 22 is a schematic partial structural view of a delivery device for a heart valve prosthesis provided in an embodiment of the present application;
FIG. 23 is a schematic cross-sectional view taken along line A-A in FIG. 22;
FIG. 24 is a schematic partial cross-sectional view of a delivery device for a heart valve prosthesis provided in an embodiment of the present application;
FIG. 25 an enlarged schematic structural view of part A in FIG. 24;
FIG. 26 is a schematic partial structural view of a delivery device for a heart valve prosthesis from one perspective provided in an embodiment of the present application;
FIG. 27 is a schematic partial structural view of a delivery device for a heart valve prosthesis from another perspective provided in an embodiment of the present application;
FIG. 28 is a schematic partial exploded view of a delivery device for a heart valve prosthesis provided in an embodiment of the present application;
FIG. 29 is an enlarged schematic structural view of part A in FIG. 28;
FIG. 30 is a schematic exploded view of a delivery device for a heart valve prosthesis provided in an embodiment of the present application;
FIG. 31 is a schematic structural view of a delivery device for a heart valve prosthesis provided in an embodiment of the present application;
FIG. 32 is a schematic cross-sectional view taken along line A-A in FIG. 31;
FIG. 33 is an enlarged schematic structural view of part A in FIG. 32;
FIG. 34 is a schematic partial structural view of a delivery device from one perspective provided in an embodiment of the present application;
FIG. 35 is a schematic cross-sectional view taken along line A-A in FIG. 34;
FIG. 36 is an enlarged schematic structural view of part A in FIG. 35;
FIG. 37 is an enlarged schematic structural view of part B in FIG. 35;
FIG. 38 is a schematic partial structural view of a delivery device from another perspective provided in an embodiment of the present application;
FIG. 39 is a schematic structural view of a first housing and a second housing provided in an embodiment of the present application;
FIG. 40 is a schematic partial structural view of a delivery device provided in an embodiment of the present application;
FIG. 41 is a schematic partial exploded view of a delivery device from one perspective provided in an embodiment of the present application;
FIG. 42 is a schematic partial exploded view of a delivery device from another perspective provided in the embodiment of the present application;
FIG. 43 is a schematic partial exploded view of a delivery device from still another perspective provided in the embodiment of the present application;
FIG. 44 is a schematic partial exploded view of a delivery device from yet another perspective provided in the embodiment of the present application.

Reference Signs: 101 - threading member; 111 - positioning area (positioning portion); 112 - passageway (channel); 114 - threading through hole (threading hole); 115 - abutment platform; 1151 - outer ring portion; 1152 - tapered portion; 116 - support tab; 117 - protrusion; 118 - accommodation space; 102 - inner tube; 103 - outer tube; 104 - heart valve prosthesis; 141 - connection ring set; 105 - suture end positioning member; 106 - traction member; 107 - handle; 108 - first suture clip; 109 - second suture clip;
201 - body (retrieval body); 211 - retrieval handle; 2111 - threading passage (threading channel); 212 - cover; 2121 - threading hole; 2122 - movable opening; 2123 - first connection hole; 2124 - second connection hole; 2125 - convex cylinder; 2126 - locking tooth groove; 202 - single-rotation unit; 221 - unidirectional rotating member; 222 - operating member; 2221 - retaining portion (fixing portion); 2222 - suture winding portion; 2223 - insertion recess (insertion groove); 2224 - operating portion; 2225 - mounting recess (mounting groove); 2226 - second through hole; 203 - rod; 231 - flange portion (flange); 232 - clamping groove; 204 - clamping member; 205 - telescopic member; 206 - rotating cap; 261 - locking portion; 207 - connection suture; 208 - support block (support member); 281 - notch; 209 - stop block; 210 - delivery device;
312 - extension block; 313 - first mounting groove; 314 - threading hole; 315 - first connection hole; 316 - second connection hole; 317 - convex cylinder; 318 - third through hole; 311 - retrieval handle; 321 - second mounting groove; 322 - threading passage; 303 - suture adjusting portion; 331 - first through hole; 332 - drive portion; 304 - suture winding portion; 341 - flange; 342 - second through hole; 306 - retrieval suture; 261 - locking portion;
401 - handle; 411 - first housing; 412 - limiting groove; 413 - chamber; 414 - mounting groove; 4141 - annular boss; 415 - end cover; 416 - sliding groove; 417 - through channel (channel); 418 - insertion post; 419 - protruding segment; 420 - second housing; 4201 - mounting cavity; 4202 - limiting space; 4203 - receiving groove; 421 - first drive assembly; 211 - first drive member; 4216 - first drive portion; 4217 - recess; 4212 - first support member; 422 - second drive assembly; 4221 - second drive member; 4226 - second drive portion; 4222 - second support member; 403 - transmission rod; 431 - sliding block; 404 - power rod; 405 - transmission member; 406 - sheath tube; 407 - reinforcing tube; 471 - tapered head; 472 - hole; 103 - outer tube (outer sheath); 410 - fixing member; 4100 - locking member;
501 - outer tube (outer sheath); 502 - second inner tube; 503 - first inner tube; 531 - receiving tube segment; 532 - extension tube segment; 504 - handle; 541 - first housing; 5411 - insertion groove; 5412 - mounting gap; 5413 - first upper housing; 5414 - first lower housing; 5415 - fixing mating hole; 5416 - mounting cavity; 5417 - receiving groove; 5418 - limiting groove; 542 - second housing; 5421 - insertion post; 5422 - through channel (channel); 5423 - chamber; 5424 - second upper housing; 5425 - second lower housing; 5426 - sliding groove; 543 - locking member; 5431 - locking gear; 544 - mounting groove; 5441 - annular boss; 545 - positioning groove; 505 - fixing member; 551 - fixing post; 552 - fixing portion; 5521 - fixing hole; 506 - locking mating member; 561 - movable member; 5611 - drive block; 5612 - connection block; 5613 - mounting block; 562 - locking post; 507 - transmission member; 508 - reinforcing tube; 581 - tapered head; 509 - guide head; 510 - transmission rod; 5101 - sliding block; 511 - first drive member; 5111 - first drive portion; 5112 - first support portion; 512 - second drive member; 5121 - second drive portion; 5122 - second support portion; 513 - end cover.

### Detailed Description of Embodiments

The following describes the technical solutions in the embodiments of the present application with reference to the accompanying drawings in the embodiments of the present application.

It should be noted that similar reference numerals and letters in the following drawings denote similar items. Therefore, once an item is defined in one drawing, it does not require further definition and explanation in subsequent drawings. Meanwhile, in the description of the present application, the term "distal end" refers to the end of the delivery device close to the cardiac tissue, the term "proximal end" refers to the end of the delivery device close to the operator. Terms such as "first" and "second" are only used for distinguishing descriptions and should not be construed as indicating or implying relative importance.

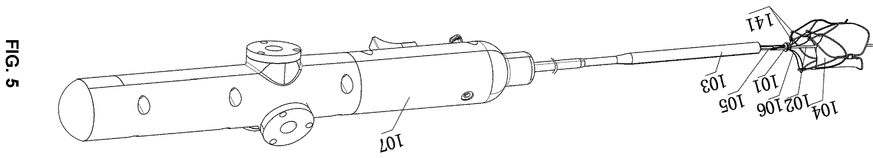

As shown in FIG. 5, an embodiment of the present application introduces an interventional delivery device for a heart valve prosthesis, which can be used to perform heart valve replacement. A receiving space for accommodating the heart valve prosthesis 104 is provided between the outer tube 103 and the inner tube 102. In the delivery state, the heart valve prosthesis 104 is in a contracted state and placed in the receiving space.

During the operation, medical staff operate the delivery device to control the outer tube 103 to slide proximally. During the sliding process, the distal end of the heart valve prosthesis 104 is expanded into an unfolded state through its own elastic deformation, while the proximal end is in a contracted state through the tension of the traction member 106. After the medical staff confirm the accurate implantation position, the traction member 106 is controlled to separate from the heart valve prosthesis 104. The proximal end of the heart valve prosthesis 104 is no longer constrained by the traction member 106, the heart valve prosthesis 104 is completely unfolded from the receiving space, and the heart valve prosthesis 104 is separated from the delivery device to complete the operation.

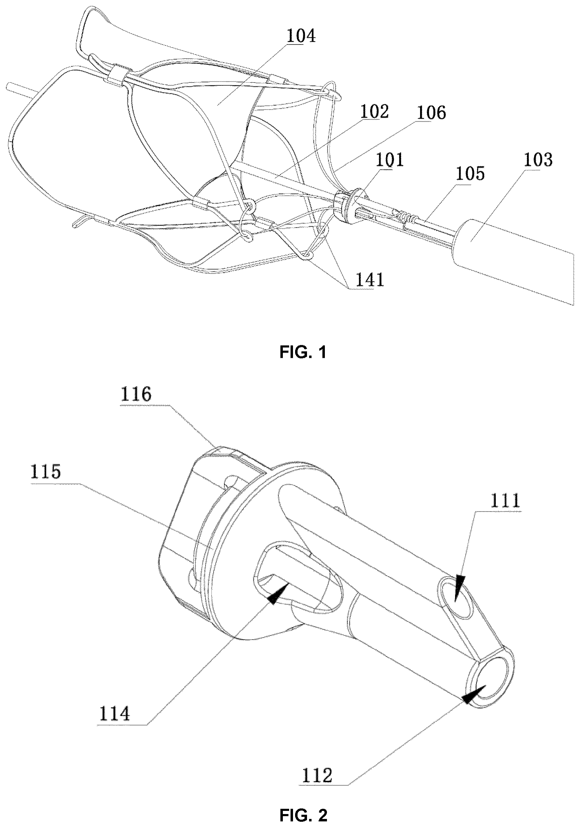

As shown in FIGS. 1 and 5, as an embodiment, the interventional delivery device for a heart valve prosthesis includes an outer tube 103, an inner tube 102 in the outer tube 103, the inner tube 102 extending in the outer tube 103, and further includes a threading member 101, a suture end positioning member 105, and a traction member 106. The threading member 101 is sleeved on the outer circumference of the inner tube 102 and is fixedly connected to the inner tube 102. The threading member 101 is provided with a threading through hole 114 and a positioning area 111. One end of the suture end positioning member 105 can be inserted into the positioning area 111 and detachably connected to the threading member 101 through the positioning area 111. One end of the traction member 106 passes through the threading through hole 114 to connect with the heart valve prosthesis 104, and is finally connected to the suture end positioning member 105, while the other end extends out of the outer tube 103. This configuration enables the delivery device to, during the deployment of the heart valve prosthesis 104, adjust the position of the heart valve prosthesis 104 relative to the heart by tensioning the traction member 106 when the implantation position of the heart valve prosthesis 104 is inaccurate. The traction member 106 exerts auxiliary force, further pulling the connection ring set 141 corresponding to the heart valve prosthesis 104, thus achieving fine-tuning of the implantation position and improving the implantation accuracy of the heart valve prosthesis 104.

In addition, during the deployment process of the heart valve prosthesis 104, the distal end of the heart valve prosthesis 104 is gradually expanded, while the proximal end is in a contracted state through the restriction of the traction member 106. Medical staff control the heart valve prosthesis 104 during the deployment process through the pulling of the traction member 106, so as to complete the precise deployment of the heart valve prosthesis 104 and improve the operation success rate. When the heart valve prosthesis 104 is in an accurate position, the suture end positioning member 105 is pulled out from the positioning area 111, so that one end of the traction member 106 falls off from the suture end positioning member 105, the proximal end of the heart valve prosthesis 104 is also in an unfolded state, and the heart valve prosthesis 104 is deployed.

Optionally, when the suture end positioning member 105 is inserted into the positioning area 111, the traction member 106 will restrict the proximal end of the heart valve prosthesis 104 to avoid the free deployment of the heart valve prosthesis 104. By controlling the deployment process of the heart valve prosthesis 104, it is possible to avoid the heart valve prosthesis 104 from causing harm to human tissues during the unfolding process, and also to avoid the inaccurate positioning of the heart valve prosthesis 104, which may affect the operation effect.

Optionally, in addition to controlling the deployment process of the heart valve prosthesis 104, the delivery device of the present application can further improve the implantation accuracy of the heart valve prosthesis 104. For example, when the deployment position of the heart valve prosthesis 104 is incorrect, since the proximal end of the heart valve prosthesis 104 is restricted by the traction member 106, the outer tube 103 can be slid distally to retract the heart valve prosthesis 104 for redeployment; alternatively, the position of the heart valve prosthesis 104 can be finely adjusted by pulling the traction member 106.

Optionally, the traction member 106 can be a connection suture, etc., and the suture end positioning member 105 is a rod-shaped structure.

As shown in FIG. 2, as an embodiment, the threading member 101 is provided with an abutment platform 115 for abutting against the heart valve prosthesis 104. By providing the abutment platform 115, when the heart valve prosthesis 104 is not accurately implanted in the correct position, the proximal end of the heart valve prosthesis 104 in the unfolded state is pulled by the traction member 106 to move toward the threading member 101 and abut against the abutment platform 115. The abutment platform 115 plays a role in limiting and supporting the heart valve prosthesis 104, and then the outer tube 103 is moved distally to retrieve the heart valve prosthesis 104. After medical staff adjust the implantation position, the heart valve prosthesis 104 is deployed again to improve the implantation accuracy of the heart valve prosthesis 104 and the success rate of the operation.

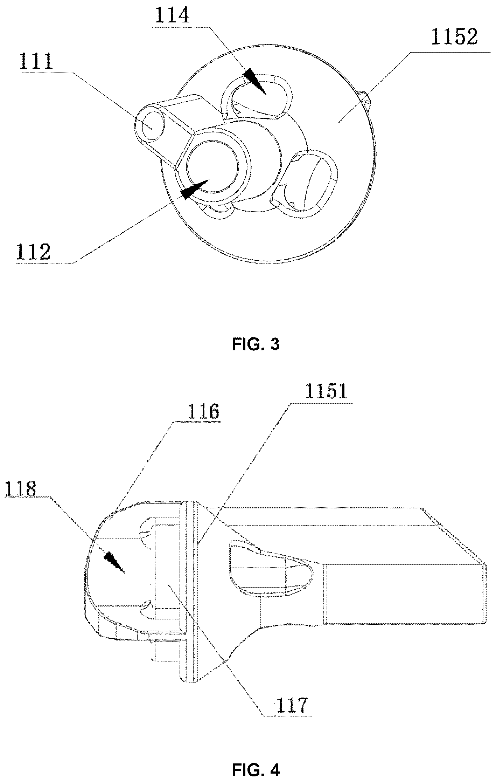

As shown in FIG. 4, as an embodiment, the abutment platform 115 includes an outer ring portion 1151 extending radially outward along the threading member 101. By providing the outer ring portion 1151, an abutment platform for the heart valve prosthesis 104 is provided, so that the heart valve prosthesis 104 can stably abut on the outer ring portion 1151, and meanwhile, the heart valve prosthesis 104 is prevented from passing through the outer circumference of the outer ring portion 1151. The abutment platform 115 further includes a tapered portion 1152 gradually tapering toward the proximal end from the outer ring portion 1151. The tapered portion 1152 is of a tapered structure, which can reduce the blockage of blood and help blood flow during the operation.

As shown in FIG. 3, as an embodiment, the tapered portion 1152 is provided with a plurality of threading through holes 114 penetrating the abutment platform 115. The threading through holes 114 penetrating the abutment platform 115 facilitate the passage of the pulling members 106, preventing the members from forming bending angles that may hinder medical staff operation. Additionally, the arrangement of multiple through holes 114 allows respective pulling members 106 to pass through, avoiding entanglement during prosthesis traction that could disrupt the surgical procedure and enhancing success rate. At the same time, by providing a plurality of threading through holes 114, a plurality of traction members 106 respectively pass through the plurality of threading through holes 114, avoiding the traction members 106 from being entangled when pulling the heart valve prosthesis 104, thereby affecting the operation process and improving the operation success rate. In addition, a plurality of traction members 106 respectively pass through a plurality of threading through holes 114, and each traction member 106 can work independently without affecting each other. When medical staff need to finely adjust the heart valve prosthesis 104, a certain one or more traction members 106 can be individually controlled to adjust the heart valve prosthesis 104 to the correct position, so as to improve the implantation accuracy of the heart valve prosthesis 104.

As a possible embodiment, the threading through holes 114 can also be provided as one, so as to reduce the number of openings in the threading member 101 and reduce the production cost.

As shown in FIGS. 2 and 3, as an embodiment, the number of the threading through holes 114 is three, and the three threading through holes 114 are circumferentially arranged along the tapered portion 1152, so as to ensure that the traction members 106 in the threading through holes 114 do not interfere with each other during operation, and each traction member 106 can work independently. When the implantation position of the heart valve prosthesis 104 is inaccurate, the position of the heart valve prosthesis 104 is adjusted through the traction members 106 to improve the precise positioning of the heart valve prosthesis 104.

Optionally, the three threading through holes 114 are evenly distributed along the circumferential direction of the tapered portion 1152, so as to facilitate medical staff to select a suitable angle to adjust the position of the heart valve prosthesis 104 through the traction members 106 and improve the convenience of operation for medical staff. In addition, the three threading through holes 114 are arranged through along the axial direction of the threading member 101 to guide blood flow, reduce the blockage of the threading member 101 to blood, and further improve the operation success rate.

As shown in FIGS. 2 and 4, as an embodiment, the threading member 101 includes a plurality of support tabs 116. The support tabs 116 are arranged to extend toward the distal end along the end surface of the outer ring portion 1151. An accommodation space 118 is formed between two adjacent support tabs 116, and the accommodation space 118 is communicated with the threading through hole 114, so as to ensure that the connection ring set 141 of the heart valve prosthesis 104 is adjusted in the corresponding accommodation space 118 during the process of retrieving the heart valve prosthesis 104, avoid being affected by other parts of the heart valve prosthesis 104. At the same time, during the process of retrieving the heart valve prosthesis 104, the corresponding connection ring set 141 of the heart valve prosthesis 104 is located in the accommodation space 118 and abuts against the outer ring portion 1151. The support tabs 116 play a role of spacing, avoid the heart valve prosthesis 104 from affecting each other during the retrieving process, and improve the retrieving success rate.

Optionally, the number of the support tabs 116 is three, and the three support tabs 116 are arranged at an angle of 120°, so as to limit three accommodation spaces 118. Each accommodation space 118 is correspondingly communicated with the threading through hole 114. When the heart valve prosthesis 104 abuts on the abutment platform 115, the support tabs 116 space the connection ring sets 141 on the adjacent heart valve prostheses 104 to avoid the connection ring sets 141 from affecting each other and improve the success rate of retrieving the heart valve prosthesis 104.

As shown in FIG. 2, as an embodiment, the positioning area 111 is provided on the side of the abutment platform 115 opposite to the support tabs 116, so as to avoid the suture end positioning member 105 from affecting the heart valve prosthesis 104 when the suture end positioning member 105 is inserted into the positioning area 111.

As an embodiment, there are three traction members 106, each of which passes through a threading member 101 and is used to connect with the heart valve prosthesis 104. This enables the heart valve prosthesis 104 to be stressed by a single traction member 106 when position adjustment is needed, while other traction members 106 do not need to apply force, improving implantation accuracy. In addition, during retrieval, the three traction members 106 can pull the heart valve prosthesis 104 from different angles while ensuring the retrieval success rate.

As shown in FIG. 5, as an embodiment, the delivery device includes a handle 107, an outer tube 103, and an inner tube 102. The outer tube 103 and inner tube 102 form an inner tube assembly, with both the outer tube 103 and inner tube 102 connected to the handle 107. The outer tube 103 is sleeved on the outer circumference of the inner tube 102, and the inner tube 102 extends inside the outer tube 103. A receiving space for the heart valve prosthesis 104 is provided between the outer tube 103 and the inner tube 102. When the heart valve prosthesis 104 is not implanted, it is located in the receiving space. When implantation is required, the outer tube 103 moves toward the proximal end of the handle 107, causing the distal end of the heart valve prosthesis 104 to expand while the proximal end remains contracted under the restraint of the traction member 106. If medical staff find the implantation position inaccurate, they can tighten the heart valve prosthesis 104, move the outer tube 103 distally to retract the heart valve prosthesis 104 into the receiving space, and deploy it again to improve implantation accuracy.

As shown in FIGS. 1 and 5, as an embodiment, the delivery device includes a heart valve prosthesis 104 with multiple connection rings, where two connection rings form a connection ring set 141. The heart valve prosthesis 104 has multiple connection ring sets 141. When the heart valve prosthesis 104 is in a contracted state, each connection ring set 141 abuts against the abutment platform 115 and corresponds to the threading through hole 114 on the same side, facilitating the traction member 106 to pass through the threading through hole 114 and connect with the connection ring set 141. Meanwhile, the support tabs 116 separate adjacent connection ring sets 141 to prevent the adjacent connection ring sets 141 from interfering with each other. One end of each of the multiple traction members 106 is connected to the handle 107, and the other end passes through the threading through hole 114 and the connection ring set 141 corresponding to the threading through hole 114, and is finally connected with the suture end positioning piece 105 in a detachable manner. When the suture end positioning piece 105 leaves the positioning area 111, the plurality of pulling members 106 are detached from the suture end positioning piece 105. After the detachment, there is no dead knot on each pulling member 106, which is convenient for medical staff to recover the pulling member 106.

Optionally, the heart valve prosthesis 104 includes 6 connection rings, with every two connection rings forming a connection ring set 141, thereby constituting three connection ring sets 141. Each traction member 106 correspondingly passes through a threading through hole 114 and one connection ring set 141, and is connected to the suture end positioning member 105 in a detachable manner. This configuration enables restriction of the heart valve prosthesis 104 during the deployment process, thereby enhancing implantation accuracy.

Optionally, the releasable connection between the traction member 106 and the suture end positioning member 105 means that after the suture end positioning member 105 is pulled out from the positioning area 111, the traction member 106 is detached from the suture end positioning member 105. Meanwhile, the detached pulling member 106 remains intact without any dead knots, facilitating its retrieval. Concurrently, the heart valve prosthesis 104 is deployed from the constraint of the pulling member 106 for full deployment.

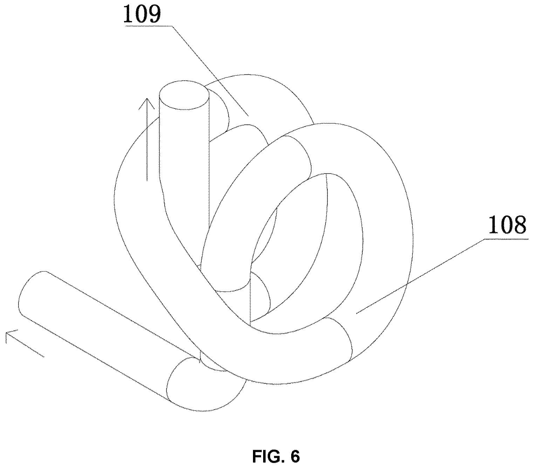

As shown in FIG. 6, which illustrates the connection mode between the traction member 106 and the suture end positioning member 105 in the embodiment of the present application. When the suture end positioning member 105 is detachably inserted into the positioning area 111, the traction member 106 is wound around the outer circumference of the suture end positioning member 105, and the suture end positioning member 105 can disengage from the winding knot of the traction member 106. One end of the traction member 106 forms a first suture clip 108, and the main body of the traction member 106 forms a second suture clip 109. The first suture clip 108 and the second suture clip 109 are arranged oppositely and wound around the outer circumference of the suture end positioning member 105 respectively. The extension directions of the extension segments of the first suture clip 108 and the second suture clip 109 are different. By applying forces in different directions to the extension segment of the first suture clip 108 and the extension segment of the second suture clip 109, the first suture clip 108 and the second suture clip 109 are wound around the outer circumference of the suture end positioning member 105. Meanwhile, when the suture end positioning member 105 is inserted into the positioning area 111, the traction member 106 cannot be detached from the suture end positioning member 105. When the suture end positioning member 105 is pulled out from the positioning area 111, the traction member 106 falls off from the suture end positioning member 105, and the traction member 106 is retrieved to prevent the traction member 106 from affecting the success rate of the surgery.

Certainly, the traction member 106 in the embodiment of the present application can also be connected to the suture end positioning member 105 in other releasable ways.

As an embodiment, the connection ring is annular. Therefore, the threading member 101 is provided with a protrusion 117 configured to fit against one side of the connection ring set 141. After the heart valve prosthesis 104 is retrieved, the traction member 106 applies a pulling force toward the threading through hole 114, causing the connection ring set 141 to fit with the protrusion 117 and abut against the outer ring portion 1151. Thus, the protrusion 117 further provides stable support for the heart valve prosthesis 104 and prevents the connection ring set 141 from being pulled into the threading through hole 114 by the traction member 106, which could lead to retrieval failure of the heart valve prosthesis 104.

Optionally, the protrusion 117 is annular, but it can also be in other shapes.

As shown in FIG. 2, as an embodiment, the threading member 101 is further provided with a passageway 112 for guiding the inner tube 102 to pass through, enabling the inner tube 102 to pass through the passageway 112 and connect with the threading member 101.

Optionally, after the inner tube 102 passes through the threading member 101 via the passageway 112, it is fixedly connected to the threading member 101 by adhesive bonding.

As shown in FIG. 3, as an embodiment, the positioning area 111 is provided with a positioning hole extending along the axial direction of the threading member 101. The positioning hole is parallel to the extending direction of the passageway 112, so that one end of the suture end positioning member 105 can be stably inserted into the positioning hole. When the suture end positioning member 105 is inserted into the positioning hole, it is parallel to the inner tube 102, facilitating medical staff to pull the suture end positioning member 105 out of the positioning hole.

As shown in FIGS. 2 and 3, an embodiment of the present application introduces a threading member 101 for an interventional delivery device for a heart valve prosthesis. The threading member 101 is installed on the delivery device described above. The threading member 101 is provided with a passageway 112 extending through along the axial direction of the threading member 101 and coaxial with the threading member 101, enabling the inner tube 102 to pass through the passageway 112 and be fixedly connected to the threading member 101. Meanwhile, the threading member 101 is further provided with threading through holes 114 for guiding the traction member 106 to pass through, so that the traction member 106 can pass through the threading through holes 114 to connect with the heart valve prosthesis 104. Additionally, the threading member 101 is further provided with a positioning area 111. Through the provision of the positioning area 111, one end of the suture end positioning member 105 can be inserted into the positioning area 111 and is detachably connected to the threading member 101 via the positioning area 111. When the suture end positioning member 105 is inserted into the positioning area 111, the pulling member 106 restricts the heart valve prosthesis 104 to prevent the heart valve prosthesis 104 from freely deploying. During the deployment process, when the implantation position is accurate, the suture end positioning member 105 is pulled out from the positioning area 111, causing the pulling member 106 to disengage from the suture end positioning member 105, thereby enabling the deployment of the heart valve prosthesis 104. Simultaneously, the pulling member 106 and the suture end positioning member 105 are retrieved.

As shown in FIG. 2, as an embodiment, the threading member 101 is provided with an abutment platform 115 for abutting against the heart valve prosthesis 104. By configuring the abutment platform 115, when the distal end of the heart valve prosthesis 104 is in the expanded state, the proximal end of the heart valve prosthesis 104 can be pulled by the pulling member 106, causing the heart valve prosthesis 104 to move toward the threading member 101 and abut against the abutment platform 115. The abutment platform 115 plays a role in limiting and supporting the heart valve prosthesis 104. When the heart valve prosthesis 104 is not accurately implanted in the correct position, the heart valve prosthesis 104 is pulled by the pulling member 106 to abut against the abutment platform 115, and then the outer tube 103 is moved distally to retrieve the heart valve prosthesis 104. After the medical staff adjusts the implantation position, the heart valve prosthesis 104 is redeployed to improve the implantation accuracy of the heart valve prosthesis 104 and enhance the success rate of the surgery.

As shown in FIG. 4, as one embodiment, the abutment platform 115 includes an outer ring portion 1151 extending radially outward along the threading member 101. By providing the outer ring portion 1151, an abutment platform for the heart valve prosthesis 104 is provided, so that the heart valve prosthesis 104 can stably abut against the outer ring portion 1151, and meanwhile, the heart valve prosthesis 104 is prevented from passing through the outer circumference of the outer ring portion 1151. The abutment platform 115 further includes a tapered portion 1152 gradually tapering toward the proximal end from the outer ring portion 1151. The tapered portion 1152 is of a tapered structure, which reduces the blockage of blood and ensures blood fluidity.

As shown in FIG. 4, as an embodiment, the tapered portion 1152 is provided with a plurality of threading through holes 114 penetrating the abutment platform 115. The threading through holes 114 penetrating the abutment platform 115 facilitate the traction member 106 to pass through, avoiding the traction member 106 from forming bending angles that may hinder medical staff operation.

As an embodiment, the number of the threading through holes 114 is three, and the three threading through holes 114 are circumferentially arranged along the tapered portion 1152, so as to ensure that the traction members 106 in the threading through holes 114 do not interfere with each other during operation, and each traction member 106 can work independently. This prevents the pulling members 106 from tangling with each other when pulling the heart valve prosthesis 104, thereby avoiding any impact on the surgical process and improving the success rate of the surgery. When medical staff need to finely adjust the heart valve prosthesis 104, a certain one or more traction members 106 can be individually controlled to adjust the heart valve prosthesis 104 to the correct position, so as to improve the implantation accuracy of the heart valve prosthesis 104.

Optionally, the three threading through holes 114 are evenly distributed along the circumferential direction of the tapered portion 1152, so as to facilitate medical staff to select a suitable angle to adjust the position of the heart valve prosthesis 104 through the traction members 106 and improve the convenience of operation for medical staff. In addition, the three threading through holes 114 are arranged through along the axial direction of the threading member 101 to guide blood flow, reduce the blockage of the threading member 101 to blood, and further improve the operation success rate.

As shown in FIG. 4, as an embodiment, the threading member 101 includes a plurality of support tabs 116. The support tabs 116 are arranged to extend toward the distal end along the end surface of the outer ring portion 1151. An accommodation space 118 is formed between two adjacent support tabs 116, and the accommodation space 118 is communicated with the threading through hole 114, so as to ensure that the connection ring set 141 of the heart valve prosthesis 104 is adjusted in the corresponding accommodation space 118 during the process of retrieving the heart valve prosthesis 104 and avoid being affected by other parts of the heart valve prosthesis 104. At the same time, during the process of retrieving the heart valve prosthesis 104, the corresponding connection ring set 141 of the heart valve prosthesis 104 is located in the accommodation space 118 and abuts against the outer ring portion 1151. The support tabs 116 play a role of spacing, avoid the heart valve prosthesis 104 from affecting each other during the retrieving process, and improve the retrieving success rate.

Optionally, the number of the support tabs 116 is three, and the three support tabs 116 are arranged at an angle of 120°, so as to limit three accommodation spaces 118. Each accommodation space 118 is correspondingly communicated with the threading through hole 114. When the heart valve prosthesis 104 abuts on the abutment platform 115, the support tabs 116 space the connection ring sets 141 on the adjacent heart valve prostheses 104 to avoid the connection ring sets 141 from affecting each other and improve the success rate of retrieving the heart valve prosthesis 104.

As an embodiment, the connection ring is of a circular ring type, and therefore, the threading member 101 is provided with a protrusion 117 for fitting with one side of the connection ring set 141. In this way, after the heart valve prosthesis 104 is retrieved, the traction member 106 applies a pulling force to the heart valve prosthesis 104 in the direction toward the threading through hole 114, so that the connection ring set 141 fits with the protrusion 117 and abuts against the outer ring portion 1151. Furthermore, the protrusion 117 further provides a stable supporting effect for the heart valve prosthesis 104, and meanwhile, prevents the connection ring set 141 from being pulled into the threading through hole 114 by the traction member 106, which would otherwise cause retrieval failure of the heart valve prosthesis 104.

As shown in FIG. 1, as an embodiment, the positioning area 111 is provided with a positioning hole extending along the axial direction of the threading member 101. The positioning hole is parallel to the extending direction of the passageway 112, so that one end of the suture end positioning member 105 can be stably inserted into the positioning hole. When the suture end positioning member 105 is inserted into the positioning hole, the suture end positioning member 105 is parallel to the inner tube 102, which is convenient for medical staff to pull the suture end positioning member 105 out of the positioning hole.

The embodiment of the present application introduces a retrieval device for recovering connection sutures. The retrieval device is installed at the proximal end of the handle 107, which is convenient for medical staff to hold and operate. On the one hand, the retrieval device can be used to individually and sequentially control the connection sutures 207, so that the connection sutures 207 can tension the heart valve prosthesis, ensuring that the heart valve prosthesis can be re-housed in the delivery device. On the other hand, it can control the deployment process of the heart valve prosthesis to improve the implantation accuracy of the heart valve prosthesis. Furthermore, after the heart valve prosthesis is deployed, the connection sutures 207 can be recovered.

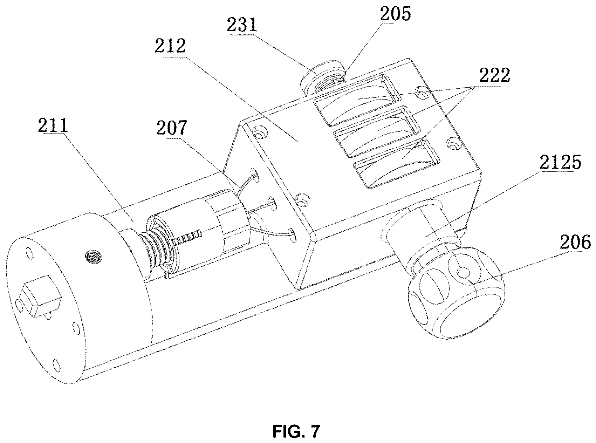

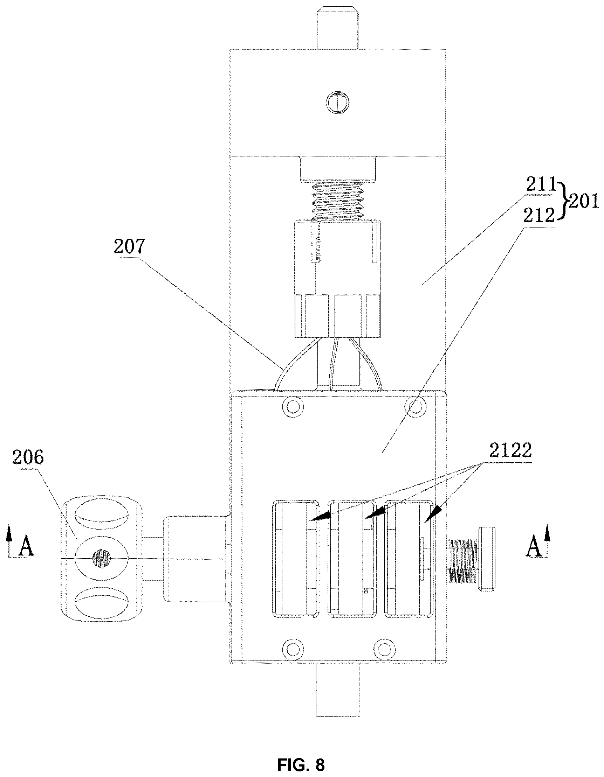

As shown in FIGS. 7 and 8, the retrieval device comprises a body 201 and a suture winding/unwinding assembly assembled on the body 201. The body 201 is connected to the handle 107, and the suture winding/unwinding assembly is used for unwinding or winding the connection suture 207. During the deployment of the heart valve prosthesis, the connection suture 207 needs to be unwound through the suture winding/unwinding assembly. When the heart valve prosthesis needs to be retrieved for redeployment or after the deployment of the heart valve prosthesis is completed, the connection suture 207 needs to be wound up through the suture winding/unwinding assembly.

As shown in FIGS. 7, 9 and 12, the suture winding/unwinding assembly includes a rod 203 rotatable relative to the body 201, and a plurality of single-rotation units 202 arranged on the rod 203 and capable of unidirectional rotation relative to the rod 203. During the unidirectional rotation, the single-rotation units 202 complete the suture winding operation of the connection suture 207. Each single-rotation units 202 is provided with a fixing portion 2221 for connecting with the connection suture 207 to prevent the connection suture 207 from falling off the single-rotation unit 202, and a suture winding portion 2222 around which the connection suture 207 can be wound.

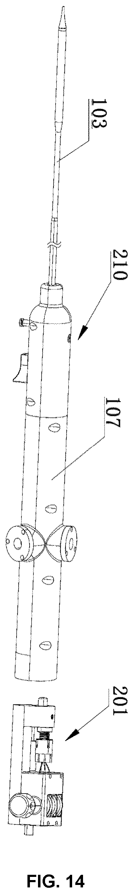

As shown in FIG. 14, the delivery device 210 includes a handle 107, an outer tube 103 and an inner tube. The outer tube and the inner tube form an inner tube assembly. Both the outer tube and the inner tube are connected with the handle 107. The outer tube 103 is sleeved on the outer circumference of the inner tube, and the inner tube extends in the outer tube. A receiving space for accommodating the heart valve prosthesis is provided between the outer tube and the inner tube. When the heart valve prosthesis is not implanted, the heart valve prosthesis is located in the receiving space.

Optionally, each single-rotation unit 202 is respectively connected with a corresponding connection suture 207.

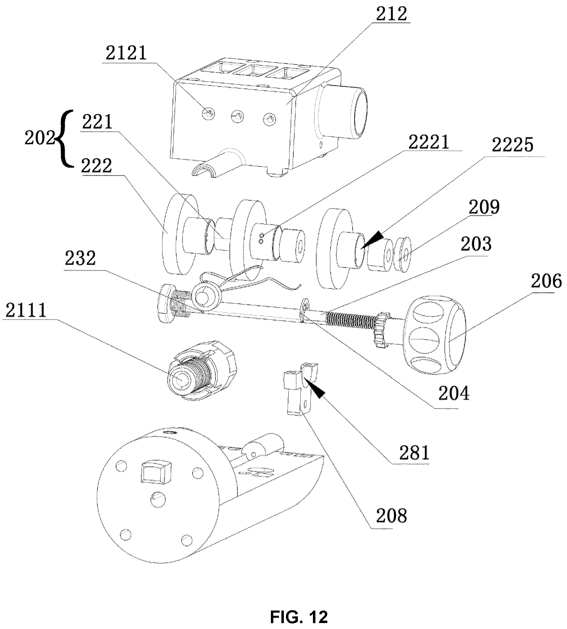

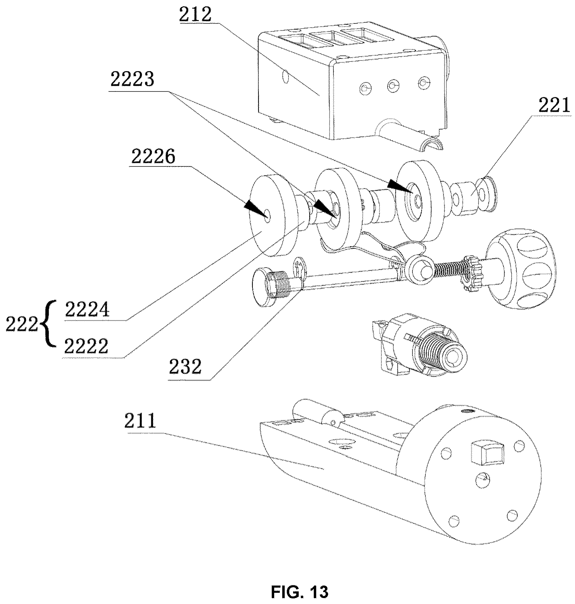

As shown in FIGS. 12 and 13, one end of the connection suture 207 is connected with the single-rotation unit 202 through the retaining portion 2221, and the other end passes through the connection ring on the heart valve prosthesis and is connected with other components on the delivery device 210. When the implantation position of the heart valve prosthesis is inaccurate and the prosthesis needs to be re-housed in the receiving space formed by the inner tube assembly, the multiple single-rotation units 202 can be individually controlled to rotate in one direction relative to the rod 203. This is equivalent to individually tensioning the multiple connection sutures 207 to pull the heart valve prosthesis. During the tensioning process, the connection sutures 207 wind around the suture winding portion 2222, enabling the connection rings on the heart valve prosthesis to be sequentially pulled back to the position abutting against the fixing head on the delivery device, thus completing the retrieval of the heart valve prosthesis and avoiding the situation where simultaneous tensioning of the connection rings of the heart valve prosthesis prevents its retrieval.

Optionally, the unidirectional rotation means that the single-rotation unit 202 can only rotate in a certain direction relative to the rod 203, but cannot rotate in the opposite direction. For example, the single-rotation unit 202 can be set to rotate clockwise relative to the rod 203 and cannot rotate counterclockwise; or the single-rotation unit 202 can be set to rotate counterclockwise relative to the rod 203 and cannot rotate clockwise. The specific rotation direction of the single-rotation unit 202 relative to the rod 203 can be adjusted according to actual use, which is not specifically limited in the embodiment of the present application.

In addition, for the retrieval device in the embodiment of the present application, when the heart valve prosthesis needs to be re-accommodated in the receiving space in the inner tube assembly, the plurality of single-rotation units 202 can be respectively controlled to rotate relative to the rod 203, so that the plurality of connection sutures 207 tighten the heart valve prosthesis, and further, the connection rings of the heart valve prosthesis can be returned to the abutting position of the fixed head one by one, thereby completing the re-housing of the heart valve prosthesis. Certainly, the implantation accuracy of the heart valve prosthesis can be further improved. For example, when the implantation position of the heart valve prosthesis is incorrect, since the proximal end of the heart valve prosthesis is restricted by the plurality of connection sutures 207, by rotating one or more single-rotation units 202, the corresponding connection sutures 207 are wound on the suture winding portion 2222, and the corresponding connection rings of the heart valve prosthesis are pulled by one or more connection sutures 207, so as to finely adjust the local position of the heart valve prosthesis, thereby improving the implantation accuracy of the heart valve prosthesis.

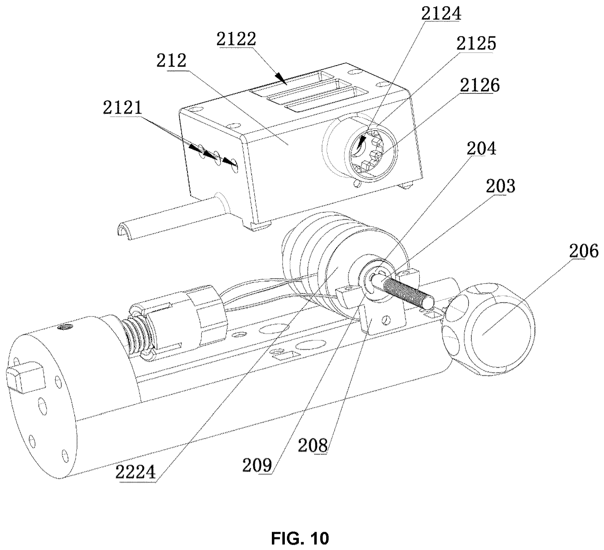

As shown in FIGS. 10 and 12, meanwhile, the rod 203 in the embodiment of the present application can rotate relative to the body 201, and a plurality of single-rotation units 202 are arranged on the rod 203. When the heart valve prosthesis needs to be deployed, by driving the rod 203 to rotate relative to the body 201, the plurality of single-rotation units 202 are synchronously driven to rotate relative to the body 201, so that the multiple connection sutures 207 wound around the corresponding suture winding portions 2222 are in an unwinding state, and the heart valve prosthesis is deployed. After the deployment of the heart valve prosthesis is completed, each connection suture 207 correspondingly connected to the single-rotation unit 202 is separated from the heart valve prosthesis. By driving the rod 203 to rotate relative to the body 201, the plurality of single-rotation units 202 are driven to synchronously rotate relative to the body 201, so that the multiple connection sutures 207 are wound around the corresponding suture winding portions 2222 to complete the suture winding operation. Therefore, the operation time for the retrieval device to wind and unwind the connection sutures 207 is reduced, the operation efficiency is improved, and the operation is convenient for medical staff, that is, only by driving the rod 203 to rotate relative to the body 201, the operation of simultaneously unwinding or winding the multiple connection sutures 207 can be realized.

Optionally, the rod 203 can rotate clockwise relative to the body 201 for suture winding operation and counterclockwise relative to the body 201 for suture unwinding operation; alternatively, the rod 203 can rotate counterclockwise relative to the body 201 for suture winding operation and clockwise relative to the body 201 for suture unwinding operation.

Optionally, the rotation direction of the single-rotation unit 202 relative to the rod 203 for completing the suture winding operation should be consistent with the rotation direction of the rod 203 relative to the body 201 for completing the suture winding operation. For example, if the single-rotation unit 202 rotates clockwise relative to the rod 203 to individually retrieve the corresponding connection suture 207, then the clockwise rotation of the rod 203 relative to the body 201 causes the multiple single-rotation units 202 on the rod 203 to synchronously rotate for suture winding operation, and the counterclockwise rotation of the rod 203 relative to the body 201 causes the multiple single-rotation units 202 on the rod 203 to synchronously rotate for suture unwinding operation.

Optionally, the number of the single-rotation units 202 is three, and each single-rotation unit 202 independently controls the connection suture 207 connected thereto. That is, there are three connection sutures 207, and each connection suture 207 passes through the connection ring of the heart valve prosthesis. When the position of the heart valve prosthesis needs to be finely adjusted, the corresponding single-rotation unit 202 can be individually controlled, so that the corresponding connection suture 207 is wound on the outer surface of the suture winding portion 2222 through the single-rotation unit 202 and pulls the heart valve prosthesis, thereby realizing the fine adjustment of the position of the heart valve prosthesis and adjusting the position or state of the heart valve prosthesis to improve the implantation accuracy.

In some cases, two single-rotation units 202 can also be simultaneously controlled to rotate relative to the rod 203 for simultaneous suture winding.

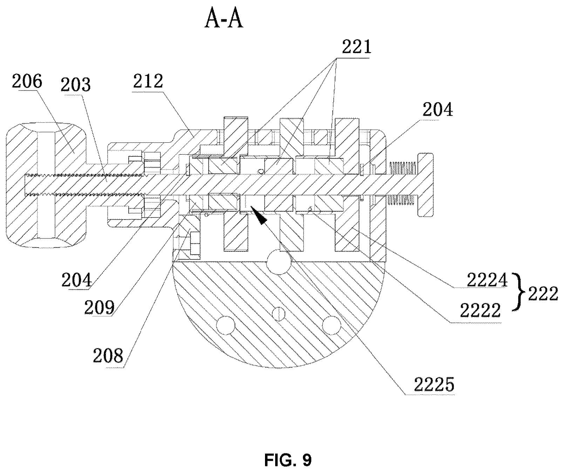

As shown in FIGS. 9, 11 to 13, as an embodiment, the single-rotation unit 202 includes a unidirectional rotating member 221 and an operating member 222 cooperatively connected with the unidirectional rotating member 221. The unidirectional rotating member 221 is installed on the outer surface of the rod 203 and can rotate unidirectionally relative to the rod 203, that is, it can only rotate in a certain direction relative to the rod 203 and is locked and cannot rotate in the opposite direction. Meanwhile, the operating member 222 is connected with the unidirectional rotating member 221 and assembled on the rod 203 through the unidirectional rotating member 221, so that medical staff can drive the operating member 222 to make the operating member 222 and the unidirectional rotating member 221 synchronously rotate unidirectionally relative to the rod 203, improving the convenience of operation for medical staff.

As shown in FIGS. 9, 10 and 13, as an embodiment, the operating member 222 includes an operating portion 2224 protruding from the body 201. The operating portion 2224 is disc-shaped and protrudes from the body 201, which is convenient for medical staff to operate. The operating member 222 further includes a suture winding portion 2222 connected with the operating portion 2224. The retaining portion 2221 is located on the suture winding portion 2222, so that the connection suture 207 is connected with the operating member 222 through the retaining portion 2221 and can also be wound on the suture winding portion 2222.

Optionally, the suture winding portion 2222 is of a cylindrical structure and is coaxially arranged with the operating portion 2224. One end of the suture winding portion 2222 is fixedly connected with the operating portion 2224, and the other end protrudes along the axial direction of the operating portion 2224, thereby providing a winding space for the connection suture 207 to wind on the outer surface of the suture winding portion 2222.

Optionally, the suture winding portion 2222 and the operating portion 2224 are integrally formed.

Optionally, the operating portion 2224 is of a disc-shaped structure, which is further convenient for medical staff to operate with fingers, improving the operation convenience.

As a possible embodiment, the operating portion 2224 can also be of a polygonal structure, such as a pentagon or a hexagon.

As shown in FIG. 12, as an embodiment, the retaining portion 2221 includes a protrusion fixed with the connection suture 207. That is, the retaining portion 2221 can be a protrusion arranged on the outer surface of the suture winding portion 2222, and one end of the connection suture 207 is wound on the protrusion, thereby realizing the connection between the connection suture 207 and the retaining portion 2221. Certainly, the retaining portion 2221 can also include at least two first through holes arranged on the outer surface of the suture winding portion 2222. That is, one end of the connection suture 207 sequentially passes through the two first through holes and is knotted after passing through the last first through hole, thereby also realizing the connection between the connection suture 207 and the retaining portion 2221.

Certainly, the retaining portion 2221 can also be other structures capable of being fixed with the connection suture 207. For example, the connection suture 207 is first wound around the suture winding portion 2222 for one circle, and then the connection suture 207 is glued to the suture winding portion 2222.

As shown in FIGS. 12 and 13, as an embodiment, the operating member 222 is provided with a mounting recess 2225 penetrating through the operating portion 2224 and the suture winding portion 2222. The mounting recess 2225 is used for installing the unidirectional rotating member 221, so that medical staff can directly drive the operating member 222, and the rotation direction of the driving operating member 222 relative to the rod 203 is the direction in which the unidirectional rotating member 221 can rotate unidirectionally relative to the rod 203, realizing that the unidirectional rotating member 221 and the operating member 222 synchronously rotate in a certain direction relative to the rod 203, and this direction is the direction in which the unidirectional rotating member 221 can rotate unidirectionally relative to the rod 203.

Optionally, since the mounting recess 2225 penetrates through the operating portion 2224 and the suture winding portion 2222, the position of the unidirectional rotating member 221 in the mounting recess 2225 is not fixed. It can be located at the position of the mounting recess 2225 in the suture winding portion 2222 or at the position of the mounting recess 2225 in the operating portion 2224.

As shown in FIG. 13, optionally, a second through hole 2226 is formed in the bottom wall of the mounting groove 2225. The second through hole 2226 is used for the rod 203 to pass through, and also constitutes the mounting recess 2225 penetrating through the operating portion 2224 and the suture winding portion 2222.

As an embodiment, the unidirectional rotating member 221 is a unidirectional bearing. By configuring the unidirectional rotating member 221 as a unidirectional bearing, the unidirectional bearing is mounted on the rod 203 and can rotate unidirectionally relative to the rod 203. It can rotate clockwise relative to the rod 203. When rotating counterclockwise, the unidirectional bearing will lock the rod 203 and prevent rotation. Alternatively, the unidirectional bearing can rotate counterclockwise relative to the rod 203 and lock the rod 203 when rotating clockwise.

Optionally, the outer ring of the unidirectional bearing is fitted to the groove peripheral wall of the mounting groove 2225, so that the unidirectional bearing and the operating member 222 can rotate synchronously relative to the rod 203.

As shown in FIGS. 7, 8 and 12, as an embodiment, the body 201 includes a retrieval handle 211 and a cover 212 detachably connected to the retrieval handle 211, which facilitates the assembly of other components inside the cover 212. The rod 203 penetrates the cover 212 perpendicular to the axial direction of the retrieval handle 211, making it convenient for medical staff to operate-one hand supports the retrieval handle 211, and the other hand drives the rod 203 to rotate, which conforms to ergonomics.

Optionally, the rod 203 is arranged to penetrate the cover 212 vertically to the axial direction of the retrieval handle 211 in the horizontal direction, and the axial direction of the retrieval handle 211 is from the proximal end to the distal end.

As shown in FIGS. 7 and 12, as an embodiment, the suture winding/unwinding assembly includes a flange portion 231 located at one end of the rod 203. The flange portion 231 is fixedly connected to the rod 203. One side of the cover 212 facing the flange portion 231 is provided with a first connection hole 2123. The rod 203 can pass through the first connection hole 2123 and rotate relative to the cover 212, and the diameter of the flange portion 231 is larger than that of the first connection hole 2123, thereby preventing the rod 203 from falling off from the position of the first connection hole 2123.

Optionally, the flange portion 231 and the rod 203 may be integrally formed or connected by screw threads.

As shown in FIG. 10, as an embodiment, the suture winding/unwinding assembly further includes a rotating cap 206 that can drive the rod 203 to rotate. The rotating cap 206 is located at the end opposite to the flange portion 231. On the one hand, it cooperates with the flange portion 231 to prevent the rod 203 from falling off the cover 212. On the other hand, it facilitates medical staff to drive the rod 203 to rotate through the rotating cap 206, improving operational convenience.

Optionally, to further increase the friction between the rotating cap 206 and medical staff, a plurality of grooves are provided on the circumference of the rotating cap 206.

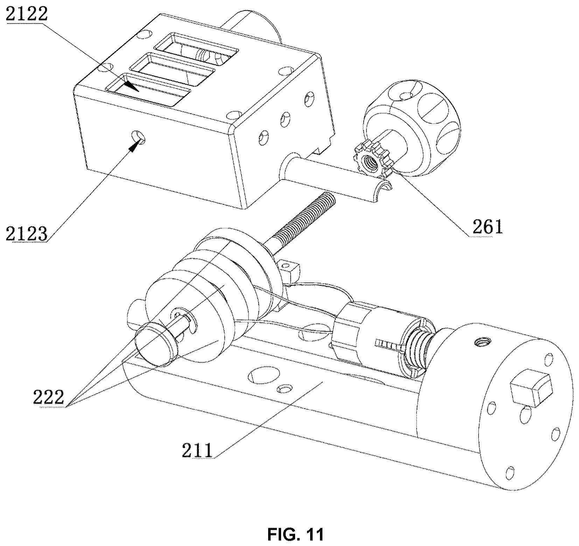

As shown in FIG. 11, as an embodiment, the suture winding/unwinding assembly further includes a locking portion 261 provided on the rotating cap 206 for cooperating with the cover 212 to prevent the rod 203 from accidentally rotating. By arranging the locking portion 261 on the side facing the cover 212, the locking portion 261 cooperates with the cover 212 to keep the rod 203 stationary relative to the cover 212, thereby avoiding accidental rotation of the rod 203 relative to the cover 212 due to medical staff's misoperation and preventing premature deployment of the heart valve prosthesis.

Optionally, the locking portion 261 and the rotating cap 206 may be integrally formed or detachably connected, such as by screw threads or clamping.

As shown in FIG. 10, as an embodiment, the locking portion 261 includes a locking gear, and the cover 212 is provided with a locking tooth groove 2126 that matches the locking gear. That is, the cover 212 includes the locking tooth groove 2126. By engaging the locking gear with the locking tooth groove 2126, accidental rotation of the rod 203 relative to the cover 212 is prevented.

As shown in FIG. 10, as an embodiment, the cover 212 includes a convex cylinder 2125 protruding toward the rotating cap 206 along the extending direction of the rod 203. The inner wall of the convex cylinder 2125 is provided with a locking tooth groove 2126. By arranging the convex cylinder 2125, the locking gear on the rotating cap 206 can easily mesh with the locking tooth groove 2126 on the inner wall of the convex cylinder 2125, further preventing the rod 203 from accidentally rotating.

As shown in FIG. 10, as an embodiment, the convex cylinder 2125 is provided with a second connection hole 2124, which is located on the side opposite to the first connection hole 2123 and is coaxially arranged with the first connection hole 2123, so that the rod 203 can pass through the first connection hole 2123 and the second connection hole 2124 and be threadedly connected to the rotating cap 206, enabling the rotating cap 206 to drive the rod 203 to rotate relative to the cover 212.

As shown in FIG. 7, as an embodiment, a telescopic member 205 is further arranged between the flange portion 231 and the cover 212. Both ends of the telescopic member 205 abut against the flange portion 231 and the cover 212, respectively. When the rod 203 does not need to rotate relative to the cover 212, the elastic force generated by the telescopic member 205 keeps the locking gear always meshed with the locking tooth groove 2126, avoiding accidental operation by medical staff that causes the heart valve prosthesis to be deployed in advance.

Optionally, the telescopic member 205 may be a spring. When the rod 203 needs to rotate relative to the cover 212, that is, when it is necessary to synchronously drive multiple single rotating bodies 20202 to wind or unwind sutures, medical staff directly push the flange portion 231 toward the rotating cap 206. The provided pushing force is greater than the elastic force of the telescopic member 205. Meanwhile, the locking gear separates from the locking tooth groove 2126. At this time, by driving the rod 203 to rotate through the rotating cap 206, the operation of winding or unwinding sutures is realized.

Certainly, in some cases, the rotating cap 206 can be directly pulled out from the convex cylinder 2125 to separate the locking gear from the locking tooth groove 2126. By driving the rod 203 to rotate through the rotating cap 206, the operation of winding or unwinding sutures is realized.

As shown in FIG. 13, as an embodiment, two clamping grooves 232 are arranged on the outer surface of the rod 203 at intervals. The retrieval device further includes two clamping members 204, which are sheet-like structures. The two clamping members 204 are respectively clamped into the clamping grooves 232. The three single rotating bodies 20202 are located between the two clamping members 204. The clamping members 204 limit and block the single rotating bodies 20202 to prevent them from moving axially along the rod 203, which could further affect the suture winding or unwinding operations of the suture winding/unwinding assembly.

As shown in FIGS. 9, 10 and 12, as an embodiment, the three single-rotation units 202 are sequentially arranged along the axial direction of the rod 203. The retrieval device includes a stop block 209 embedded in the suture winding portion 2222 of the operating member 222 adjacent to the rotating cap 206, which is used to block the notch of the mounting recess 2225 of the operating member 222 adjacent to the rotating cap 206 and prevent the unidirectional rotating member 221 in the mounting recess 2225 of the operating member 222 adjacent to the rotating cap 206 from falling off the mounting groove 2225. In addition, the stop block 209 also fits with the clamping member 204 on the corresponding side, increasing the contact area with the clamping member 204 and further improving the stop effect of the clamping member 204.

Optionally, one clamping member 204 fits with the stop block 209, and the other clamping member 204 fits with the operating portion 2224 of the operating member 222 adjacent to the flange portion 231.

As shown in FIG. 13, as an embodiment, multiple single-rotation units 202 are sequentially arranged along the axial direction of the rod 203. The operating member 222 near the rotating cap 206 is the first operating member, the operating member 222 abutted against the first operating member is the second operating member, and the operating member 222 abutted against the second operating member is the third operating member. The operating portions 2224 on the sides of the first and second operating members facing away from the rotating cap 206 are each provided with an insertion recess 2223 adapted to the suture winding portion 2222, so that the suture winding portion 2222 of the second operating member can be inserted into the insertion recess 2223 of the first operating member, and the suture winding portion 2222 of the third operating member can be inserted into the insertion recess 2223 of the second operating member. This shortens the axial distance occupied by the three operating members 222 on the rod 203 to improve space utilization. On the other hand, it prevents the connection sutures 207 corresponding to the operating members 222 from being tangled with other operating members 222, which could lead to abnormal suture winding or unwinding.

Optionally, the operating portion 2224 of the third operating member on the side facing the flange portion 231 has a planar structure without the insertion recess 2223 to increase the contact area with the clamping member 204. The operating portion 2224 of the second operating member on the side facing the flange portion 231 is provided with the insertion recess 2223, and the suture winding portion 2222 of the third operating member is inserted into the insertion recess 2223 of the second operating member, shortening the axial distance between the second and third operating members on the rod 203. Meanwhile, it avoids the connection suture 207 connected to the third operating member from being tangled with the second operating member during suture winding or unwinding, otherwise it may lead to abnormal suture winding/unwinding. Similarly, the operating portion 2224 of the first operating member on the side facing the flange portion 231 is also provided with the insertion recess 2223, and the suture winding portion 2222 of the second operating member is inserted into the insertion recess 2223 of the first operating member, shortening the axial distance between the first and second operating members on the rod 203, and avoiding the connection suture 207 connected to the second operating member from being tangled with the first operating member during suture winding or unwinding, otherwise it may lead to abnormal suture winding/unwinding.

Optionally, the designation of first, second, and third operating members is only for facilitating the description of the three operating members 222 on the rod 203, and do not specifically limit the installation positions of the three operating members 222 on the rod 203. That is, the operating member 222 near the flange portion 231 may be not provided with the insertion recess 2223, while the operating member 222 near the rotating cap 206 and the abutted operating member 222 may be provided with the insertion recess 2223.

As shown in FIGS. 7 and 11, as an embodiment, the cover 212 is provided with multiple movable openings 2122. The cover 212 is provided with a move chamber, which is communicated with the movable openings 2122. The number of the movable openings 2122 corresponds to the number of the single-rotation units 202. Each operating portion 2224 is partially protruded from the movable opening 2122 to facilitate medical staff to drive the operating portion 2224, and the other part of each operating portion 2224 is located in the move chamber.

As shown in FIG. 12, as an embodiment, the retrieval handle 211 is provided with a threading passage 2111 for guiding the connection suture 207. The distal side of the cover 212 is further provided with threading holes 2121. The number of the threading holes 2121 is three, and the three threading holes 2121 are all communicated with the move chamber. Each connection suture 207 passes through the threading passage 2111 and the corresponding threading hole 2121, and is correspondingly connected to the retaining portion 2221 on the three suture winding portions 2222 in the move chamber.

As shown in FIGS. 9 and 12, as an embodiment, the retrieval device includes a support block 208, which is arranged in the move chamber and fixedly connected to the cover 212 by bolts. The support block 208 is used to support the operating member 222 near the rotating cap 206. Specifically, the support block 208 is provided with a notch 281, which is adapted to the suture winding portion 2222 of the operating member 222 near the rotating cap 206, so that the suture winding portion 2222 of the operating member 222 near the rotating cap 206 can be embedded in the notch 281. The support block 208 not only supports the operating member 222 near the rotating cap 206 but also prevents the connection suture 207 connected to the operating member 222 near the rotating cap 206 from being stuck by other components during suture winding or unwinding, which may lead to the failure of suture winding or unwinding functions.

As shown in FIG. 14, an embodiment of the present application introduces a delivery device for a heart valve prosthesis, including a handle 107 and the aforementioned connection suture retrieval device for the heart valve prosthesis. The body 201 is arranged at the proximal end of the handle 107 to facilitate operation by medical staff.

As an embodiment, the body 201 is detachably connected to the handle 107 to facilitate the assembly between the delivery device 210 and the handle 107.

Optionally, the distal end of the body 201 is provided with a convex block, and the proximal end of the handle 107 is provided with a slot. The convex block is correspondingly inserted into the slot to realize the detachable connection between the body 201 and the handle 107. Certainly, the retrieval handle 211 can also be connected to the handle 107 by bolts, that is, the handle 107 and the retrieval handle 211 are fixed by bolts along the axial direction of the retrieval handle 211 and the handle 107.

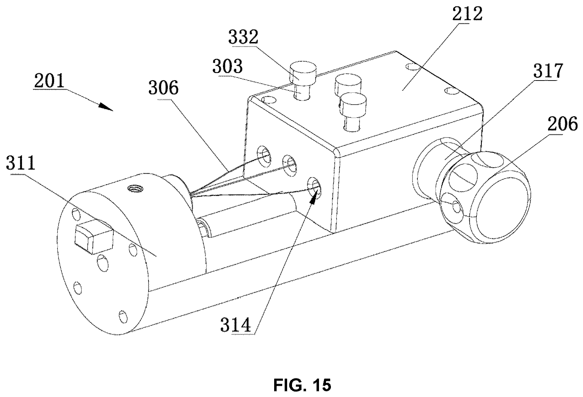

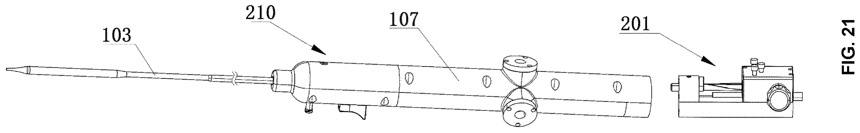

As shown in FIGS. 15 and 21, an embodiment of the present application introduces a retrieval device for a retrieval suture of a heart valve prosthesis, which is installed at the proximal end of the handle 107 of the delivery device 210 to facilitate operation by medical staff holding it. The retrieval device for a retrieval suture can, on the one hand, adjust the corresponding retrieval suture 306 through the auxiliary adjusting member to further fine-tune the position of the heart valve prosthesis to improve the implantation accuracy of the heart valve prosthesis; on the other hand, it can deploy the heart valve prosthesis by rotating the main rotating member relative to the body 201 and retrieve the retrieval suture 306 after the heart valve prosthesis is deployed.

As shown in FIG. 21, the retrieval device for a retrieval suture includes a body 201 and a suture winding/unwinding assembly arranged on the body 201. The body 201 is connected to the handle 107, and the suture winding/unwinding assembly is used to control the retrieval suture 306, that is, to control the deployment or retraction of the retrieval suture 306. During the deployment of the heart valve prosthesis, the retrieval suture 306 needs to be deployed through the suture winding/unwinding assembly; when the heart valve prosthesis needs to be retrieved for redeployment or after the heart valve prosthesis is deployed, the retrieval suture 306 needs to be retracted through the suture winding/unwinding assembly. This realizes the deployment of the heart valve prosthesis and the retrieval of the retrieval suture 306 after deployment, facilitating the operation by medical staff and improving the success rate of the surgery.

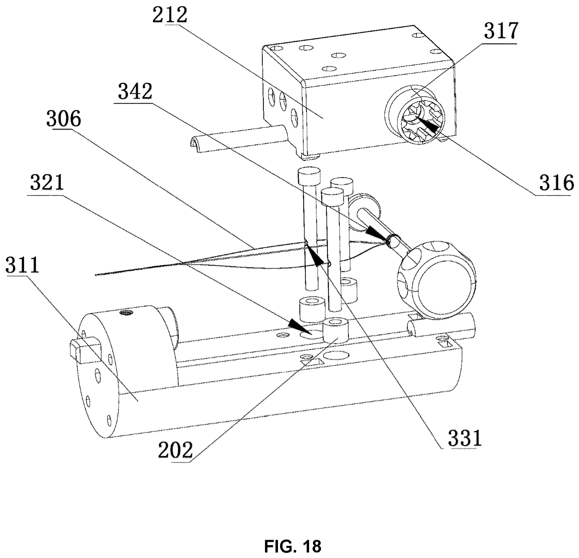

As shown in FIGS. 18 to 20, the suture winding/unwinding assembly includes a main rotating member, which can rotate relative to the body 201 and synchronously drive multiple retrieval sutures 306 during rotation. Multiple retrieval sutures 306 are all connected to the main rotating member to avoid the retrieval sutures 306 from falling off.

The body 201 extends from the distal end to the proximal end, and the axial direction of the body 201 is consistent with the extending direction of the body 201.

Each retrieval suture 306 passes through the heart valve prosthesis. The main rotating member includes a suture winding portion 304. By providing the suture winding portion 304, during the deployment of the heart valve prosthesis by the delivery device, the main rotating member rotates clockwise relative to the body 201 in the horizontal direction perpendicular to the axial direction of the body 201, and the retrieval suture 306 unwinds from the suture winding portion 304. When the heart valve prosthesis needs to be recovered or after the deployment of the heart valve prosthesis is completed, the main rotating member rotates counterclockwise relative to the body 201, and the retrieval suture 306 is wound around the outer circumference of the suture winding portion 304, which is convenient for medical staff to operate, so that medical staff can realize the deployment and retrieval of the heart valve prosthesis only by operating the main rotating member.

Certainly, the main rotating member may also rotate counterclockwise relative the body in a horizontal plane perpendicular to the axial direction of the body 201, causing the retrieval suture 306 to unwind from the suture winding portion 304. When the heart valve prosthesis needs to be recovered or after the deployment of the heart valve prosthesis is completed, the main rotating member rotates clockwise relative to the body 201, and the retrieval suture 306 is wound around the outer circumference of the suture winding portion 304, which is not specifically limited in the embodiment of the present application.

The suture winding/unwinding assembly further includes an auxiliary adjusting member, which can drive a corresponding single retrieval suture 306. The auxiliary adjusting member includes a suture adjusting portion 303. When the implantation position of the heart valve prosthesis is inaccurate, adjusting the suture adjusting portion 303 enables it to apply tension to the retrieval suture 306, thereby fine-tuning the local position of the heart valve prosthesis to improve the implantation accuracy of the heart valve prosthesis. The suture adjusting portion 303 and the suture winding portion 304 are located at different positions along the axial direction of the body 201 to prevent interference: when the suture adjusting portion 303 is in operation, the suture winding portion 304 does not interfere with it; conversely, when the suture winding portion 304 is working, the suture adjusting portion 303 does not interfere with it. This ensures that the suture adjusting portion 303 can smoothly adjust the position of the heart valve prosthesis, and the retrieval suture 306 can unwind from or wind around the suture winding portion 304.

Optionally, the suture adjusting portion 303 and the suture winding portion 304 are spaced apart along the body 201 from the distal end to the proximal end, with the suture adjusting portion 303 positioned closer to the distal end of the body 201 and the suture winding portion 304 closer to the proximal end. This configuration ensures that when the suture winding portion 304 unwinds or winds the retrieval suture 306, the retrieval suture 306 moves through the first through hole 31 of the suture adjusting portion 303 without interference from the suture adjusting portion 303.

Optionally, the number of the retrieval sutures 306 corresponds to the number of the auxiliary adjusting members, and the multiple auxiliary adjusting members respectively adjust a retrieval suture 306 correspondingly.

As shown in FIG. 21, the delivery device 210 includes a handle 107, an outer tube 103 and an inner tube. The outer tube 103 and the inner tube form an inner tube assembly. Both the outer tube 103 and the inner tube are connected with the handle 107. The outer tube 103 is sleeved on the outer circumference of the inner tube, the inner tube extends in the outer tube 103, and a receiving space for accommodating the heart valve prosthesis is provided between the outer tube 103 and the inner tube. When the heart valve prosthesis is not implanted, the heart valve prosthesis is located in the receiving space.

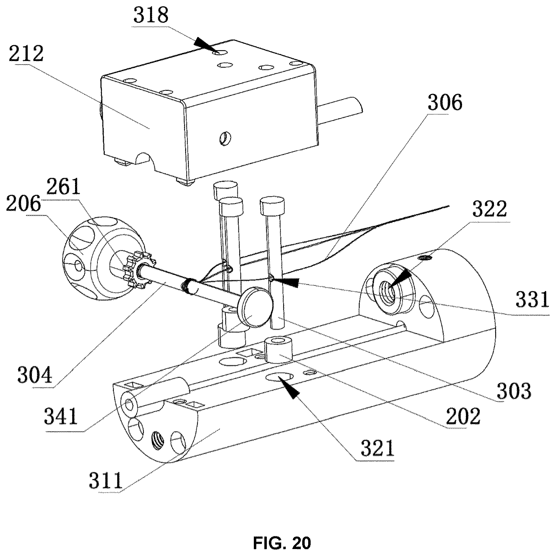

As shown in FIG. 20, one end of the retrieval suture 306 passes through the first through hole 31 to be connected with the suture winding portion 304, and the other end passes through the connection ring on the heart valve prosthesis and is connected with other components on the delivery device 210. When the implantation position of the heart valve prosthesis is inaccurate and the heart valve prosthesis needs to be re-accommodated in the receiving space formed by the inner tube assembly, the main rotating member is controlled to rotate relative to the body 201, so that the retrieval suture 306 is wound on the outer surface of the suture winding portion 304 to tighten the heart valve prosthesis, so that the connection ring on the heart valve prosthesis can be pulled back to the position abutting against the fixed head on the delivery device 210, and the retrieval of the heart valve prosthesis is completed.

As shown in FIG. 20, as an embodiment, the suture adjusting portion 303 is provided with a first through hole 31 for a retrieval suture 306 to pass through. The suture adjusting portion 303 can rotate relative to the body 201, so that the retrieval suture 306 generates a pulling force on the heart valve prosthesis to adjust the position or state of the heart valve prosthesis.

Optionally, the suture adjusting portion 303 is columnar, and the rotation angle of the suture adjusting portion 303 relative to the body 201 is constrained, that is, it cannot rotate more than 180 degrees to make the retrieval suture 306 wind around the suture adjusting portion 303 for one circle. If the suture adjusting portion 303 rotates more than 180 degrees relative to the body 201, the main rotating member will fail and cannot complete the suture winding/unwinding operations of the retrieval suture 306. As a preferred implementation manner, the rotation angle of the suture adjusting portion 303 relative to the body 201 is less than 90 degrees, so that the local part of the corresponding retrieval suture 306 conforms to the local outer circumference of the suture adjusting portion 303 to provide a tightening force to the retrieval suture 306, and the retrieval suture 306 further adjusts the position or state of the heart valve prosthesis.

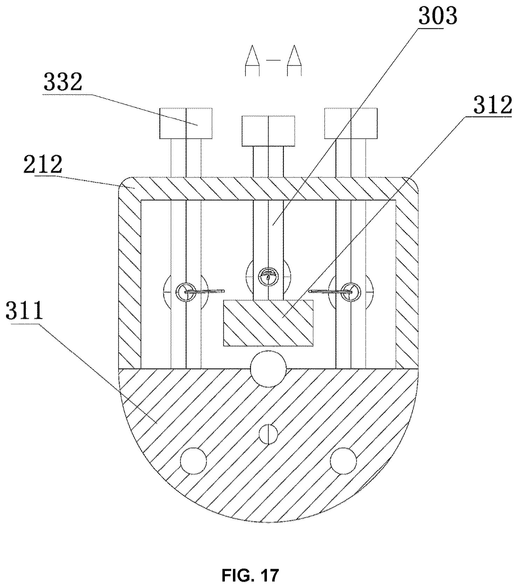

As shown in FIGS. 15 and 17, as an embodiment, the suture adjusting portion 303 is columnar, which is convenient for adjusting the retrieval suture 306. The auxiliary adjusting member includes a drive portion 332, which is fixedly connected with the suture adjusting portion 303. The radial dimension of the drive portion 332 is larger than that of the suture winding portion 304, so that medical staff can conveniently operate the drive portion 332 to make the auxiliary adjusting member rotate relative to the body 201 to finely adjust the position of the heart valve prosthesis.

Optionally, the drive portion 332 and the suture adjusting portion 303 can be integrally formed or connected by screw threads.

As shown in FIG. 18, as an embodiment, the auxiliary adjusting member is installed on the body 201 through the single-rotation unit 202, and the single-rotation unit 202 is located on the opposite side of the drive portion 332. By setting the single-rotation unit 202, the auxiliary adjusting member can rotate unidirectionally relative to the body 201 and correspondingly tighten the retrieval suture 306 to realize the fine adjustment of the local position of the heart valve prosthesis.

Optionally, unidirectional rotation means that the auxiliary adjusting member can rotate in one direction relative to the body 201 through the single-rotation unit 202, but cannot rotate in the opposite direction. For example, the auxiliary adjusting member can rotate clockwise relative to the body 201 in the vertical direction, but cannot rotate counterclockwise; or the auxiliary adjusting member can rotate counterclockwise relative to the body 201 in the vertical direction, but cannot rotate clockwise. The specific rotation direction of the auxiliary adjusting member relative to the body 201 can be adjusted according to actual use, which is not specifically limited in this embodiment.

Optionally, the single-rotation unit 202 is a unidirectional bearing. The inner ring of the unidirectional bearing is tightly connected with the suture adjusting portion 303, and the outer ring is connected with the body 201. Setting the single-rotation unit 202 as a unidirectional bearing can, on the one hand, enable the auxiliary adjusting member to rotate unidirectionally relative to the body 201 along the set direction of the unidirectional bearing. On the other hand, after the auxiliary adjusting member rotates unidirectionally relative to the body 201 through the unidirectional bearing, if the auxiliary adjusting member rotates in the opposite direction, the unidirectional bearing will have a locking function, that is, the unidirectional bearing will lock the auxiliary adjusting member to prevent the failure of adjusting the position of the heart valve prosthesis due to the rotation of the auxiliary adjusting member. In addition, it is convenient for medical staff to operate. After the auxiliary adjusting member rotates relative to the body 201, no more medical staff are required to control the auxiliary adjusting member.

Optionally, reverse rotation means that the auxiliary adjusting member rotates in a certain direction relative to the body 201 and then rotates in the opposite direction.

As an embodiment, the number of auxiliary adjusting members is three, and each auxiliary adjusting member respectively controls a retrieval suture 306. Each retrieval suture 306 passes through the connection ring of the heart valve prosthesis. When the position of the heart valve prosthesis needs to be finely adjusted, the corresponding auxiliary adjusting member can be individually controlled, so that a retrieval suture 306 passing through the auxiliary adjusting member generates a pulling force, and further pulls the connection ring of the heart valve prosthesis to realize the fine adjustment of the position of the heart valve prosthesis, to adjust the position or state of the heart valve prosthesis, and improve the implantation accuracy.

As an embodiment, the three auxiliary adjusting members are arranged in a staggered manner, so that each auxiliary adjusting member has an independent operation space, which is convenient for medical staff to operate each auxiliary adjusting member individually.

As an embodiment, the three auxiliary adjusting members include a first auxiliary adjusting member and two second auxiliary adjusting members, wherein the first auxiliary adjusting member and the two second auxiliary adjusting members are arranged at intervals along the direction from the distal end to the proximal end of the body 201, so that the first auxiliary adjusting member and the two second auxiliary adjusting members are arranged in a staggered manner to leave an operation space. At the same time, the distances from the first auxiliary adjusting member to the two second auxiliary adjusting members are equal, so that the main rotating member can ensure the symmetry of the deployment of the heart valve prosthesis during the suture unwinding or retraction process, and improve the implantation accuracy.

Optionally, the first auxiliary adjusting member is arranged near the distal end of the body 201, and the two second auxiliary adjusting members are arranged near the proximal end of the body 201. Certainly, the first auxiliary adjusting member can also be arranged near the proximal end of the body 201, and the two second auxiliary adjusting members can be arranged near the distal end of the body 201.

As a possible embodiment, the three auxiliary adjusting members can also be arranged at intervals in a straight line perpendicular to the axial direction of the body 201.

As shown in FIG. 20, as an embodiment, the suture winding portion 304 and the suture adjusting portion 303 are installed on the body 201 perpendicular to each other, so that the suture winding portion 304 and the suture adjusting portion 303 can work independently and are convenient for medical staff to operate.

Optionally, the suture winding portion 304 is arranged perpendicular to the axial direction of the body 201 in the horizontal direction, and the suture adjusting portion 303 is arranged perpendicular to the axial direction of the body 201 in the vertical direction, so that medical staff can operate the suture winding portion 304 and the suture adjusting portion 303 at different angles. At the same time, it can ensure that the suture winding portion 304 and the suture adjusting portion 303 do not affect each other during work, which is convenient for medical staff to operate.

As shown in FIG. 20, as an embodiment, the body 201 includes a retrieval handle 311 and a cover 212 detachably connected with the retrieval handle 311, which is convenient for assembling other parts in the cover 212. The suture winding portion 304 penetrates the cover 212 perpendicular to the axial direction of the retrieval handle 311, which is convenient for medical staff to operate, that is, one hand holds the retrieval handle 311, and the other hand drives the suture winding portion 304 to rotate, which conforms to ergonomics. In addition, the three auxiliary adjusting members are arranged perpendicular to the axial direction of the cover 212, which is also convenient for medical staff to operate.

Optionally, the cover 212 is provided with three third through holes 318 for guiding the suture adjusting portion 303 to pass through.

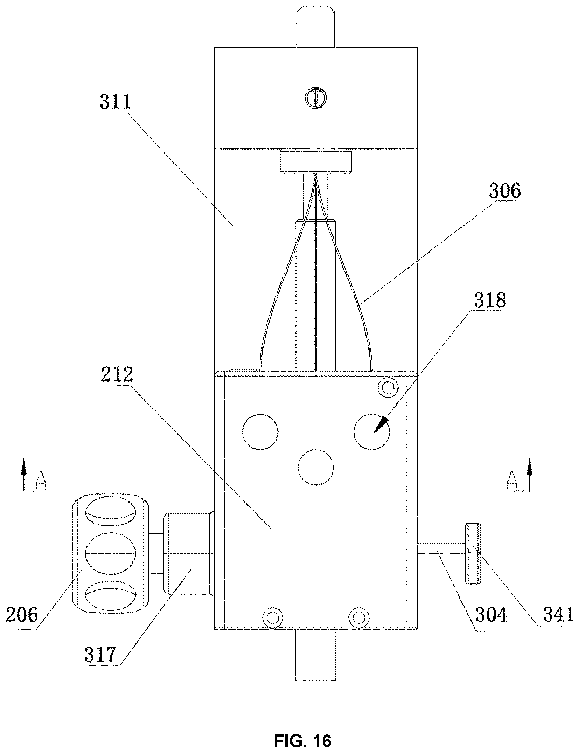

As shown in FIGS. 16 and 20, as an embodiment, the main rotating member includes a rotating cap 206 that can drive the suture winding portion 304 to rotate. The rotating cap 206 is located at one end of the suture winding portion 304, which is convenient for medical staff to drive the suture winding portion 304 to rotate through the rotating cap 206 and improves operation convenience.

Optionally, in order to further increase the friction between the rotating cap 206 and the hands of the medical staff, a plurality of grooves are provided on the circumference of the rotating cap 206.

Optionally, the rotating cap 206 is threadedly connected with the suture winding portion 304.

As shown in FIG. 20, as an embodiment, the main rotating member further includes a locking portion 261 provided on the rotating cap 206 for cooperating with the cover 212 to prevent the suture winding portion 304 from accidentally rotating. By setting the locking portion 261, which is arranged on the side of the rotating cap 206 facing the cover 212, the locking portion 261 cooperates with the cover 212 to keep the suture winding portion 304 stationary relative to the cover 212, avoiding accidental operation by medical staff that causes the suture winding portion 304 to rotate relative to the cover 212, thus avoiding premature deployment of the heart valve prosthesis.

Optionally, the locking portion 261 and the rotating cap 206 may be integrally formed or detachably connected, such as by screw threads or clamping.

As an embodiment, the locking portion 261 includes a locking gear, and the cover 212 is provided with a locking tooth groove matching the locking gear, that is, the cover 212 includes the locking tooth groove, and the locking gear meshes with the locking tooth groove to prevent the suture winding portion 304 from accidentally rotating relative to the cover 212.

As shown in FIGS. 15 and 16, as an embodiment, the cover 212 includes a convex cylinder 317 protruding toward the rotating cap 206 along the extending direction of the rod. The inner wall of the convex cylinder 317 is provided with a locking tooth groove. By providing the convex cylinder 317, the locking gear on the rotating cap 206 can easily mesh with the locking tooth groove on the inner wall of the convex cylinder 317, further preventing the rod from accidentally rotating.

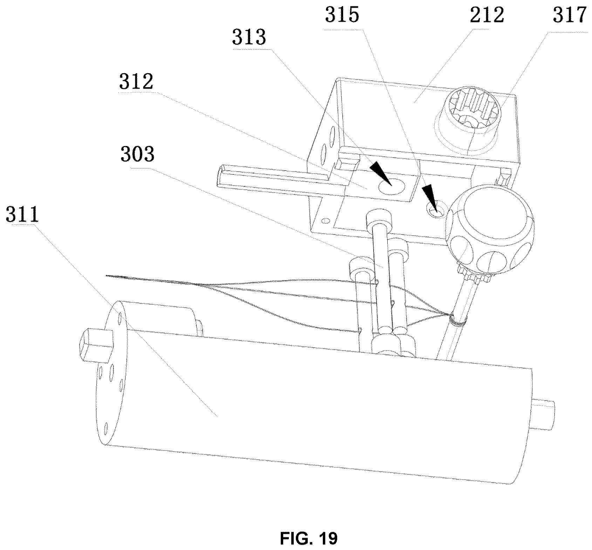

As shown in FIG. 19, as an embodiment, the suture winding portion 304 is of a rod-shaped structure. One side of the cover 212 is provided with a first connection hole 315. The suture winding portion 304 can pass through the first connection hole 315 and rotate relative to the cover 212. The outer surface of the suture winding portion 304 is provided with a protrusion, and one end of the retrieval suture 306 is wound on the protrusion, so as to realize the connection between the retrieval suture 306 and the suture winding portion 304. Certainly, at least two second through holes 342 can also be provided on the outer surface of the suture winding portion 304, that is, one end of the retrieval suture 306 sequentially passes through the two second through holes 342 and is knotted after passing through the last second through hole 342, so as to realize the connection between the retrieval suture 306 and the suture winding portion 304.

As shown in FIGS. 16 and 20, as an embodiment, one end of the suture winding portion 304 is provided with a flange 341. The flange 341 is fixedly connected with the suture winding portion 304. The flange 341 is located on the side opposite to the rotating cap 206, and the diameter of the flange 341 is larger than that of the first connection hole 315, so as to prevent the suture winding portion 304 from falling off from the position of the first connection hole 315.

Optionally, the flange 341 and the suture winding portion 304 may be integrally formed or connected by screw threads.

As shown in FIG. 18, as an embodiment, the convex cylinder 317 is provided with a second connection hole 316, which is located on the side opposite to the first connection hole 315 and is coaxially arranged with the first connection hole 315. This facilitates the suture winding portion 304 to pass through the first connection hole 315 and the second connection hole 316 and be threadedly connected to the rotating cap 206, enabling the rotating cap 206 to drive the suture winding portion 304 to rotate relative to the cover 212.

As shown in FIGS. 17 and 21, as an embodiment, the cover 212 is provided therein with an extension block 312 extending from the distal end to the proximal end. The extension block 312 is provided with a first mounting groove 313 for installing the single-rotation unit 202. The extension block 312 allows the single-rotation unit 202 to be installed in the first mounting groove 313, providing stable support for the auxiliary adjusting member. Meanwhile, the retrieval handle 311 is also provided with two second mounting grooves 321 for installing unidirectional rotating members, enabling the unidirectional rotating members to be installed in the second mounting grooves 321 and providing stable support for the auxiliary adjusting member.

Optionally, since the number of single-rotation units 202 is three, two second mounting grooves 321 are provided on the retrieval handle 311, and one first mounting groove 313 is provided on the cover 212.

As shown in FIG. 20, as an embodiment, the retrieval handle 311 is provided with a threading passage 322 for guiding the retrieval suture 306 to pass through. The distal side of the cover 212 is further provided with multiple threading holes 314. The number of threading holes 314 is three. Each retrieval suture 306 passes through the corresponding threading hole 314 via the threading passage 322, then sequentially passes through the three suture adjusting portions 303, and finally is fixedly connected to the suture winding portion 304.

As shown in FIG. 21, an embodiment of the present application further introduces a delivery device for a heart valve prosthesis, including a handle 107 and the aforementioned retrieval device for a retrieval suture for the heart valve prosthesis. The body 201 is arranged at the proximal end of the handle 107 and is detachably connected to the handle 107, facilitating the assembly between the body 201 and the handle 107.

As an implementation, the delivery device 210 includes a heart valve prosthesis, which includes multiple connection rings. Multiple retrieval sutures 306 respectively pass through the corresponding connection ring sets. The auxiliary adjusting member can rotate a certain angle relative to the body 201 to adjust the position or state of the heart valve prosthesis through the corresponding retrieval suture 306.

Optionally, each connection ring set includes two connection rings. The heart valve prosthesis includes three connection ring sets. One ends of the three retrieval sutures 306 respectively pass through the corresponding connection ring sets and are connected to other components in the delivery device 210, and the other ends pass through the corresponding suture adjusting portions 303 and are finally connected to the suture winding portion 304. By rotating a certain angle of the auxiliary adjusting member relative to the body 201, the position or state of the heart valve prosthesis is adjusted.

Optionally, the auxiliary adjustment component's rotational angle relative to the body 201 is limited to no more than 180 degrees. In a preferred embodiment, the rotational angle is no more than 90 degrees.

When the heart valve prosthesis is in the deployed state but the implantation position is inaccurate, medical staff may need to recapture it into the containment space. In this case, the main rotating component can be driven to rotate relative to the body 201, causing the multiple retrieval sutures 306 to wind around the outer surface of the suture-suture winding portion 304. As the retrieval sutures 306 are tensioned, they exert a pulling force on the proximal end of the heart valve prosthesis, thereby inducing a contracted state at the proximal end. Concurrently, the outer tube 103 is advanced distally relative to the delivery device 210, effectively guiding the heart valve prosthesis back into the containment space.

Certainly, in other scenarios, after the deployment of the heart valve prosthesis is completed, the plurality of retrieval sutures 306 are detached from the prosthesis. Subsequently, by rotating the main rotating component relative to the body 201, the retrieval sutures 306 can be smoothly retracted.

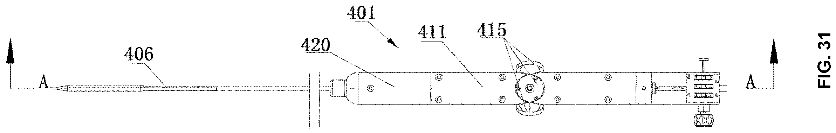

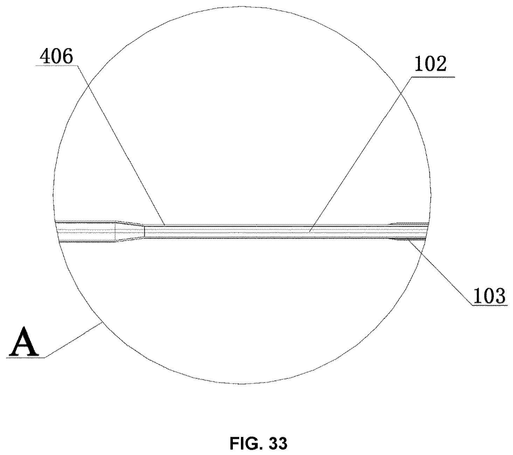

As shown in FIGS. 31 and 33, the present application introduces an interventional delivery device for a heart valve prosthesis, which includes a handle 401, as well as a sheath tube 406 and an inner tube 102 extend in the handle 401, wherein the inner tube 102 extends in the sheath tube 406, and the distal end of the inner tube 102 is connected with a guide head. A receiving space for accommodating the heart valve prosthesis is provided between the sheath tube 406 and the inner tube 102. The sheath tube 406 can move axially relative to the inner tube 102 to deploy or retrieve the heart valve prosthesis.

The sheath tube 406 includes a containment tube section and an extension tube section. The receiving tube segment, located at the distal end of the sheath, has a larger radial dimension than the extension tube segment and is configured to house the heart valve prosthesis.

Optionally, the guide head is of a conical structure. The end of the guide head has a streamlined shape, which can avoid scratching the inner wall of the blood vessel and is beneficial for guiding the entire delivery device to advance along the blood vessel. The tail of the guide head is of a planar structure for abutting against the heart valve prosthesis.

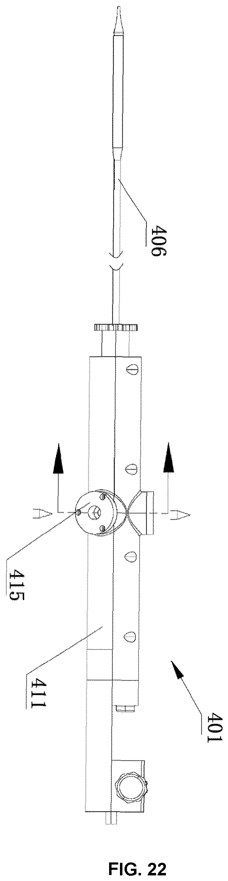

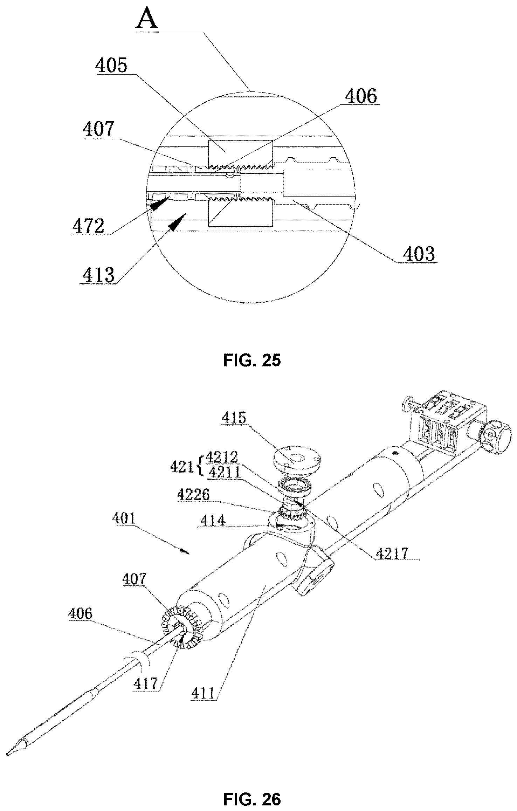

As shown in FIGS. 22 and 26, an embodiment of the present application provides an interventional delivery device for a heart valve prosthesis, which further includes a delivery assembly extending in the handle 401 and axially movable relative to the handle 401. The delivery assembly is used for delivering the heart valve prosthesis, and includes a transmission rod 403 axially movable relative to the handle 401. The delivery device further includes at least two first drive assemblies 421 arranged along the circumference of the transmission rod 403. The first drive assemblies 421 are in transmission connection with the transmission rod 403 to drive the transmission rod 403 to move axially relative to the handle 401. By arranging at least two first drive assemblies 421 along the circumference of the transmission rod 403, after the handle 401 is rotated, medical staff can select one of the first drive assemblies 421 to drive the transmission rod 403 to move axially relative to the handle 401 according to their suitable operation position, facilitating the operation of medical staff.

Optionally, each first drive assembly 421 can drive the transmission rod 403 to move axially relative to the handle 401. The inner tube 102 is connected to the handle 401 by adhesive bonding or other means, and the transmission rod 403 is connected to the sheath tube 406. When the transmission rod 403 moves axially relative to the handle 401, the sheath tube 406 is synchronously driven to move axially relative to the handle 401, so that the sheath tube 406 moves axially relative to the inner tube 102, thereby enabling deployment or retrieval of the heart valve prosthesis.

Optionally, the two first drive assemblies 421 are evenly distributed along the circumference of the transmission rod 403, so that after the handle 401 is rotated, medical staff can select one of the first drive assemblies 421 suitable for their operation to operate, thereby improving the convenience of operation for medical staff.

As shown in FIG. 26, as a preferred implementation, three first drive assemblies 421 are evenly distributed in the circumferential direction of the transmission rod 403, with the axes of adjacent first drive assemblies 421 arranged at 120-degree angles. Since the circumferential rotation angle of the handle 401 is unknown, disposing three first drive assemblies 421 along the circumference of the transmission rod 403 allows medical staff to operate any one of the first drive assemblies 421 to drive the axial movement of the transmission rod 403 relative to the handle 401, thereby enabling deployment or retrieval of the heart valve prosthesis and improving the operational convenience for medical staff using the delivery device.

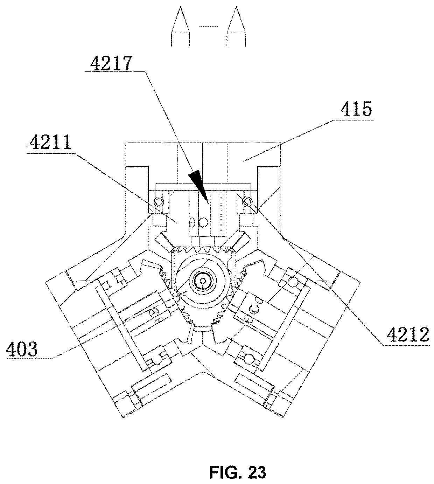

As shown in FIGS. 23, 24, 28 and 29, as an embodiment, the delivery device further includes a second drive assembly 422 arranged on the transmission rod 403. The first drive assembly 421 is in transmission connection with the second drive assembly 422. The first drive assembly 421 is adapted to drive the second drive assembly 422 to rotate, so that the transmission rod 403 moves axially relative to the handle 401, where "adapted" means that the first drive assembly 421 drives the second drive assembly 422 to rotate when the transmission rod 403 needs to move axially relative to the handle 401.

Optionally, the three first drive assemblies 421 are all in transmission connection with the second drive assembly 422. Each first drive assembly 421 can drive the second drive assembly 422 to rotate relative to the transmission rod 403. In addition, when the first drive assembly 421 drives the second drive assembly 422 to rotate, the first drive assembly 421 itself also rotates. Moreover, the three first drive assemblies 421 rotate synchronously.

As shown in FIGS. 23 and 26, as an embodiment, the first drive assembly 421 includes a first drive member 4211 and a first support member 4212 sleeved on the outer circumference of the first drive member 4211. The first support member 4212 is in tight fit connection with the first drive member 4211, so that the first support member 4212 has a clamping force on the first drive member 4211 to ensure structural stability. One end of the first drive member 4211 facing the transmission rod 403 is provided with a first drive portion 4216. The second drive assembly 422 includes a second drive member 4221 and a second support member 4222 sleeved on the outer circumference of the second drive member 4221. The second support member 4222 is in tight fit connection with the second drive member 4221. The distal end of the second drive member 4221 is provided with a second drive portion 4226. The first drive portion 4216 is meshed with the second drive portion 4226. The second drive member 4221 is sleeved on the outer circumference of the transmission rod 403 and is in threaded transmission connection with the transmission rod 403. The first drive member 4211 is adapted to drive the second drive member 4221 to rotate, so that the second drive member 4221 is in transmission relative to the transmission rod 403, and the transmission rod 403 moves axially relative to the handle 401, where "adapted" means that when the transmission rod 403 needs to move axially relative to the handle 401, the first drive member 4211 drives the second drive member 4221 to rotate, so that the transmission rod 403 moves axially relative to the handle 401.

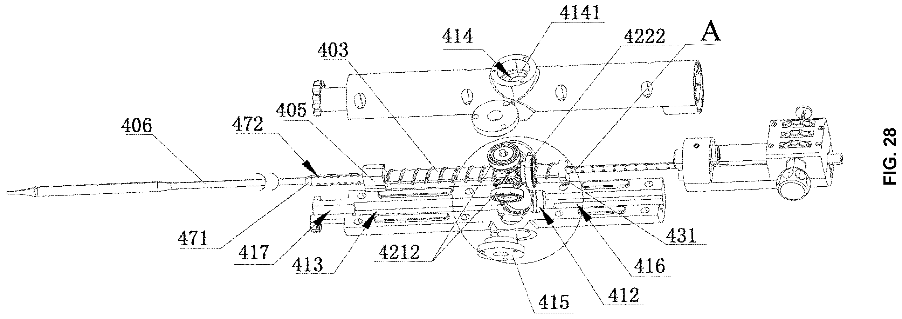

As shown in FIGS. 26 and 28, as an embodiment, the first drive portion 4216 includes multiple first drive teeth, and the second drive portion 4226 includes multiple second drive teeth. Drive tooth grooves are formed between adjacent second drive teeth. The multiple first drive teeth are correspondingly meshed with the multiple drive tooth grooves, so that when the first drive member 4211 rotates, the second drive member 4221 is synchronously driven to rotate.

Optionally, the ends of the three first drive members 4211 are all provided with first drive portions 4216, and the three first drive portions 4216 are all meshed with the second drive portion 4226. At the same time, the axes of the three first drive members 4211 are all perpendicular to the axis of the second drive member 4221, thereby ensuring the meshing angle between the first drive portion 4216 and the second drive portion 4226, so that when the first drive member 4211 rotates around its own axis, it can more smoothly drive the second drive member 4221 to rotate relative to the transmission rod 403.

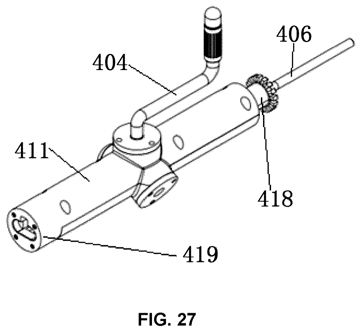

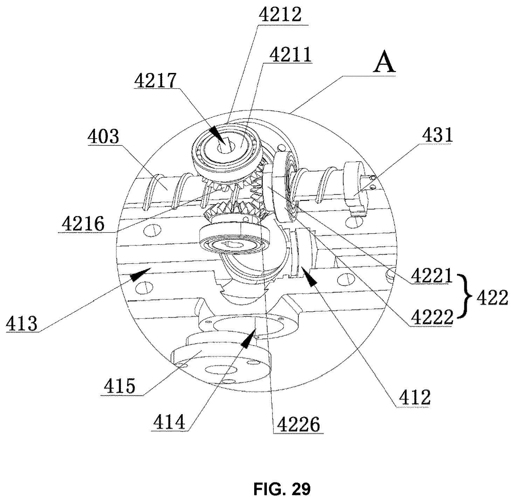

As shown in FIGS. 27 and 29, as an embodiment, a power rod 404 is included. The first drive member 4211 is provided with a recess 4217, which is formed by recessing one end of the first drive member 4211 toward the first drive portion 4216. One end of the power rod 404 can be inserted into the recess 4217 to drive the first drive member 4211 to rotate.

In the implementation of the above solution, the delivery device further includes a power rod 404. The first drive member 4211 is provided with a recess 4217, which is formed by recessing the end of the first drive member 4211 facing away from the first drive portion 4216 toward the first drive portion 4216. One end of the power rod 404 is adapted to the recess 4217. The power rod 404 includes an insertion end that can be inserted into the recess 4217. When the power rod 404 is inserted into the recess 4217, it can drive the first drive member 4211 to rotate, causing the first drive member 4211 to synchronously drive the second drive member 4221 to rotate. When one end of the power rod 404 is separated from the recess 4217, the first drive member 4211 stops rotating.

As shown in FIG. 29, optionally, the three first drive members 4211 are all provided with recesses 4217. The recess 4217 is a columnar chamber 413, and any cross-section of the recess 4217 along its axial direction is a non-circular special shape. The end of the power rod 404 inserted into the recess 4217 is adapted to the structure of the recess 4217, so that the insertion end of the power rod 404 is clamped with the recess 4217.

As shown in FIGS. 22 and 26, as an embodiment, the handle 401 includes a first housing 411. The first housing 411 is provided with a mounting groove 414 corresponding to the first drive assembly 421. The mounting groove 414 is configured to accommodate the first drive assembly 421, so that the first drive assembly 421 can rotate in the mounting groove 414, and the groove peripheral wall of the mounting groove 414 plays a role in stable support for the first drive assembly 421.

An annular boss 4141 is further provided within the mounting groove 414. The first support member 4212 is positioned within the mounting groove 414 and abuts against the annular boss 4141. The first drive member 4211 is in tight fit connection with the first support member 4212, such that when the insertion end of the power rod 404 drives the first drive member 4211 to rotate, the first support member 4212 rotates synchronously with the first drive member 4211.

Optionally, the number of the mounting grooves 414 corresponds to the number of the first drive assemblies 421. The multiple mounting grooves 414 are evenly arranged along the circumference of the first housing 411, so that medical staff can drive the first drive assemblies 421 to rotate at multiple angles, so that the sheath tube 406 moves axially relative to the first housing 411, improving the convenience of operation for medical staff.

Optionally, the first support member 4212 is a ball bearing, including an inner ring and an outer ring, which are in transmission connection via a plurality of balls. The diameter of the inner ring is equal to the radial dimension of the end of the first drive member 4211 facing away from the first drive portion 4216, so that the first support member 4212 can be sleeved on the outer circumference of the first drive member 4211 to achieve a tight fit connection. The outer ring abuts against the annular boss 4141 and is embedded in the mounting groove 414 to ensure the stability of the first support member 4212. When the first drive member 4211 rotates, the inner ring of the first support member 4212 rotates synchronously with the first drive member 4211, and the outer ring of the first support member 4212 is relatively stationary. The first support member 4212 not only provides support for the first drive member 4211, but also ensures the stability of the first drive member 4211 during rotation.

Optionally, the first housing 411 is formed by snapping a first upper housing and a second lower housing, which facilitates the installation and maintenance of other components within the first housing 411. The first upper housing is provided with one mounting groove 414 and also provided with a part of the other two mounting grooves 414, while the remaining parts of the other two mounting grooves 414 are arranged on the second lower housing. By mutually snapping the first upper housing and the second lower housing, three mounting grooves 414 evenly distributed along the circumference of the first housing 411 are formed.

As shown in FIGS. 28 and 29, as an embodiment, a limiting groove 412 is arranged in the first housing 411, and the second support member 4222 is embedded in the limiting groove 412. The first drive member 4211 is suitable for driving the second drive member 4221 to rotate, so that the second support member 4222 and the second drive member 4221 rotate synchronously.

In the implementation of the above solution, a limiting groove 412 is further arranged in the first housing 411, and the second support member 4222 is embedded in the limiting groove 412. The second drive member 4221 is in tight fit connection with the second support member 4222. When the first drive member 4211 drives the second drive member 4221 to rotate, the second support member 4222 provides stable support for the second drive member 4221 to ensure that the second drive member 4221 and the first drive member 4211 rotate synchronously.

Optionally, the second support member 4222 has the same structure as the first support member 4212, and is also a ball bearing, including an inner ring and an outer ring. The outer ring of the second support member 4222 is in close contact with the wall of the limiting groove 412, while the inner ring is in tight fit connection with the second drive member 4221. When the second drive member 4221 rotates, the inner ring of the second support member 4222 rotates synchronously with the second drive member 4221, and the outer ring of the second support member 4222 is relatively stationary. The second support member 4222 not only provides support for the second drive member 4221, but also ensures the stability of the second drive member 4221 during rotation.

Optionally, both the first upper housing and the second lower housing are provided with limiting grooves 412, and the positions of the two limiting grooves 412 correspond to each other. When the first upper housing and the second lower housing are snapped together, the second support member 4222 is accommodated in the limiting groove 412.

As shown in FIGS. 26 to 29, as an embodiment, an end cover 415 for blocking the first support member 4212 is further arranged at the opening position of the mounting groove 414. The end cover 415 is detachably connected with the groove peripheral wall of the mounting groove 414.

In the implementation of the above solution, the end cover 415 is further arranged at the opening position of the mounting groove 414. The end cover 415 is detachably connected with the groove peripheral wall of the mounting groove 414 to protect the first drive assembly 421. At the same time, the end cover 415 plays a role in limiting the first support member 4212 to prevent the first support member 4212 from falling out of the mounting groove 414.

Optionally, the center of the end cover 415 is also provided with a through hole corresponding to the recess 4217, so that the insertion end of the power rod 404 can be inserted into the recess 4217.

The end cover 415 is fastened to the peripheral wall of the mounting groove 414 by multiple bolts. Certainly, it can also be connected by means of clamping or the like.

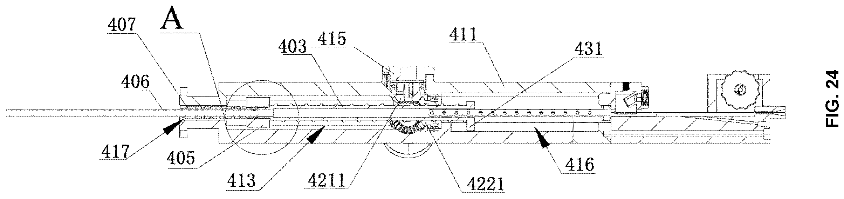

As shown in FIGS. 24 and 25, as an embodiment, a chamber 413 is arranged in the first housing 411. The chamber 413 is communicated with the limiting groove 412, and the chamber 413 extends along the axial direction of the first housing 411.

A transmission member 405 is installed in the chamber 413. The delivery assembly further includes a sheath tube 406. The proximal end of the sheath tube 406 and the distal end of the transmission rod 403 are respectively connected with the transmission member 405. The transmission rod 403 is suitable for moving axially along the chamber 413, so that the transmission member 405 slides along the chamber 413 and drives the sheath tube 406 to move axially relative to the first housing 411.

In the implementation of the above solution, the chamber 413 is arranged in the first housing 411. The chamber 413 is communicated with the limiting groove 412, and at the same time, the chamber 413 extends along the axial direction of the first housing 411. The transmission member 405 is arranged in the first housing 411. The transmission member 405 is installed in the chamber 413 and can move axially relative to the first housing 411 along the chamber 413. The delivery assembly further includes the sheath tube 406. The sheath tube 406 is used for delivering the heart valve prosthesis. The proximal end of the sheath tube 406 and the distal end of the transmission rod 403 are connected through the transmission member 405 and are respectively in threaded connection with the transmission member 405. The transmission rod 403 is adapted to move axially along the chamber 413, and at the same time drives the transmission member 405 and the sheath tube 406 to move axially relative to the first housing 411 synchronously. When the sheath tube 406 moves axially relative to the first housing 411, the deployment or retrieval of the heart valve prosthesis is realized. Here, "adapted to" means that the transmission member 405 only moves axially along the chamber 413 when such axial movement is required.

Optionally, the transmission member 405 is a block-shaped structure, including two opposite arc surfaces. The first upper housing and the second lower housing are respectively provided with recesses. When the first upper housing and the second lower housing are snapped, the two recesses form the chamber 413. The walls of the two recesses in the first upper housing and the second lower housing are respectively adapted to the two arc surfaces, thereby reducing resistance generated when the transmission member 405 moves axially along the chamber 413 and making it easier for the transmission member 405 to move axially along the chamber 413. When the transmission member 405 moves axially along the chamber 413, it simultaneously drives the sheath 406 to move axially, thus enabling deployment of the heart valve prosthesis located within the sheath 406.

As shown in FIGS. 26 and 28, as an embodiment, a reinforcing tube 407 is sleeved on the proximal end of the sheath tube 406. The reinforcing tube 407 is fixedly connected with the sheath tube 406. The sheath tube 406 is a flexible tube. By sleeving the reinforcing tube 407 on the outer circumference of the sheath tube 406, stable support can be provided for the sheath tube 406.

As an embodiment, the reinforcing tube 407 is provided with a plurality of holes 472 to facilitate adhesive application, thereby achieving a fixed connection between the reinforcement tube 407 and the sheath 406.

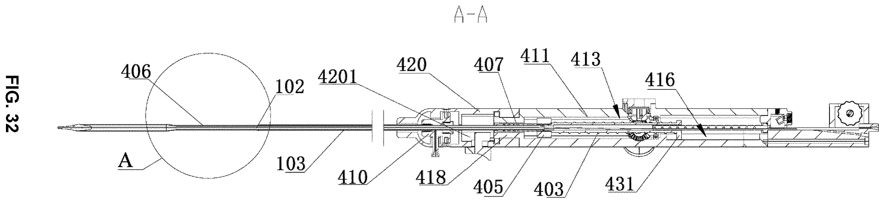

As shown in FIGS. 24, 28 and 32, as an embodiment, a sliding block 431 is arranged at the proximal end of the transmission rod 403. A sliding groove 416 is further arranged in the first housing 411. The sliding block 431 is embedded in the sliding groove 416 and is in sliding fit with the sliding groove 416. A stop portion for stop-limiting engagement with the sliding block 431 is provided at the proximal end of the sliding groove 416.

In the implementation of the above solution, the transmission rod 403 and the transmission member 405 are coaxially arranged. The distal end of the transmission rod 403 is in threaded connection with the transmission member 405. The sliding groove 416 is further arranged in the first housing 411. The sliding groove 416 extends along the axial direction of the first housing 411. The sliding groove 416 is located beside the chamber 413. The sliding groove 416 is the end close to the operator and is arranged at intervals from the chamber 413. The sliding block 431 is arranged at the proximal end of the transmission rod 403. The sliding block 431 is embedded in the sliding groove 416 to provide stable support for the transmission rod 403. At the same time, the sliding block 431 can slide in the sliding groove 416. When the transmission rod 403 is driven, the transmission rod 403 moves axially along the chamber 413 and at the same time drives the transmission member 405 to move axially along the chamber 413. In addition, the stop portion is further arranged at the proximal end of the sliding groove 416. When the sliding block 431 moves axially from the distal end of the sliding groove 416 toward the proximal end of the sliding groove 416, the stop portion can stop the sliding block 431 to limit the movement of the sliding block 431 and prevent the sliding block 431 from falling out of the sliding groove 416.

Optionally, the stop portion is the groove wall at the distal end of the sliding groove 416.

As shown in FIGS. 24 and 26, as an embodiment, a through channel 417 is arranged in the first housing 411. A conical head 471 is arranged at the distal end of the reinforcing tube 407. When the transmission member 405 moves axially along the first housing 411, the conical head 471 at the distal end of the reinforcing tube 407 makes it easier for the reinforcing tube 407 to pass through the through channel 417, improving the operability of the delivery device. At the same time, the through channel 417 is communicated with the chamber 413. By arranging the through channel 417 in the first housing 411, the sheath tube 406 can pass through the through channel 417 and be fixedly connected with the transmission member 405 in the chamber 413.

As an embodiment, the delivery device further includes an outer tube 103. The outer tube 103, the sheath tube 406 and the inner tube 102 are all connected with the handle 401. The outer tube 103 is sleeved on the outer circumference of the sheath tube 406. The sheath tube 406 is sleeved on the outer circumference of the inner tube 102. The inner tube 102 extends in the sheath tube 406. The sheath tube 406 and the inner tube 102 both extend in the outer tube 103 and can rotate circumferentially relative to the outer tube 103 synchronously. When the sheath tube 406 and the inner tube 102 rotate circumferentially relative to the outer tube 103 synchronously, the sheath tube 406 and the inner tube 102 clamp the heart valve prosthesis to rotate circumferentially relative to the outer tube 103 in synchronization, so as to realize the circumferential positioning of the heart valve prosthesis. After the circumferential positioning of the heart valve prosthesis, the prosthesis is deployed to improve the accuracy of the implantation position of the heart valve prosthesis by the delivery device.

Optionally, the proximal ends of the sheath tube 406 and the inner tube 102 are both connected with the handle 401. One end of the extension tube section is connected with the handle 401, and the other end protrudes and extends toward the distal end of the outer tube 103 and is connected with the containing tube section. The containing tube section is located at the distal end of the outer tube 103.

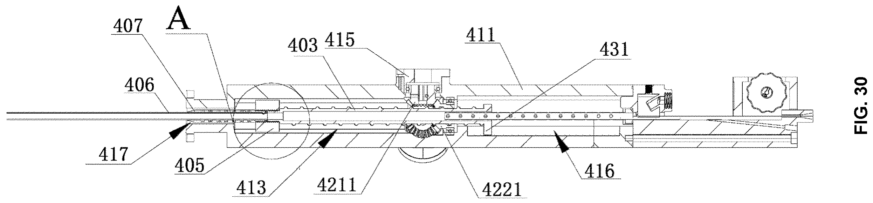

As shown in FIG. 30, as an embodiment, the delivery device further includes a second housing 420. The first housing 411 can rotate circumferentially relative to the second housing 420. When the heart valve prosthesis needs to be positioned circumferentially, the first housing 411 rotates relative to the second housing 420. When deploying or retrieving the heart valve prosthesis, the first housing 411 is stationary relative to the second housing 420 synchronously. A fixing member 410 is arranged in the second housing 420. The proximal end of the outer tube 103 is fixedly connected with the fixing member 410, thereby improving the stability of the outer tube 103. Meanwhile, the outer tube 103 is sleeved on the outer circumference of the sheath tube 406. When both the sheath tube 406 and the outer tube 103 are located inside the human body, the outer tube 103 provides stable support for the sheath tube 406. The proximal end of the sheath tube 406 is connected with the transmission member 405. The inner tube 102 is fixedly connected with the proximal end of the first housing 411. The chamber 413 extends along the axial direction of the first housing 411. The transmission member 405 is embedded in the chamber 413. When the first housing 411 rotates relative to the sheath tube 406, the transmission member 405 and the first housing 411 rotate relative to the second housing 420 synchronously. Since the proximal end of the sheath tube 406 is fixedly connected with the transmission member 405 and the outer tube 103 is fixedly connected with the fixing member 410, when the transmission member 405 rotates relative to the second housing 420, the sheath tube 406 also rotates relative to the second housing 420 and also rotates circumferentially relative to the outer tube 103. In addition, the proximal end of the inner tube 102 is fixedly connected with the proximal end of the first housing 411. Therefore, when the inner tube 102 rotates relative to the sheath tube 406, the inner tube 102 also rotates relative to the second housing 420 and also rotates circumferentially relative to the outer tube 103. Finally, when the first housing 411 rotates relative to the second housing 420, the sheath tube 406 and the inner tube 102 rotate circumferentially relative to the outer tube 103 synchronously. When rotating to the position where the heart valve prosthesis needs to be implanted, the heart valve prosthesis is deployed, thereby improving the accuracy of the implantation position of the heart valve prosthesis.

As shown in FIG. 30, optionally, the second housing 420 is provided with a mounting cavity 4201. The fixing member 410 includes a fixing post and a fixing portion connected to the fixing post. The fixing post is embedded in the mounting cavity 4201 to achieve preliminary fixing of the fixing member 410 and the second housing 420. The fixing portion is provided with at least one fixing hole, and the second housing 420 is provided with at least one fixing mating hole corresponding to the fixing hole. A fastener passes through the fixing mating hole and the fixing hole to fixedly connect the fixing member 410 and the second housing 420. Meanwhile, the proximal end of the outer tube 103 is threadedly connected to the fixing post to achieve fixed connection between the outer tube 103 and the fixing post.

Optionally, the fixing member 410 is provided with a hollow through-channel in the axial direction. Both the sheath tube 406 and the inner tube 102 pass through the hollow through-channel and are respectively connected to the transmission member 405 and the proximal end of the first housing 411. When the first housing 411 rotates circumferentially relative to the second housing 420, the sheath tube 406 and the inner tube 102 can synchronously rotate circumferentially relative to the second housing 420, further realizing the circumferential positioning of the heart valve prosthesis by the delivery device.

As shown in FIGS. 28 and 30, an embodiment of the present application further provides a handle 401 for an interventional delivery device for a heart valve prosthesis, including a first housing 411. The circumferential direction of the first housing 411 is provided with at least two mounting grooves 414 for accommodating the first drive assemblies 421, so that the first drive assemblies 421 can rotate in the mounting grooves 414. The peripheral wall of the mounting groove 414 provides stable support for the first drive assemblies 421, thereby facilitating medical staff to drive the first drive assemblies 421 to rotate from multiple angles and improving the operability of the handle 401.

Optionally, two mounting grooves 414 are evenly distributed in the circumferential direction of the first housing 411, so that after the handle 401 rotates, medical staff can select one suitable angle to operate the first drive assembly 421, improving the convenience of operation for medical staff.

As shown in FIG. 27, as an embodiment, the distal end of the first housing 411 is further provided with a insertion post 418, and the end of the insertion post 418 is provided with multiple teeth, with tooth grooves formed between adjacent teeth.

In the implementation of the above solution, the distal end of the first housing 411 is further provided with a insertion post 418. The handle 401 further includes a second housing 420, and the proximal end of the second housing 420 is provided with an insertion groove corresponding to the insertion post 418. The insertion post 418 is inserted into the second housing 420 to achieve connection between the insertion post 418 and the second housing 420. Meanwhile, the second housing 420 is of a columnar structure. When the insertion post 418 is inserted into the insertion groove, the first housing 411 can rotate circumferentially relative to the second housing 420, and the sheath tube 406 synchronously rotates circumferentially relative to the second housing 420. The sheath tube 406 and the inner tube 102 clamp the heart valve prosthesis, thereby achieving circumferential positioning of the heart valve prosthesis.

Additionally, the end of the insertion groove is provided with multiple teeth, with tooth grooves formed between adjacent teeth. A locking member 4100 is arranged in the second housing 420 and can be inserted into the tooth grooves to lock or unlock the relative position between the first housing 411 and the second housing 420, thereby determining the circumferential rotation angle of the first housing 411 relative to the second housing and facilitating circumferential positioning of the heart valve by the handle 401.

As shown in FIG. 30, the second housing 420 is further provided with a limiting space 4202 extending along the axial direction of the second housing 420. One side of the limiting space 4202 is separated from the mounting cavity 4201 by a partition, and the other side is communicated with the receiving groove 4203. A part of the locking member 4100 is located in the limiting space 4202 and can move axially relative to the limiting space 4202, thereby realizing locking or unlocking of the tooth grooves. Further, when the first housing 411 rotates relative to the second housing 420 to a suitable circumferential angle for deploying the heart valve prosthesis, the locking member 4100 locks the tooth grooves to prevent the first housing 411 from continuing to rotate, achieving precise implantation of the heart valve prosthesis.

Optionally, the proximal end of the second housing 420 is further provided with a receiving groove 4203 for accommodating multiple teeth, which is communicated with the insertion groove. When the first housing 411 rotates relative to the second housing 420, the multiple teeth at the end of the insertion post also rotate in the receiving groove 4203.

As shown in FIG. 28, as an embodiment, three mounting slots 414 are circumferentially arranged on the first housing 411.

In the implementation of the above solution, three mounting grooves 414 are evenly distributed circumferentially on the first housing 411, with the axes of adjacent mounting grooves 414 arranged at 120 degrees. Since the circumferential rotation angle of the handle 401 is unknown, providing three first drive assemblies 421 circumferentially on the first housing 411 allows medical staff to operate any one of the first drive assemblies 421, thereby enabling deployment or retrieval of the heart valve prosthesis and improving the convenience of medical staff in operating the delivery device.

As shown in FIG. 28, as an embodiment, the first housing 411 includes a first upper housing and a second lower housing, which are mutually snapped to facilitate installation and maintenance of other parts in the first housing 411.

As shown in FIG. 27, optionally, the length of the first upper housing is greater than that of the second lower housing, and the proximal end of the first upper housing is provided with a protruding segment 419. When the first upper housing and the second lower housing are mutually snapped, the protruding segment 419 is connected to the proximal end of the second lower housing. The protruding segment 419 is configured to connect the retrieval suture assembly on the handle.

Optionally, the second housing 420 is also in mutually snapped configuration to facilitate installation and maintenance of other parts in the second housing 420.

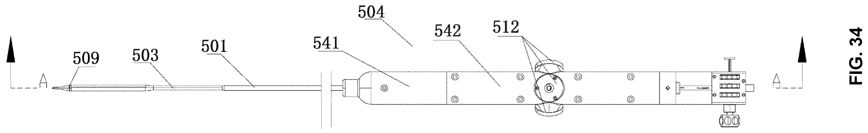

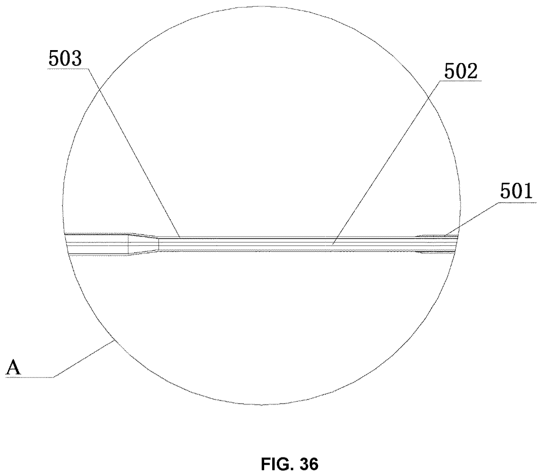

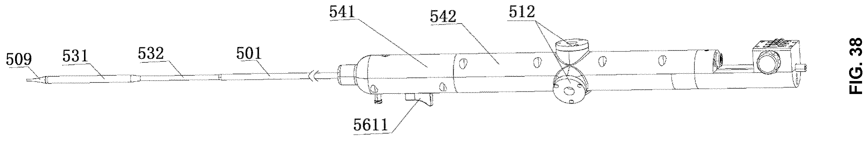

As shown in FIGS. 34 to 38, an embodiment of the present application provides a delivery device for a heart valve prosthesis, including a handle 504, an outer tube 501 connected to the handle 504, and an inner tube assembly extending in the outer tube 501 and capable of circumferentially rotating relative to the outer tube 501. The inner tube assembly includes a first inner tube 503 and a second inner tube 502 extending in the first inner tube 503, and an accommodating space for accommodating the heart valve prosthesis is provided between the first inner tube 503 and the second inner tube 502.

In the implementation of the above solution, the delivery device includes a handle 504, an outer tube 501, a first inner tube 503, and a second inner tube 502. The first inner tube 503 and the second inner tube 502 form the inner tube assembly. The outer tube 501, the first inner tube 503, and the second inner tube 502 are all connected to the handle 504. The outer tube 501 is sleeved on the outer circumference of the first inner tube 503, the first inner tube 503 is sleeved on the outer circumference of the second inner tube 502, and the second inner tube 502 extends in the first inner tube 503. The first inner tube 503 and the second inner tube 502 both extend in the outer tube 501 and can synchronously rotate circumferentially relative to the outer tube 501. An accommodating space for accommodating the heart valve prosthesis is provided between the first inner tube 503 and the second inner tube 502. When the first inner tube 503 and the second inner tube 502 synchronously rotate circumferentially relative to the outer tube 501, the first inner tube 503 and the second inner tube 502 clamp the heart valve prosthesis and synchronously rotate circumferentially relative to the outer tube 501 to achieve circumferential positioning of the heart valve prosthesis. After the circumferential positioning of the heart valve prosthesis, the heart valve prosthesis is deployed to improve the accuracy of the implantation position of the heart valve prosthesis by the delivery device.

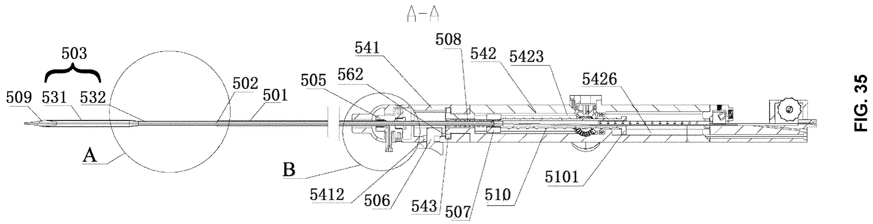

As shown in FIGS. 35 to 37, in some embodiments, the proximal ends of both the first inner tube 503 and the second inner tube 502 are connected to the handle 504. The first inner tube 503 includes an extension tube segment 532 connected to the handle 504 and extending distally within the outer tube 501, as well as a receiving tube segment 531 protruding from the distal end of the outer tube 501 for accommodating the heart valve prosthesis. The radial dimension of the receiving tube segment 531 is larger than that of the extension tube segment 532.

In the implementation of the above solution, the proximal ends of the first inner tube 503 and the second inner tube 502 are both connected to the handle 504. The first inner tube 503 includes the extension tube segment 532 and the receiving tube segment 531. One end of the extension tube segment 532 is connected to the handle 504, and the other end protrudes and extends toward the distal end of the outer tube 501 and is connected to the receiving tube segment 531. The receiving tube segment 531 is located at the distal end of the outer tube 501, and its radial dimension is larger than that of the extension tube segment 532, serving to accommodate the heart valve prosthesis.

Specifically, the distal end of the second inner tube 502 is also fixedly connected with a guide head 509. The guide head 509 is of a conical structure. The end of the guide head 509 has a streamlined shape, which can avoid scratching the inner wall of the blood vessel and is beneficial for guiding the entire delivery device to advance along the blood vessel. The tail of the guide head 509 is of a planar structure, used for abutting against the heart valve prosthesis.

As shown in FIGS. 35 to 39, 41 and 42, in some embodiments, the handle 504 includes a first housing 541 and a second housing 542 connected to the first housing 541. The second housing 542 can rotate circumferentially relative to the first housing 541. A fixing member 505 is arranged in the first housing 541 and connected to the proximal end of the outer tube 501. The handle 504 further includes a transmission member 507. A chamber 5423 is arranged in the second housing 542, and the transmission member 507 is installed in the chamber 5423. The proximal end of the first inner tube 503 is connected to the transmission member 507, the proximal end of the second inner tube 502 is connected to the second housing 542. When the transmission member 507 rotates with the second housing 542 relative to the first housing 541, the first inner tube 503 and the second inner tube 502 synchronously rotate circumferentially relative to the outer tube 501.

In the implementation of the above solution, the handle 504 includes the first housing 541 and the second housing 542 connected to the first housing 541. The second housing 542 can rotate circumferentially relative to the first housing 541. When circumferential positioning of the heart valve prosthesis is required, the second housing 542 rotates relative to the first housing 541. When deploying or retrieving the heart valve prosthesis, the second housing 542 remains stationary relative to the first housing 541. The fixing member 505 is arranged in the first housing 541, and the proximal end of the outer tube 501 is fixedly connected to the fixing member 505, thereby improving the stability of the outer tube 501. Meanwhile, the outer tube 501 is sleeved on the outer circumference of the first inner tube 503. When both the first inner tube 503 and the outer tube 501 are inside the human body, the outer tube 501 provides stable support for the first inner tube 503. The handle 504 also includes the transmission member 507. The proximal end of the first inner tube 503 is connected to the transmission member 507, and the second inner tube 502 is fixedly connected to the proximal end of the second housing 542. The chamber 5423 is arranged in the second housing 542 and extends along the axial direction of the second housing 542. The transmission member 507 is installed in the chamber 5423. When the second housing 542 rotates relative to the first inner tube 503, the transmission member 507 and the second housing 542 synchronously rotate relative to the first housing 541. Meanwhile, since the proximal end of the first inner tube 503 is fixedly connected to the transmission member 507 and the outer tube 501 is fixedly connected to the fixing member 505, when the transmission member 507 rotates relative to the first housing 541, the first inner tube 503 also rotates relative to the first housing 541 and simultaneously rotates circumferentially relative to the outer tube 501. In addition, the proximal end of the second inner tube 502 is fixedly connected to the proximal end of the second housing 542. Therefore, when the second inner tube 502 rotates relative to the first inner tube 503, the second inner tube 502 also rotates relative to the first housing 541 and simultaneously rotates circumferentially relative to the outer tube 501. Finally, when the second housing 542 rotates relative to the first housing 541, the first inner tube 503 and the second inner tube 502 synchronously rotate circumferentially relative to the outer tube 501. When rotating to the position where the heart valve prosthesis needs to be implanted, the heart valve prosthesis is deployed, thereby improving the accuracy of the implantation position of the heart valve prosthesis.

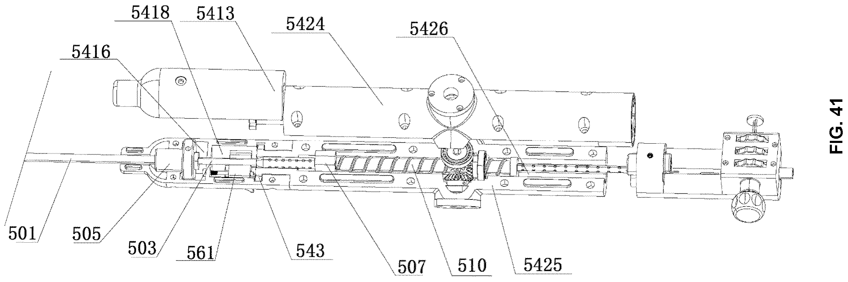

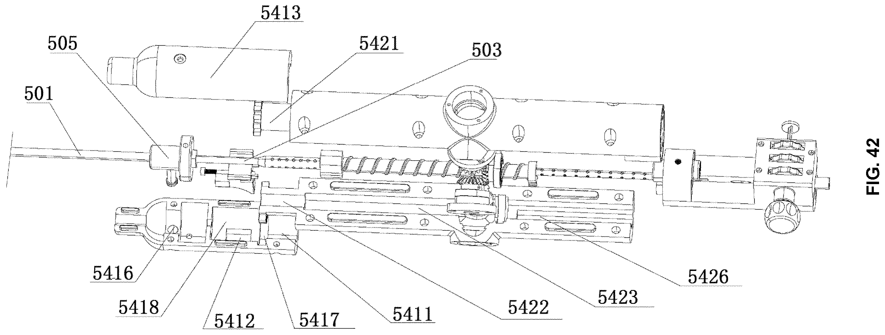

As specifically shown in FIGS. 41 and 42, the first housing 541 is formed by snapping a first upper housing 5413 and a first lower housing 5414, and the second housing 542 is formed by snapping a second upper housing 5424 and a second lower housing 5425. The proximal end of the second inner tube 502 is fixedly connected to the proximal end of the second housing 542 by means of gluing, etc. Both the second upper housing 5424 and the second lower housing 5425 are provided with recesses, and the positions of the two recesses correspond to each other. When the second upper housing 5424 and the second lower housing 5425 are snapped, the two recesses form the chamber 5423, and the transmission member 507 is embedded in the chamber 5423, so that when the second housing 542 rotates relative to the first housing 541, the transmission member 507 and the second housing 542 synchronously rotate relative to the first housing 541.

The first housing 541 is designed to be formed by snapping the first upper housing 5413 and the first lower housing 5414, which facilitates the assembly of other components in the first housing 541. Similarly, the second housing 542 is designed to be formed by snapping the second upper housing 5424 and the second lower housing 5425, which also facilitates the assembly of other components in the second housing 542.

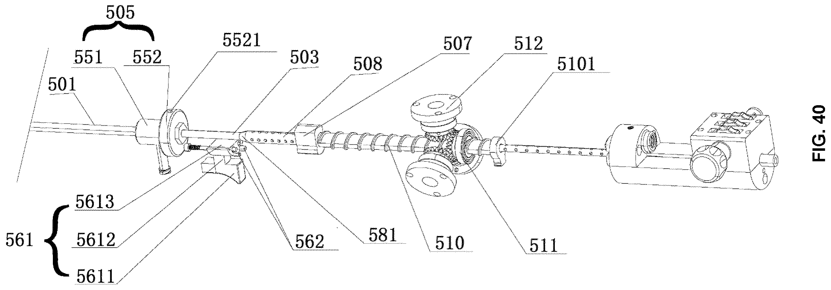

As shown in FIG. 40, in some embodiments, the fixing member 505 includes a fixing post 551 and a fixing portion 552 connected to the fixing post 551. The proximal end of the outer tube 501 is fixedly connected to the fixing post 551. A mounting cavity 5416 is arranged in the first housing 541, and the fixing post 551 is embedded in the mounting cavity 5416. The fixing portion 552 is provided with at least one fixing hole 5521, and the first housing 541 is provided with at least one fixing mating hole 5415 corresponding to the fixing hole 5521. A fastener passes through the fixing hole 5521 and the fixing mating hole 5415 to fixedly connect the fixing member 505 and the first housing 541.

In the implementation of the above solution, a mounting cavity 5416 is arranged in the first housing 541. The fixing member 505 includes a fixing post 551 and a fixing portion 552 connected to the fixing post 551. The fixing post 551 is embedded in the mounting cavity 5416 to achieve preliminary fixing of the fixing member 505 and the first housing 541. The fixing portion 552 is provided with at least one fixing hole 5521, and the first housing 541 is provided with at least one fixing mating hole 5415 corresponding to the fixing hole 5521. A fastener passes through the fixing mating hole 5415 and the fixing hole 5521 to fixedly connect the fixing member 505 and the first housing 541. Meanwhile, the proximal end of the outer tube 501 is threadedly connected to the fixing post 551 to achieve fixed connection between the outer tube 501 and the fixing post 551.

Specifically, the fixing post 551 is a cylindrical structure, and the fixing portion 552 is also a cylindrical structure with a diameter larger than that of the fixing post 551. This design shortens the distance between the fixing portion 552 and the first housing 541, facilitating the fixed connection between the fixing member 505 and the first housing 541 via fasteners. Two fixing holes 5521 are oppositely arranged on the fixing portion 552. The first upper housing 5413 is provided with a fixing mating hole 5415 corresponding to one of the fixing holes 5521, and the first lower housing 5414 is provided with a fixing mating hole 5415 corresponding to the other fixing hole 5521. Two fasteners then fix the fixing member 505 and the first housing 541 from two directions respectively.

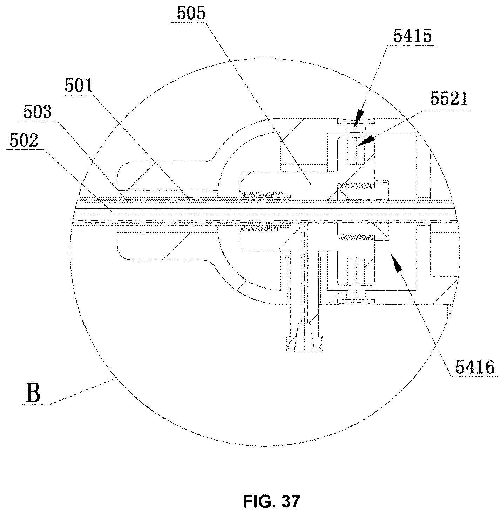

As shown in FIG. 37, in some embodiments, both the first inner tube 503 and the second inner tube 502 pass through the fixing member 505 and are respectively connected to the transmission member 507 and the proximal end of the second housing 542.

In the implementation of the above solution, the fixing member 505 is provided with a hollow channel in the axial direction. The first inner tube 503 and the second inner tube 502 both pass through the hollow channel and are respectively connected to the transmission member 507 and the proximal end of the second housing 542. When the second housing 542 rotates circumferentially relative to the first housing 541, the first inner tube 503 and the second inner tube 502 can synchronously rotate circumferentially relative to the first housing 541, further realizing the circumferential positioning of the heart valve prosthesis by the delivery device.

As shown in FIG. 40, in some embodiments, a reinforcing tube 508 is sleeved on the proximal end of the first inner tube 503. The reinforcing tube 508 is fixedly connected to the first inner tube 503, and the proximal end of the reinforcing tube 508 is fixedly connected to the transmission member 507.

In the implementation of the above solution, the reinforcing tube 508 is sleeved on the proximal end of the first inner tube 503 and fixedly connected to the first inner tube 503. Since the first inner tube 503 is a flexible tube, sleeving the reinforcing tube 508 on its outer circumference provides stable support for the first inner tube 503.

In some embodiments, the reinforcing tube 508 is provided with multiple holes for glue application.

In the implementation of the above solution, the reinforcing tube 508 is provided with multiple holes to facilitate glue application, thereby realizing the fixed connection between the reinforcing tube 508 and the first inner tube 503.

As shown in FIG. 42, in some embodiments, the first housing 541 is located at the distal end of the handle 504, and the second housing 542 is located at the proximal end of the handle 504. The chamber 5423 extends axially along the second housing 542, and the transmission member 507 is adapted to move axially along the second housing 542 relative to the chamber 5423, thereby driving the first inner tube 503 to move axially relative to the second inner tube 502.

In the implementation of the above solution, the first housing 541 is located at the distal end of the handle 504, the second housing 542 is located at the proximal end of the handle 504, and the chamber 5423 extends along the axial direction of the second housing 542. When the transmission member 507 is adapted to move axially along the chamber 5423, it drives the first inner tube 503 to move synchronously. Additionally, since the second inner tube 502 is fixedly connected to the proximal end of the second housing 542, the first inner tube 503 moves axially relative to the second inner tube 502 when the transmission member 507 drives the first inner tube 503 to move synchronously. Here, "adapted to" means that the transmission member 507 moves axially only when required to move axially along the chamber 5423.

Specifically, the transmission member 507 is a block structure with two opposite arc surfaces. The two recesses of the second upper housing 5424 and the second lower housing 5425 are respectively adapted to the two arc surfaces, thereby reducing the resistance generated when the transmission member 507 moves axially along the chamber 5423 and making it easier for the transmission member 507 to move axially along the chamber 5423. When the transmission member 507 moves axially along the chamber 5423, it drives the first inner tube 503 to move axially, while the second inner tube 502 remains fixed. Therefore, when the first inner tube 503 moves axially relative to the second inner tube 502, the heart valve prosthesis located in the receiving tube segment 531 is deployed.

As shown in FIGS. 35, 41 and 42, in some embodiments, a transmission rod 510 is further arranged in the second housing 542. The distal end of the transmission rod 510 is fixedly connected to the transmission member 507, and a sliding block 5101 is provided at the proximal end of the transmission rod 510. A sliding groove 5426 is also provided within the second housing 542. The sliding block 5101 is embedded in the sliding groove 5426 and slidably engaged therewith. A stop portion for stop-limiting engagement with the sliding block 5101 is provided at the proximal end of the sliding groove 5426.

In the implementation of the above solution, the transmission rod 510 is arranged in the second housing 542 and is coaxially arranged with the transmission member 507. The distal end of the transmission rod 510 is threadedly connected to the transmission member 507, the proximal end of the transmission rod 510 is provided with the sliding block 5101. The sliding groove 5426 is further arranged in the second housing 542 and extends along the axial direction of the second housing 542. The sliding groove 5426 is located beside the chamber 5423 and is spaced from the chamber 5423. The sliding block 5101 is embedded in the sliding groove 5426 to provide stable support for the transmission rod 510. Meanwhile, the sliding block 5101 can slide in the sliding groove 5426. When the transmission rod 510 is driven, the transmission rod 510 moves axially along the chamber 5423 and drives the transmission member 507 to move axially along the chamber 5423. Additionally, the stop portion is further arranged at the proximal end of the sliding groove 5426. When the sliding block 5101 moves axially from the distal end of the sliding groove 5426 toward the proximal end of the sliding groove 5426, the stop portion can stop the sliding block 5101 to limit its movement and prevent the sliding block 5101 from falling out of the sliding groove 5426.

Specifically, both the transmission member 507 and the transmission rod 510 are of hollow structures. The second inner tube 502 extends in the first inner tube 503 and passes through the first inner tube 503, the transmission member 507 and the transmission rod 510 to be fixedly connected to the proximal end of the second housing 542.

In some embodiments, a drive assembly is further arranged on the second housing 542, and the drive assembly is used to drive the transmission rod 510 to move axially relative to the handle 504.

In the implementation of the above solution, the drive assembly is arranged on the second housing 542. When the drive assembly drives the transmission rod 510, the transmission rod 510 drives the transmission member 507 to move axially relative to the second housing 542 along the chamber 5423, that is, to move axially relative to the handle 504, thereby realizing the synchronous movement of the transmission member 507 and the transmission rod 510.

As shown in FIGS. 40 to 42, in some embodiments, the drive assembly includes a first drive member 511 disposed on the transmission rod 510 and threadedly connected thereto. The drive assembly further includes at least two second drive members 512 arranged circumferentially along the transmission member 507. The second drive members 512 are drivingly connected to the first drive member 511 and are configured to drive the first drive member 511 to rotate.

In the implementation of the above solution, the drive assembly includes a first drive member 511 sleeved on the outer circumference of the transmission rod 510 and threadedly connected thereto. The drive assembly further includes at least two second drive members 512, both of which are located on the second housing 542 and evenly distributed circumferentially thereon. This facilitates medical staff to operate the second drive members 512 from different angles, thereby enabling the first inner tube 503 to move axially relative to the second inner tube 502, improving the operational convenience for medical staff and the success rate of the operation. One end of the second drive member 512 is provided with multiple teeth, and one end of the first drive member 511 is provided with tooth grooves corresponding to the multiple teeth. The teeth on the second drive member 512 mesh with the tooth grooves on the first drive member 511, realizing the transmission connection between the first drive member 511 and the second drive member 512. When the second drive member 512 rotates, the first drive member 511 rotates synchronously with the second drive member 512. Since the transmission rod 510 is threadedly connected to the first drive member 511 and the sliding block 5101 is embedded in the sliding groove 5426, the transmission rod 510 moves axially along the chamber 5423, and the sliding block 5101 moves axially along the sliding groove 5426.

Specifically, three second drive members 512 are arranged on the second housing 542. One end of each of the three second drive members 512 is provided with multiple teeth, and all three second drive members 512 mesh with the first drive member 511. The axes of adjacent second drive members 512 are at 120°, which is designed to allow medical staff, when holding the delivery device and needing to rotate the second housing 542 circumferentially relative to the first housing 541, to operate any of the three second drive members 512 conveniently to drive the first inner tube 503 to move axially relative to the second inner tube 502, thereby deploying the heart valve prosthesis and improving the operational convenience for medical staff.

Optionally, the ends of the three second drive members 512 are each provided with multiple teeth, and the axes of the three second drive members 512 are all perpendicular to the axis of the first drive member 511, ensuring the meshing angle between the first drive member 511 and the second drive members 512. This enables the second drive members 512 to more smoothly drive the first drive member 511 to rotate relative to the transmission rod 510 when rotating around their own axes.

Optionally, each second drive member 512 can drive the transmission rod 510 to move axially relative to the handle 504. The second inner tube 502 is connected to the handle 504 by gluing or other means, and the transmission rod 510 is connected to the first inner tube 503. When the transmission rod 510 moves axially relative to the handle 504, it simultaneously drives the first inner tube 503 to move axially relative to the handle 504, so that the first inner tube 503 moves axially relative to the second inner tube 502, thereby achieving deployment or retrieval of the heart valve prosthesis.

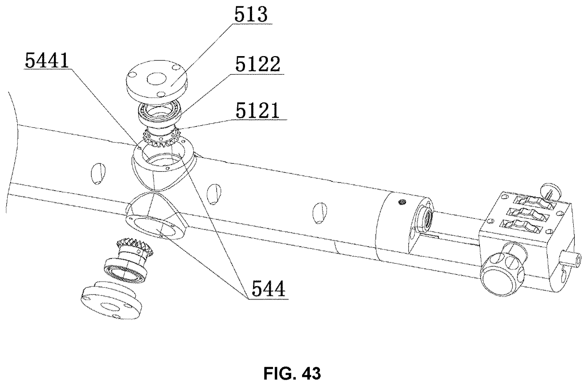

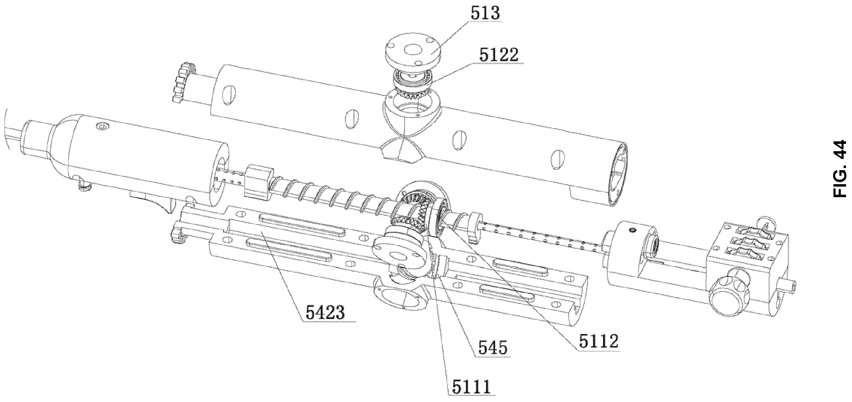

As shown in FIGS. 43 and 44, the first drive member 511 includes a first drive portion 5111 and a first support portion 5112 sleeved on the outer circumference of the first drive portion 5111. The first support portion 5112 is in a tight fit with the first drive portion 5111, so that the first support portion 5112 exerts a clamping force on the first drive portion 5111 to ensure structural stability. The distal end of the first drive portion 5111 is provided with multiple tooth grooves. The second drive member includes a second drive portion 5121 and a second support portion 5122 sleeved on the outer circumference of the second drive portion 5121. The second support portion 5122 is in a tight fit with the second drive portion 5121. One end of the second drive portion 5121 facing the transmission rod 510 is provided with multiple teeth. The first drive portion 5111 meshes with the second drive portion 5121. The first drive portion 5111 is sleeved on the outer circumference of the transmission rod 510 and in threaded transmission connection therewith. The second drive portion 5121 is adapted to drive the first drive portion 5111 to rotate, so that the first drive portion 5111 transmits relative to the transmission rod 510 and the transmission rod 510 moves axially relative to the handle 504. Here, "adapted to" means that when the transmission rod 510 needs to move axially relative to the handle 504, the second drive portion 5121 drives the first drive portion 5111 to rotate, so that the transmission rod 510 moves axially relative to the handle 504.

The delivery device further includes a power rod. The second drive portion 5121 is provided with a slot recessed from the end facing away from the multiple teeth toward the end with the multiple teeth. One end of the power rod is adapted to the slot, and the power rod includes an insertion end that can be inserted into the slot. When the power rod is inserted into the slot, it can drive the second drive portion 5121 to rotate, so that the second drive portion 5121 synchronously drives the first drive portion 5111 to rotate. When one end of the power rod is separated from the slot, the second drive portion stops rotating.

As shown in FIG. 43, optionally, the second housing 542 is provided with a mounting groove 544 corresponding to the second drive member 512. The mounting groove 544 is configured to accommodate the second drive member 512, so that the second drive member 512 can rotate in the mounting groove 544. The peripheral wall of the mounting groove 544 provides stable support for the second drive member 512.

As shown in FIG. 43, an annular boss 5441 is further arranged in the mounting groove 544. The second support portion 5122 is located in the mounting groove 544 and abuts against the annular boss 5441. The second drive portion 5121 is in a tight fit with the second support portion 5122. When the insertion end of the power rod drives the second drive portion 5121 to rotate, the second support portion 5122 and the second drive portion 5121 rotate synchronously.

Optionally, the number of mounting grooves 544 corresponds to the number of second drive members 512, and multiple mounting grooves 544 are evenly distributed circumferentially along the second housing 542, so that medical staff can drive the second drive members 512 to rotate from multiple angles, enabling the first inner tube 503 to move axially relative to the second housing 542 and improving the operational convenience for medical staff.

Optionally, the second support portion 5122 is a ball bearing, including an inner ring and an outer ring, which are in transmission connection through multiple balls. The diameter of the inner ring is equal to the radial dimension of the end of the second drive portion 5121 facing away from the multiple teeth, so that the second support portion 5122 can be sleeved on the outer circumference of the second drive portion 5121 to achieve a tight fit connection. The outer ring abuts against the annular boss 5441 and is embedded in the mounting groove 544 to ensure the stability of the second support portion 5122. When the second drive portion 5121 rotates, the inner ring of the second support portion 5122 rotates synchronously with the second drive portion 5121, and the outer ring of the second support portion 5122 remains relatively stationary. The second support portion 5122 not only provides support for the second drive portion 5121 but also ensures the stability of the second drive portion 5121 during rotation.

As shown in FIG. 44, optionally, a positioning groove 545 is further arranged in the second housing 542. The first support portion 5112 is embedded in the positioning groove 545, and the chamber 5423 is communicated with the positioning groove 545. The first drive portion 5111 is in a tight fit with the first support portion 5112. When the second drive portion 5121 drives the first drive portion 5111 to rotate, the first support portion 5112 provides stable support for the first drive portion 5111 to ensure that the first drive portion 5111 and the second drive portion 5121 rotate synchronously.

Optionally, the first support portion 5112 has the same structure as the second support portion 5122, both being ball bearings including an inner ring and an outer ring. The outer ring of the first support portion 5112 fits against the wall of the positioning groove 545, while the inner ring is in tight fit with the first drive part 5111. When the first drive part 5111 rotates, the inner ring of the first support portion 5112 rotates synchronously with the first drive part 5111, and the outer ring of the first support portion 5112 remains relatively stationary. The first support portion 5112 not only provides support for the first drive part 5111, but also ensures the stability of the first drive part 5111 during rotation.

Optionally, both the second upper housing 5424 and the second lower housing 5425 are provided with positioning grooves 545, and the positions of the two positioning grooves 545 correspond to each other. When the second upper housing 5424 and the second lower housing 5425 are snapped together, the first support portion 5112 is accommodated in the positioning groove 545.

As shown in FIG. 44, an end cover 513 is further arranged at the opening of the mounting groove 544. The end cover 513 is detachably connected with the peripheral wall of the mounting groove 544 to protect the second drive part 512. At the same time, the end cover 513 plays a role in limiting the second support portion 5122 to prevent the second support portion 5122 from falling out of the mounting groove 544.

Optionally, the center of the end cover 513 is also provided with a through hole corresponding to the slot, so that the insertion end of the power rod can be inserted into the slot.

The end cover 513 and the peripheral wall of the mounting groove 544 are fixedly connected by a plurality of bolts, and can also be connected by clamping or other means.

As shown in FIGS. 41 and 42, in some embodiments, the proximal end of the first housing 541 is provided with an insertion groove 5411, and the distal end of the second housing 542 is provided with an insertion post 5421, and the insertion post 5421 is inserted into the insertion groove 5411. The end of the insertion post 5421 is provided with a locking member 543, and the proximal end of the first housing 541 is also provided with a receiving groove 5417 for accommodating the locking member 543. The receiving groove 5417 is communicated with the insertion groove 5411, and the receiving groove 5417 can allow the locking member 543 to rotate.

In the implementation of the above solution, the proximal end of the first housing 541 is provided with the insertion groove 5411, the distal end of the second housing 542 is provided with the insertion post 5421, and the insertion post 5421 is inserted into the insertion groove 5411, so that the second housing 542 can rotate relative to the first housing 541. The end of the insertion post 5421 is provided with the locking member 543, and the proximal end of the first housing 541 is also provided with the receiving groove 5417, which is communicated with the insertion groove 5411 and used for accommodating the locking member 543. When the second housing 542 rotates relative to the first housing 541, the locking member 543 and the second housing 542 rotate synchronously relative to the first housing 541, and the locking member 543 rotates in the receiving groove 5417.

Specifically, the radial dimension of the receiving groove 5417 is larger than that of the insertion groove 5411, so that when the insertion post 5421 is inserted into the insertion groove 5411, one side of the locking member 543 abuts against one side of the receiving groove 5417, which can prevent the second housing 542 from falling out of the insertion groove 5411 of the first housing 541.

As shown in FIGS. 40 to 42, in some embodiments, a locking mating member 506 is further arranged in the first housing 541, and the locking mating member 506 cooperates with the locking member 543 to lock or unlock the relative position of the first housing 541 and the second housing 542.

In the implementation of the above solution, the locking mating member 506 is further arranged in the first housing 541, and the locking mating member 506 cooperates with the locking member 543 to realize the relative position between the first housing 541 and the second housing 542 through unlocking or locking.

Specifically, a limiting groove 5418 is further arranged in the first housing 541, and the limiting groove 5418 extends along the axial direction of the first housing 541. One side of the limiting groove 5418 is separated from the mounting cavity 5416 by a partition, and the other side is communicated with the receiving groove 5417. A part of the locking mating member 506 is located in the limiting groove 5418 and can move axially relative to the limiting groove 5418, so as to lock or unlock the locking member 543. Furthermore, when the second housing 542 rotates relative to the first housing 541 to a suitable circumferential angle for deployment of the heart valve prosthesis, the locking mating member 506 locks the locking member 543 to prevent the second housing 542 from continuing to rotate, so as to achieve the purpose of precise implantation of the heart valve prosthesis.

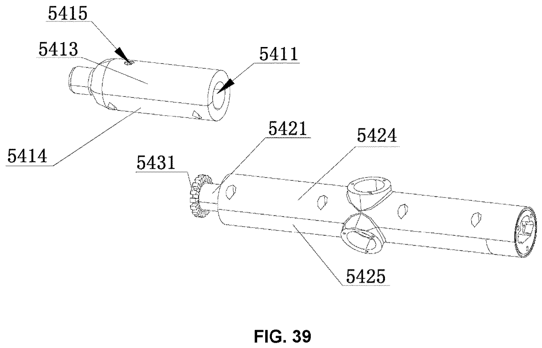

As shown in FIGS. 35 and 39, in some embodiments, the locking member 543 includes a locking gear 5431 arranged at the end of the insertion post 5421, and the locking gear 5431 forms a plurality of locking grooves. The locking mating member 506 includes a movable member 561 and at least one locking post 562 arranged on the movable member 561. When the movable member 561 is driven, the movable member 561 moves axially along the first housing 541, so that the locking post 562 is adapted to be inserted into or disengaged from the locking grooves.

In the implementation of the above solution, the locking member 543 includes the locking gear 5431 located at the end of the insertion post 5421, and the locking gear 5431 is provided with a plurality of locking grooves. The locking mating member 543 includes the movable member 561 and at least one locking post 562 connected with the movable member 561. The locking post 562 extends along the axial direction of the limiting groove 5418. When the movable member 561 moves axially along the first housing 541 towards the locking gear 5431, the locking post 562 is inserted into the locking groove, and the relative position of the first housing 541 and the second housing 542 will be locked. When the movable member 561 moves axially along the first housing 541 away from the locking gear 5431, the locking post 562 is separated from the locking groove, and the second housing 542 can rotate circumferentially relative to the first housing 541.

Specifically, the number of the locking posts 562 is set to two. The two locking posts 562 are located on one side of the movable member 561 and extend convexly towards the locking post 562. The two locking posts 562 are symmetrically arranged with the axis of the movable member 561 as the symmetry line. By setting two locking posts 562, the locking effect between the locking member 543 and the locking mating member 506 is enhanced, preventing the second housing 542 from wobbling when the locking member 543 is engaged with the locking engagement member 506.

Specifically, the locking gear 5431 is provided with a plurality of teeth, and the gaps between adjacent teeth form the locking grooves. The teeth are structured as quadrangular frustums. The width of the locking posts 562 matches the gaps between adjacent teeth, such that when the locking posts 562 are inserted into the locking grooves, both sides of the locking posts 562 fit snugly against the adjacent teeth, enhancing the locking effect. Additionally, since the teeth are quadrangular frustums, the top surface of each tooth connects to the plug-in 5421, and the gaps between adjacent teeth gradually decrease outward in the radial direction of the insertion post 5421. This configuration allows the adjacent teeth to clamp the locking posts 562 when inserted, further improving the locking effect.

As shown in FIGS. 35 and 42, in some embodiments, the first housing 541 is provided with an installation gap 5412. The movable member 561 includes a drive block 5611, a connection block 5612 connected with the drive block 5611, and a mounting block 5613 connected with the connection block 5612. The connection block 5612 is slidably connected with the installation gap 5412, the drive block 5611 and the mounting block 5613 are respectively located inside and outside the first housing 541, and the mounting block 5613 is provided with the locking post 562.

In the implementation of the above solution, the first housing 541 is provided with the installation gap 5412, and the installation gap 5412 is communicated with the limiting groove 5418. The movable member 561 includes the drive block 5611, the connection block 5612 and the mounting block 5613. The connection block 5612 is respectively connected with the drive block 5611 and the mounting block 5613, and the mounting block 5613 is fixedly connected with the insertion post 5421, so as to form the locking mating member 506. The drive block 5611 is located outside the first housing 541, the mounting block 5613 is located inside the first housing 541, and the connection block 5612 is slidably connected with the installation gap 5412, so that the movable member 561 can move axially along the installation gap 5412.

In some embodiments, the insertion post 5421 is provided with a through channel 5422 inside, and the distal end of the reinforcing pipe 508 is provided with a tapered head 581 for passing through the through channel 5422. The through channel 5422 is communicated with the chamber 5423. The first inner tube 503 passes through the through channel 5422, and the proximal end of the first inner tube 503 is connected with the transmission member 507 in the chamber 5423. The second inner tube 502 extends in the first inner tube 503 and passes through the through channel 5422 to be connected with the proximal end of the second housing 542.

In the implementation of the above solution, the insertion post 5421 is provided with the through channel 5422 inside, and the distal end of the reinforcing pipe 508 is provided with the tapered head 581. When the transmission member 507 moves axially along the second housing 542, the tapered head 581 at the distal end of the reinforcing pipe 508 makes the reinforcing pipe 508 easier to pass through the through channel 5422 in the insertion post 5421, which improves the operability of the delivery device. At the same time, the through channel 5422 is communicated with the chamber 5423. The through channel 5422 is arranged in the insertion post 5421, so that the first inner tube 503 can pass through the through channel 5422 and be fixedly connected with the transmission member 507 in the chamber 5423, and the second inner tube 502 passes through the through channel 5422 and is fixedly connected with the proximal end of the second housing 542.

The above describes some specific embodiments of the present application, but the protection scope of the present application is not limited thereto. Any person skilled in the art can easily think of changes or substitutions within the technical scope disclosed in the present application, which should all be covered by the protection scope of the present application. Therefore, the protection scope of the present application should be subject to the protection scope of the claims.

It should be noted that in the present application, relational terms such as "first" and "second" are only used to distinguish one entity or operation from another, and do not necessarily require or imply any actual relationship or sequence between these entities or operations. Additionally, the term "comprise," "include," or any other variation thereof is intended to cover a non-exclusive inclusion, such that a process, method, article, or device comprising a series of elements not only includes those elements but also includes other elements not explicitly listed, or elements inherent to such process, method, article, or device. Without further limitation, process, method, article, or device "comprising" an element does not exclude the presence of additional identical elements in the process, method, article, or device comprising the element.

### Industrial Applicability

The present application relates to the technical field of medical devices, and provides an interventional delivery device for a heart valve prosthesis and threading member thereof. The interventional delivery device for a heart valve prosthesis includes: an outer tube; an inner tube located in the outer tube; a threading member arranged on the inner tube, which is provided with a threading through hole and a positioning area; a suture end positioning member, one end of which is detachably positioned in the positioning area of the threading member; and a traction member, one end of which passes through the threading through hole to connect the heart valve prosthesis and is connected to the suture end positioning member, and the other end of which extends out of the outer tube. The present application provides a retrieval device for retrieving a connection suture and a delivery device for a heart valve prosthesis. The retrieval device for retrieving a connection suture includes: a body used for connecting to a handle, and a suture winding/unwinding assembly assembled on the body; the suture winding/unwinding assembly includes a rod capable of rotating relative to the body, and a plurality of single-rotation units arranged on the rod and capable of unidirectional suture-winding rotation relative to the rod, each single-rotation unit being provided with a fixing part for connecting connection sutures and a suture winding part for winding connection sutures. The present application provides a retrieval device for a retrieval suture of a heart valve prosthesis and delivery device thereof. The retrieval device for a retrieval suture of a heart valve prosthesis includes: a body, and a suture winding/unwinding assembly arranged on the body; the suture winding/unwinding assembly includes a main rotating part for synchronously driving a plurality of retrieval sutures and an auxiliary adjusting part for driving one retrieval suture, the auxiliary adjusting part including a suture adjusting portion, the main rotating part including a suture winding part, and the suture adjusting portion and the suture winding portion being respectively located at different positions in the axial direction of the body. The present application provides an interventional delivery device for a heart valve prosthesis and handle thereof. The interventional delivery device for a heart valve prosthesis includes: a handle; a delivery assembly extending in the handle and capable of moving axially relative to the handle; the delivery assembly including a transmission rod; at least two first drive assemblies, which are arranged along the circumferential direction of the transmission rod, are in transmission connection with the transmission rod, and are used for driving the transmission rod to move axially relative to the handle. Through the technical solutions of the present application, the relative position between the heart valve prosthesis and the heart can be adjusted, and the accuracy of implantation of the heart valve prosthesis can be improved. The technical problem that in the retrieval process of the heart valve prosthesis, multiple connection rings on the heart valve prosthesis cannot be simultaneously and normally returned to the position abutting against the fixing head, resulting in the retrieval failure of the heart valve prosthesis, can be solved. The implantation position of the heart valve prosthesis can be finely adjusted to improve the accuracy of implantation of the heart valve prosthesis. An interventional delivery device for a heart valve prosthesis and handle thereof that improve the operation convenience of medical staff can be provided. The technical solutions of the present application have excellent industrial applicability.

## Claims

1. An interventional delivery device for a heart valve prosthesis, **characterized by** comprising:
an outer tube;
an inner tube located in the outer tube;
a threading member, provided on the inner tube, the threading member being provided with a threading through hole and a positioning area;
a suture end positioning member, one end of the suture end positioning member being detachably positioned in the positioning area of the threading member; and
a traction member, one end of the traction member passing through the threading through hole to be connected to the heart valve prosthesis and to be connected to the suture end positioning member, and the other end of the traction member extending out of the outer tube.

2. The interventional delivery device for a heart valve prosthesis according to claim 1, wherein the threading member is provided with an abutment platform configured to abut against the heart valve prosthesis.

3. The interventional delivery device for a heart valve prosthesis according to claim 2, wherein the abutment platform comprises an outer ring portion extending radially outward from the threading member and a tapered portion gradually narrowing toward the proximal end from the outer ring portion.

4. The interventional delivery device for a heart valve prosthesis according to claim 3, wherein the tapered portion is provided with a plurality of threading through holes penetrating through the abutment platform.

5. The interventional delivery device for a heart valve prosthesis according to claim 4, wherein the number of the threading through holes is three, and the three threading through holes are circumferentially arranged along the tapered portion.

6. The interventional delivery device for a heart valve prosthesis according to any one of claims 3 to 5, wherein the threading member comprises a plurality of support tabs extending distally from the outer ring portion, wherein an accommodation space is formed between two adjacent support tabs, and the accommodation space is in communication with the threading through hole.

7. The interventional delivery device for a heart valve prosthesis according to claim 6, wherein the positioning area is provided on a side of the abutment platform facing away from the support tabs.

8. The interventional delivery device for a heart valve prosthesis according to claim 5, wherein the number of the traction members is three, and the three traction members respectively pass through the three threading through holes and are configured to connect with the heart valve prosthesis.

9. The interventional delivery device for a heart valve prosthesis according to any one of claims 1 to 5, further comprising a handle, wherein the outer tube extends from the handle, a receiving space for accommodating the heart valve prosthesis is provided between the outer tube and the inner tube, and the outer tube is adapted to move axially along the handle to unfold the heart valve prosthesis or retract the heart valve prosthesis into the receiving space.

10. The interventional delivery device for a heart valve prosthesis according to claim 9, further comprising the heart valve prosthesis provided with a plurality of connection ring sets, wherein in a contracted state of the heart valve prosthesis, each of the connection ring sets abuts against the abutment platform and corresponds to the threading through holes on the same side;
one end of each of the plurality of traction members is connected to the handle, and the other end respectively passes through the threading through hole and the connection ring set corresponding to the threading through hole, and is connected to the suture end positioning member in a detachable manner.

11. The interventional delivery device for a heart valve prosthesis according to any one of claims 1 to 5, wherein the threading member is provided with a protrusion for fitting against one side of the connection ring set.

12. The interventional delivery device for a heart valve prosthesis according to any one of claims 1 to 5, wherein the threading member is provided with a passageway configured to guide the inner tube therethrough.

13. The interventional delivery device for a heart valve prosthesis according to claim 12, wherein the positioning area is provided with a positioning hole extending axially along the threading member, an extending direction of the positioning hole is parallel to an extending direction of the passageway, and one end of the suture end positioning member is insertable into the positioning hole.

14. A threading member of an interventional delivery device for a heart valve prosthesis, **characterized by** comprising:
a passageway configured to guide an inner tube therethrough; a threading through hole configured to guide a traction member therethrough; and a positioning area configured to position a suture end positioning member.

15. The threading member according to claim 14, further comprising an abutment platform configured to abut against a heart valve prosthesis, wherein the abutment platform comprises an outer ring portion and a tapered portion gradually narrowing toward a proximal end from the outer ring portion, and the threading through hole is circumferentially disposed on the abutment platform.

16. The threading member according to claim 15, wherein an end face of the abutment platform is further provided with a plurality of support tabs, the support tabs extend from an end face of the outer ring portion toward a distal end of the threading member, an accommodation space is formed between two adjacent support tabs, and the accommodation space is in communication with the threading through hole.

17. The threading member according to claim 16, wherein the threading member is provided with a protrusion, and the protrusion and the support tabs are located on the same side of the abutment platform.

18. The threading member according to any one of claims 14 to 17, wherein the positioning area is provided with an axially extending positioning hole, and an extending direction of the positioning hole is parallel to an extending direction of the passageway.

19. A retrieval device for retrieving a connection suture, **characterized by** comprising:
a body configured to be connected to a handle; and a suture winding/unwinding assembly mounted on the body, wherein
the suture winding/unwinding assembly comprises a rod rotatable relative to the body; and a plurality of single-rotation units disposed on the rod and configured to rotate in one direction for suture winding relative to the rod, wherein each single-rotation unit is provided with a retaining portion configured to connect to the connection suture; and a suture winding portion configured to wind the connection suture.

20. The retrieval device according to claim 19, wherein the single-rotation unit comprises a unidirectional rotating member and an operating member cooperatively connected to the unidirectional rotating member, the unidirectional rotating member being mounted on the rod, and the operating member being assembled to the rod via the unidirectional rotating member.

21. The retrieval device according to claim 20, wherein the operating member comprises an operating portion protruding from the body and the suture winding portion connected to the operating portion, wherein the retaining portion is located on the suture winding portion.

22. The retrieval device according to claim 21, wherein the retaining portion comprises a protrusion or at least two first through holes configured to be fixed to the connection suture.

23. The retrieval device according to claim 21 or 22, wherein the operating member is provided with a mounting recess extending through the operating portion and the suture winding portion, the unidirectional rotating member being located in the mounting recess.

24. The retrieval device according to any one of claims 20 to 22, wherein the unidirectional rotating member is a unidirectional bearing.

25. The retrieval device according to claim 21 or 22, wherein the body comprises a retrieval handle and a cover detachably connected to the retrieval handle, the rod extending through the cover perpendicular to an axial direction of the retrieval handle.

26. The retrieval device according to claim 25, wherein the suture winding/unwinding assembly comprises a flange portion located at one end of the rod and fixedly connected to the rod, a first connection hole is provided on a side of the cover facing the flange portion, and a diameter of the flange portion is greater than a diameter of the first connection hole.

27. The retrieval device according to claim 26, wherein the suture winding/unwinding assembly comprises a rotating cap capable of driving the entire rod to rotate, and the rotating cap is located at the other end opposite to the flange portion.

28. The retrieval device according to claim 27, wherein the suture winding/unwinding assembly further comprises a locking portion provided on the rotating cap for cooperating with the cover to prevent the rod from accidental rotation.

29. The retrieval device according to claim 28, wherein the locking portion comprises a locking gear, and the cover is provided with a locking tooth groove adapted to the locking gear.

30. The retrieval device according to claim 29, wherein the cover comprises a convex cylinder protruding toward the rotating cap along an extending direction of the rod, and the locking tooth groove is provided on an inner wall of the convex cylinder.

31. The retrieval device according to claim 30, wherein the convex cylinder is provided with a second connection hole, the second connection hole is coaxial with the first connection hole, and the rod is threadedly connected to the rotating cap through the first connection hole and the second connection hole.

32. The retrieval device according to any one of claims 29 to 31, wherein a telescopic member is further provided between the flange portion and the cover, with both ends of the telescopic member abutting against the flange portion and the cover respectively, for engaging the locking gear with the locking tooth groove.

33. The retrieval device according to any one of claims 27 to 31, wherein an outer surface of the rod is provided with two clamping grooves at intervals;
the retrieval device comprises a stop block embedded in the suture winding portion of the operating member adjacent to the rotating cap, and further comprises two clamping members that cooperate with the clamping grooves, wherein the plurality of single-rotation units are positioned between the two clamping members, one of the clamping members abuts against the single-rotation unit distal to the rotating cap, the other clamping member abuts against the stop block, and the two clamping members are configured to axially retain and restrict the plurality of single-rotation units along the rod.

34. The retrieval device according to any one of claims 27 to 31, wherein the plurality of single-rotation units are sequentially arranged along an axial direction of the rod, the operating member adjacent to the rotating cap is a first operating member, the operating member abutting against the first operating member is a second operating member, and insertion grooves adapted to the suture winding portion are provided on sides of the first operating member and the second operating members facing away from the rotating cap.

35. The retrieval device according to any one of claims 25 to 31, wherein the cover is provided with a plurality of movable openings corresponding in number to the single-rotation units, and the operating portion partially protrudes from the movable openings.

36. The retrieval device according to claim 35, wherein the retrieval handle is provided therein with a threading passage for guiding the connection suture, the cover is provided therein with a move chamber in communication with the movable opening, and a threading hole for guiding the connection suture into the move chamber is further provided at a side of a distal end of the cover.

37. The retrieval device according to claim 36, further comprising a support block disposed in the move chamber and fixedly connected to the cover, wherein the support block is provided with a notch, and the suture winding portion on the operating member adjacent to the rotating cap is embedded in the notch.

38. A delivery device for a heart valve prosthesis, **characterized by** comprising: a handle; and the retrieval device for retrieving a connection suture of a heart valve prosthesis according to any one of claims 19 to 37, wherein the body is disposed at a proximal end of the handle.

39. The delivery device according to claim 38, wherein the body is detachably connected to the handle.

40. A retrieval device for a retrieval suture of a heart valve prosthesis, **characterized by** comprising:
a body; and a suture winding/unwinding assembly disposed on the body,
wherein the suture winding/unwinding assembly comprises a main rotating member for synchronously driving a plurality of retrieval sutures and an auxiliary adjusting member for driving one retrieval suture, the auxiliary adjusting member comprises a suture adjusting portion, the main rotating member comprises a suture winding portion, and the suture adjusting portion and the suture winding portion are respectively located at different positions along an axial direction of the body.

41. The retrieval device for a retrieval suture according to claim 40, wherein the suture adjusting portion is provided with a first through hole for one retrieval suture to pass through, and the suture adjusting portion is rotatable relative to the body to adjust a position or state of the heart valve prosthesis via the retrieval suture.

42. The retrieval device for a retrieval suture according to claim 41, wherein the suture adjusting portion is of a columnar structure, the auxiliary adjusting member comprises a drive portion fixedly connected to the suture winding portion, and a radial dimension of the drive portion is larger than that of the suture winding portion.

43. The retrieval device for a retrieval suture according to claim 42, wherein the auxiliary adjusting member is mounted on the body via a single-rotation unit, and the single-rotation unit is located at a side opposite to the drive portion.

44. The retrieval device for a retrieval suture according to any one of claims 40 to 43, wherein the number of the auxiliary adjusting members is three.

45. The retrieval device for a retrieval suture according to claim 44, wherein the three auxiliary adjusting members are arranged in a staggered manner.

46. The retrieval device for a retrieval suture according to claim 45, wherein the three auxiliary adjusting members comprise one first auxiliary adjusting member and two second auxiliary adjusting members, wherein the first auxiliary adjusting member and the two second auxiliary adjusting members are spaced apart along a distal-to-proximal direction of the body, and the distances from the first auxiliary adjusting member to the two second auxiliary adjusting members are equal.

47. The retrieval device for a retrieval suture according to any one of claims 40 to 43, wherein the suture winding portion and the suture adjusting portion are mounted on the body perpendicularly to each other.

48. The retrieval device for a retrieval suture according to claim 43, wherein the body comprises a cover and a retrieval handle, and the cover is detachably connected to the retrieval handle.

49. The retrieval device for a retrieval suture according to claim 48, wherein the main rotating member comprises a rotating cap capable of driving the entire suture winding portion to rotate, and the rotating cap is located at one end of the suture winding portion.

50. The retrieval device for a retrieval suture according to claim 49, wherein the main rotating member further comprises a locking portion provided on the rotating cap and facing the cover, for cooperating with the cover to prevent the suture winding portion from accidental rotation.

51. The retrieval device for a retrieval suture according to claim 50, wherein the locking portion comprises a locking gear, and the cover is provided with a locking tooth groove adapted to the locking gear.

52. The retrieval device for a retrieval suture according to claim 51, wherein the cover comprises a convex cylinder protruding toward the rotating cap along an extending direction of the suture winding portion, and the locking tooth groove is provided on an inner wall of the convex cylinder.

53. The retrieval device for a retrieval suture according to claim 52, wherein the suture winding portion is of a rod-like structure, the cover is provided with a first connection hole, the suture winding portion is rotatably connected to the cover through the first connection hole, and the suture winding portion is provided with a protrusion or a second through hole for connecting the retrieval suture.

54. The retrieval device for a retrieval suture according to claim 53, wherein one end of the suture winding portion is provided with a flange located on a side opposite to the rotating cap, and a diameter of the flange is larger than that of the first connection hole.

55. The retrieval device for a retrieval suture according to claim 54, wherein the convex cylinder is provided with a second connection hole coaxial with the first connection hole, and the suture winding portion is threadedly connected to the rotating cap through the first connection hole and the second connection hole.

56. The retrieval device for a retrieval suture according to any one of claims 48 to 55, wherein the cover is provided therein with an extension block extending toward a proximal end of the cover, and the extension block and the retrieval handle are respectively provided with a first mounting groove and a second mounting groove for mounting the single-rotation unit.

57. The retrieval device for a retrieval suture according to any one of claims 48 to 55, wherein the retrieval handle is provided with a threading passage for guiding the retrieval suture to pass out, and a side of the cover facing the threading passage is provided with a plurality of threading holes for guiding the retrieval suture to penetrate into the cover.

58. A delivery device for a heart valve prosthesis, **characterized by** comprising: a handle; and the retrieval device for a retrieval suture of a heart valve prosthesis according to any one of claims 40 to 57, wherein the body is disposed at a proximal end of the handle and is detachably connected to the handle.

59. The delivery device according to claim 58, further comprising the heart valve prosthesis, wherein a plurality of retrieval sutures respectively pass through a plurality of connection ring sets on the heart valve prosthesis, and the auxiliary adjusting member is rotatable relative to the body by a certain angle to adjust a position or state of the heart valve prosthesis via one of the retrieval sutures.

60. An interventional delivery device for a heart valve prosthesis, **characterized by** comprising:
a handle;
a delivery assembly extending within the handle and axially movable relative to the handle,
wherein the delivery assembly comprises a transmission rod, and at least two first drive assemblies circumferentially arranged along the transmission rod, the first drive assemblies being in transmission connection with the transmission rod and configured to drive the transmission rod to move axially relative to the handle.

61. The interventional delivery device for a heart valve prosthesis according to claim 60, further comprising a second drive assembly disposed on the transmission rod, wherein the first drive assemblies are in transmission connection with the second drive assembly, and the first drive assemblies are adapted to drive the second drive assembly to rotate, so that the transmission rod moves axially relative to the handle.

62. The interventional delivery device for a heart valve prosthesis according to claim 61, wherein the first drive assembly comprises a first drive member and a first support member sleeved on an outer circumference of the first drive member, the first support member being in tight fit connection with the first drive member, and an end of the first drive member being provided with a first drive portion;
the second drive assembly comprises a second drive member and a second support member sleeved on an outer circumference of the second drive member, the second support member being in tight fit connection with the second drive member, an end of the second drive member being provided with a second drive portion, the second drive portion being engaged with the first drive portion, the transmission rod passing through the second drive member and being in threaded transmission connection with the second drive member, and the first drive member being adapted to drive the second drive member to rotate, so that the transmission rod moves axially relative to the handle.

63. The interventional delivery device for a heart valve prosthesis according to claim 62, wherein the first drive portion comprises a plurality of first drive teeth, the second drive portion comprises a plurality of second drive teeth, a drive tooth groove is formed between two adjacent second drive teeth, and the plurality of first drive teeth are engaged with the plurality of drive tooth grooves.

64. The interventional delivery device for a heart valve prosthesis according to claim 62 or 63, further comprising a power rod, wherein the first drive member is provided with a recess formed by recessing from one end of the first drive member toward the first drive portion, and one end of the power rod is insertable into the recess to drive the first drive member to rotate.

65. The interventional delivery device for a heart valve prosthesis according to claim 64, wherein the handle comprises a first housing provided with a mounting groove corresponding to the first drive assembly, the mounting groove is provided with an annular boss therein, and the first support member is located in the mounting groove and abuts against the annular boss.

66. The interventional delivery device for a heart valve prosthesis according to claim 65, wherein a limiting groove is provided in the first housing, and the second support member is embedded in the limiting groove.

67. The interventional delivery device for a heart valve prosthesis according to claim 66, wherein an end cover for blocking the first support member is further provided at an opening of the mounting groove, and the end cover is detachably connected to a peripheral wall of the mounting groove.

68. The interventional delivery device for a heart valve prosthesis according to claim 66, wherein a chamber is provided in the first housing, the chamber being in communication with the limiting groove and extending axially along the first housing, and a transmission member being mounted in the chamber,
the delivery assembly further comprises a sheath tube, both a proximal end of the sheath tube and a distal end of the transmission rod being respectively connected to the transmission member, the transmission rod being adapted to move axially along the chamber such that the transmission member slides along the chamber and drives the sheath tube to move axially relative to the first housing.

69. The interventional delivery device for a heart valve prosthesis according to claim 68, wherein a reinforcing tube is sleeved on the proximal end of the sheath tube and fixedly connected to the sheath tube, a proximal end of the reinforcing tube is fixedly connected to the transmission member, and
the reinforcing tube is provided with a plurality of holes for glue application.

70. The interventional delivery device for a heart valve prosthesis according to claim 68, wherein a sliding block is provided at a proximal end of the transmission rod, a sliding groove is further provided in the first housing, the sliding block is embedded in the sliding groove and slidably engaged with the sliding groove, and a proximal end of the sliding groove is provided with a stop portion for stopping and position limiting for the sliding block.

71. The interventional delivery device for a heart valve prosthesis according to claim 67, wherein a through channel is provided at a distal end of the first housing, and a distal end of the reinforcing tube is provided with a tapered head for passing through the through channel;
the through channel is in communication with the chamber, and the sheath tube passes through the through channel and is connected to the transmission member in the chamber.

72. A handle of an interventional delivery device for a heart valve prosthesis, **characterized by** comprising a first housing, wherein at least two mounting grooves for accommodating first drive assemblies are circumferentially provided on the first housing.

73. The handle according to claim 72, wherein an insertion post is provided at a distal end of the first housing, and a plurality of teeth are provided at a terminal of the insertion post;
the handle comprises a second housing, an insertion groove is provided at a proximal end of the second housing, the insertion post is inserted into the insertion groove; a receiving groove for accommodating the plurality of teeth is provided at the proximal end of the second housing, the receiving groove is in communication with the insertion groove, and the receiving groove allows the plurality of teeth to rotate.

74. The handle according to claim 73, wherein the first housing comprises a first upper housing and a second lower housing, and the first upper housing and the second lower housing are mutually snapped;
the second housing comprises a second upper housing and a second lower housing, and the second upper housing and the second lower housing are mutually snapped.

75. The handle according to claim 74, wherein three mounting grooves are circumferentially provided on the first housing.

76. A delivery device for a heart valve prosthesis, **characterized by** comprising: a handle; an outer tube connected to the handle; and an inner tube assembly extending within the outer tube and rotatable circumferentially relative to the outer tube,
wherein the inner tube assembly comprises a first inner tube and a second inner tube extending within the first inner tube, and a receiving space for accommodating the heart valve prosthesis is provided between the first inner tube and the second inner tube.

77. The delivery device for a heart valve prosthesis according to claim 76, wherein proximal ends of both the first inner tube and the second inner tube are connected to the handle; the first inner tube comprises an extension tube segment connected to the handle and extending distally within the outer tube, and a receiving tube segment protruding from a distal end of the outer tube for accommodating the heart valve prosthesis, wherein a radial dimension of the receiving tube segment is larger than that of the extension tube segment.

78. The delivery device for a heart valve prosthesis according to claim 76 or 77, wherein the handle comprises a first housing and a second housing connected to the first housing, the second housing being rotatable circumferentially relative to the first housing, wherein a fixing member is provided within the first housing and connected to a proximal end of the outer tube;
the handle further comprises a transmission member, a chamber is provided within the second housing, the transmission member is mounted within the chamber, a proximal end of the first inner tube is connected to the transmission member, and a proximal end of the second inner tube is connected to the second housing, such that when the transmission member rotates with the second housing relative to the first housing, the first inner tube and the second inner tube rotate circumferentially in synchronization relative to the outer tube.

79. The delivery device for a heart valve prosthesis according to claim 78, wherein the fixing member comprises a fixing post and a fixing portion connected to the fixing post, a proximal end of the outer tube is fixedly connected to the fixing post, a mounting cavity is provided within the first housing, the fixing post is embedded in the mounting cavity, the fixing portion is provided with at least one fixing hole, the first housing is provided with at least one fixing mating hole corresponding to the fixing hole, and a fastener passes through the fixing hole and the fixing mating hole to fixedly connect the fixing member to the first housing.

80. The delivery device for a heart valve prosthesis according to claim 79, wherein the first inner tube and the second inner tube both pass through the fixing member and are respectively connected to the transmission member and a proximal end of the second housing.

81. The delivery device for a heart valve prosthesis according to claim 78, wherein a reinforcing tube is sleeved on a proximal end of the first inner tube and fixedly connected to the first inner tube, and a proximal end of the reinforcing tube is fixedly connected to the transmission member.

82. The delivery device for a heart valve prosthesis according to claim 81, wherein the reinforcing tube is provided with a plurality of holes for glue application.

83. The delivery device for a heart valve prosthesis according to claim 81, wherein the first housing is located at a distal end of the handle, the second housing is located at a proximal end of the handle, the chamber extends axially along the second housing, and the transmission member is adapted to move axially along the second housing relative to the chamber to drive the first inner tube to move axially relative to the second inner tube.

84. The delivery device for a heart valve prosthesis according to claim 83, wherein a transmission rod is further provided in the second housing, a distal end of the transmission rod is fixedly connected to the transmission member, a proximal end of the transmission rod is provided with a sliding block, a sliding groove is further provided in the second housing, the sliding block is embedded in the sliding groove and slidably engaged with the sliding groove, and a proximal end of the sliding groove is provided with a stop portion for stopping and position limiting for the sliding block.

85. The delivery device for a heart valve prosthesis according to claim 84, wherein a drive assembly is further provided on the second housing for driving the transmission rod to move axially relative to the handle, the drive assembly comprises a first drive member disposed on the transmission rod and in threaded connection with the transmission rod,
the drive assembly further comprises at least two second drive members circumferentially arranged along the transmission rod, the second drive members are in transmission connection with the first drive member for driving the first drive member to rotate.

86. The delivery device for a heart valve prosthesis according to any one of claims 81 to 85, wherein an insertion groove is provided at a proximal end of the first housing, an insertion post is provided at a distal end of the second housing, the insertion post is inserted into the insertion groove; a locking member is provided at a terminal of the insertion post, a receiving groove for accommodating the locking member is further provided at the proximal end of the first housing, the receiving groove is in communication with the insertion groove, and the receiving groove allows the locking member to rotate.

87. The delivery device for a heart valve prosthesis according to claim 86, wherein a locking mating member is further provided in the first housing, and the locking mating member is mated with the locking member to lock or unlock a relative position between the first housing and the second housing.

88. The delivery device for a heart valve prosthesis according to claim 87, wherein the locking member comprises a locking gear provided at a terminal of the insertion post, the locking gear forming a plurality of locking grooves;
the locking mating member comprises a movable member and at least one locking post provided on the movable member, and when the movable member is driven, the movable member moves axially along the first housing, so that the locking post is adapted to be inserted into or disengaged from the locking groove.

89. The delivery device for a heart valve prosthesis according to claim 88, wherein
a mounting gap is provided in the first housing;
the movable member comprises a drive block, a connection block connected to the drive block, and a mounting block connected to the connection block, the connection block being slidably connected to the mounting gap, the drive block and the mounting block being located inside and outside the first housing respectively, and the mounting block being provided with the locking post.

90. The delivery device for a heart valve prosthesis according to claim 86, wherein a channel is provided in the insertion post, a distal end of the reinforcing tube is provided with a tapered head for passing through the through channel;
the through channel is in communication with the chamber, the first inner tube passes through the through channel, a proximal end of the first inner tube is connected to the transmission member in the chamber; the second inner tube extends within the first inner tube and passes through the through channel to be connected to a proximal end of the second housing.
